# EUROPÄISCHE PATENTSCHRIFT

(11) **EP 3 079 894 B1**
(45) Veröffentlichungstag und Bekanntmachung des Hinweises auf die Patenterteilung: **30.11.2022**
(21) Anmeldenummer: 14897728.3
(22) Anmeldetag: 11.12.2014
(51) Int. Cl.: B29C 43/08, B30B 11/34, B30B 11/08, B29C 43/36, A61J 3/10, A61J 3/00, B29K 105/00, B29C 43/32

(54) **VERFAHREN ZUR HERSTELLUNG EINES FORMTEILS**
METHOD FOR PRODUCING A MOULDED PIECE
PROCÉDÉ DE FABRICATION D'UNE PIÈCE MOULÉE

(30) Priorität: 11.12.2013 DE 102013113891; 11.01.2014 DE 102014100269; 14.04.2014 DE 102014105325; 03.06.2014 DE 102014107820
(43) Veröffentlichungstag der Anmeldung: 19.10.2016
(73) Patentinhaber: Saur-Brosch, Roland, 69126 Heidelberg (DE)
(72) Erfinder: Saur-Brosch, Roland, 69126 Heidelberg (DE)
(86) Internationale Anmeldenummer: PCT/IB2014/003244
(87) Internationale Veröffentlichungsnummer: WO 2016/009248

(56) Entgegenhaltungen:
- EP-A1- 1 440 790
- EP-A2- 0 470 679
- EP-B1- 1 302 304
- US-A1- 2008 116 606
- US-A1- 2008 116 606
- HARIHARAN M AND GUPTA V K: "A novel compression-coated tablet dosage form", PHARMACEUTICAL TECHNOLOGY, ADVANSTAR COMMUNICATIONS,INC, US , Nr. YEARBOOK 2001 1. Januar 2001 (2001-01-01), Seiten 14-19, XP002678748, ISSN: 1543-2521 Gefunden im Internet: URL:http://www.pharmtech.com/pharmtech/dat a/articlestandard/pharmtech/512001/5072/ar ticle.pdf

## Beschreibung

Die Erfindung beträgt ein Verfahren zur Herstellung eines Formteils (Formpresslings, Formkörpers) unter Verwendung einer Formpresseinrichtung.

Im Stand der Technik ist es bekannt, Formteile durch die Verwendung einer Formpresseinrichtung herzustellen. Beispiele für solche Formteile wären unter anderem Tabletten für pharmazeutische oder ernährungsphysiologische Anwendungen.

Üblicherweise werden hierfür Rohmaterialien wie z.B. Pulver, Pellets oder Granulate, ggf. auch Mischungen davon, in Formen (auch als Matrizen bezeichnet) gefüllt und mit Hilfe von Stempeln, über welche Druck auf die Rohmaterialien ausgeübt wird, in die gewünschte Form gepresst.

Ein Beispiel für die Herstellung solcher Formteile ist die Verpressung von pharmazeutischen Wirkstoffen, ggf. vermischt oder sonst wie verarbeitet mit Hilfsstoffen, zu Tabletten. Hierzu wird beispielsweise eine Teilform bereitgestellt, welche die lateralen Außenmaße des Formteils definiert (Matrize), und die beispielsweise aus einer waagerecht angeordneten Platte mit einer oder mehreren runden, ovalen oder sonst wie geformten Durchdringungen (Löchern, Öffnungen) besteht.

Die Matrize oder mehrere davon kann/können in einer weiteren Scheibe (Matrizentisch) befestigt sein. Teilweise stellt der Matrizentisch direkt die Matrize mit ihren Bohrungen/Öffnungen dar.

Des Weiteren wird ein unterer Stempel bereitgestellt, welcher die unteren Ausmaße des Formteils definiert, sowie ein oberer Stempel bereitgestellt, welcher die oberen Ausmaße des Formteils definiert. Diese beiden Stempel bestehen beispielsweise aus Zylindern, welche von der Form des Zylindermantels her so geformt sind, dass sie mit möglichst geringem mechanischem Spiel in und/oder durch die vorgenannten Durchdringungen hinein und/oder hindurch ragen können.

Lässt man den unteren Zylinder etwas in die Durchdringung hinein ragen, bilden der untere Zylinder und die Platte mit der Durchdringung eine Art Wanne (Füllraum), in welche die zu verpressenden Rohmaterialien gefüllt werden können. Anschließend kann durch das Eindringen des oberen Stempels in die Durchdringung und die Ausübung eines nach unten gerichteten Druckes auf den oberen Stempel, sowie die vertikale Fixierung des unteren Stempels oder eines nach oben gerichteten Druckes auf diesen das Rohmaterial zu dem gewünschten Formteil verpresst werden.

### Üblicherweise bestehen solche Verfahren aus drei Arten von Vorgängen

### 1. Füllen

Der untere Stempel ragt von unten in Matrize hinein, jedoch nur so weit, dass seine Oberkante um ein bestimmtes Maß unterhalb der Oberkante der Matrize bleibt

Der verbleibende Raum zwischen Oberkante des unteren Stempels und der Oberkante (Füllraum) der Matrize wird mit Rohmaterial befüllt

Dies erfolgt üblicherweise dadurch, dass ein nach unten hin offenes Behältnis (Füllschuh), in dem sich Rohmaterial befindet, über die Öffnung (Durchdringung) der Matrize hinweg bewegt wird (oder die Öffnung unter dem Füllschuh hinweg).

Dabei fällt Rohmaterial aus dem Füllschuh in den verbleibenden Füllraum.

Die Kante der Seitenwand des Füllschuhes nimmt alles Rohmaterial oberhalb der Oberkante der Matrize weg, so dass die Größe des Füllraums die Menge des Rohmaterials bestimmt.

### 2. Pressen

Der obere Stempel, der zuvor ggf. oberhalb der Matrize bereitgehalten wurde, wird abgesenkt, so dass er in die Matrizenöffnung ragt.

Hierbei wird das Rohmaterial gegen den unteren Stempel gepresst (und auch gegen die Seitenwände der Matrizenöffnung).

Der untere Stempel wird ggf. vor dem Absenken des oberen Stempels etwas abgesenkt, so dass der obere Stempel etwas in den Füllraum eindringen und ihn nach oben hin abdichten kann, ohne an der Oberfläche befindliche Partikel unbeabsichtigt zur Seite zu verdrängen.

Danach wird er in Position gehalten oder ggf. angehoben um die Verpressung zu unterstützen.

Die Absenkung des oberen Stempels und ggf. die Anhebung des unteren Stempels bestimmen die Presskraft und die Höhe des Formteils.

Nach dem Verpressen wird der obere Stempel wieder angehoben, üblicherweise mit seiner Unterkante um mehr als die Höhe des Formteils über die Oberkante der Matrize um den folgenden Ausstoßschritt zu ermöglichen, bzw. um mehr als die Höhe des Füllschuhs um einen weiteren Füllschritt zu ermöglichen.

Es können auch zwei Pressschritte statt einem erfolgen, wobei das Rohmaterial beim ersten Pressschritt meist mit einer geringeren Presskraft verdichtet und dann beim zweiten Pressschritt mit einer stärkeren Presskraft weiter verdichtet und/oder plastisch verformt wird.

### 3. Ausstoßen

Der untere Stempel wird angehoben, üblicherweise mit seiner Oberkante bis zur oder über die Oberkante der Matrize.

Dadurch wird das Formteil über die Matrize angehoben und dann üblicherweise durch eine Ausstoßvorrichtung oder ein Abweisblech aus der Formpresseinrichtung entnommen.

Zur Herstellung mehrschichtiger Formteile können die Schritte 1 und 2 mehrfach nacheinander ausgeführt werden, vorzugsweise abwechselnd.

Hierbei ist es ggf. vorteilhaft, den Pressdruck der anfänglichen Pressschritte niedriger zu halten als den des letzten Pressschrittes.

Üblicherweise werden solche Verfahren mithilfe von Rundlaufpressen durchgeführt.

Der Matrizentisch und die Stempel bewegen sich um eine gemeinsame, meist senkrecht stehende Achse, wobei die Stempel mit Hilfe schienenartiger Kurvenbahnen während des Umlaufs in die Positionen für Befüllung, Verdichtung, plastische Verformung und Ausstoß gebracht werden. An den Stellen, an denen eine besonders gravierende Anhebung bzw. Absenkung der Stempel erforderlich ist (Befüllen, Verdichten, Ausstoßen), werden diese Kurvenbahnen durch zusätzliche Niederdruckstücke, Niederzugschienen und Aushebebahnen unterstützt. Die Befüllung der Matrize erfolgt über eine starr angeordnete Zuführeinichtung, den sogenannten Füllschuh, der mit einem Vorratsbehälter für das Vorgemisch verbunden ist. Der Pressdruck auf das Vorgemisch ist über die Presswege für Ober- und Unterstempel individuell einstellbar, wobei der Druckaufbau durch das Vorbeirollen der Stempelschaftköpfe an verstellbaren Druckrollen geschieht.

Zur Herstellung von Formteilen, die einen Kern aus einem ersten Rohmaterial (Kernmaterial) enthalten, welcher von einem zweiten Rohmaterial (Hüllmaterial) umhüllt ist (sogenannte Kernformteile), sind verschiedene Verfahren bekannt:
Eine Möglichkeit solch ein Kernformteil, herzustellen, besteht darin, vor dem zweiten Füllschritt einen Kern in den Füllraum einzubringen, der sowohl in lateraler als auch in vertikaler Dimension kleiner ist als der Füllraum.

Danach wird mit dem zweiten Füllschritt fortgefahren, wodurch der verbleibende Füllraum seitlich und oberhalb des eingebrachten Kerns mit Rohmaterial aufgefüllt wird.

Bei der Einbringung des Kerns sollte auf eine möglichst zentrale Positionierung geachtet werden, damit die Stärke der lateralen Umfüllung mit Rohmaterial möglichst gleichmäßig ist.

Unter Umständen kann auf den ersten Pressschritt vor dem Einbringen des Kerns verzichtet werden.

Ein Nachteil dieses Verfahrens ist, dass ein bereits vorgefertigter Kern zur Verfügung gestellt werden muss und kein Pulver, Granulat oder sonstiges nicht kernförmiges Material verwendet werden kann.

Weiterhin ist die möglichst mittige Platzierung des Kerns schwierig, insbesondere wenn Matrize(n) und Stempel Teile einer Rundlaufpresse sind, in der durch die Drehbewegung Zentrifugalkräfte auftreten, welche den Kern dezentrieren können.

In EP 0470679A2 wird eine Presse beschrieben, welche diese Möglichkeit realisiert, indem vorgefertigte Kernteile durch Übergabeköpfe, bei welchen die vorgefertigten Kerne über eine Haltevorrichtung an der Unterseite der Übergabeköpfe gehalten werden, in den Füllraum eingebracht werden.

Ein Verfahren ein Kernformteil herzustellen, welches beide Nachteile vermeidet, ist in "A Novel Compression-Coated Tablet Dosage Form", Madhusudan Hariharan und Vishal K. Gupta, Pharmaceutical Technology YEARBOOK 2001, beschrieben.

Dort wird ein oberer Stempel beschrieben, welcher aus Innen- und Außenstempel besteht, welche vertikal gegeneinander verschiebbar sind (quasi ein zweiteiliger koaxialer Stempel).

Üblicherweise hat der Außenstempel eine bestimmte, über den gesamten Umfang gleichmäßige Wandstärke und der Innenstempel füllt die Öffnung des Außenstempels mit einem möglichst geringen mechanischen Spiel aus.

Beim ersten Füllschritt wird Rohmaterial für die Umhüllung (Hüllmaterial) eingefüllt.

Beim ersten Pressschritt ist der Innenstempel gegenüber dem Außenstempel nach unten verschoben.

Beim Eindringen des Innenstempels in den Füllraum muss ein Teil des Rohmaterials zu den seitlichen Matrizenwänden hin verdrängt werden, damit Seitenwände entstehen können, die höher sind als die unter dem Innenstempel gepresste Bodenschicht (Bodenplatte) und eine Art tassenförmiges Formteil entsteht.

Hierfür ist es ggf. erforderlich den Innenstempel unten in einer stumpfen Spitze angeschrägt zulaufen zu lassen.

Durch die Verschiebung des Innenstempels nach unten dringt der Außenstempel später in die Matrizenöffnung ein und verpresst das seitlich verdrängte Material zu den Seitenwänden des tassenförmigen Formteils.

Üblicherweise wird die Schräge des Innenstempels beim Außenstempel fortgesetzt, damit die Seitenwände an ihrer Oberkante eine entsprechend nach innen geneigte Schräge aufweisen.

Nach dem Anheben des oberen Stempels wird der Füllschritt für das Rohmaterial des Kerns (Kernmaterial) durchgeführt.

Durch eine weitere Absenkung des oberen Stempels mit nach unten verschobenem Innenstempel wird das Kernmaterial in den nun von dem tassenförmigen Teilformteil umfassten Füllraum gepresst.

Nach dem Anheben des oberen Stempels sorgt die schräg nach innen geneigte Oberkante der Seitenwände dafür, dass darauf liegen gebliebenes Kernmaterial nach innen auf den vorgepressten Kern rutscht.

Wenn erforderlich kann dieses Material durch eine weitere Absenkung des oberen Stempels mit nach unten verschobenem Innenstempel nochmals an das bereits vorgepresste Kernmaterial angepresst werden.

Danach erfolgt der dritte Füllschritt. Dieser wird mit Hüllmaterial durchgeführt.

Beim letzten Pressschritt sind Innenstempel und Außenstempel des oberen Stempels nicht mehr vertikal gegeneinander verschoben.

Somit ergibt sich als untere Fläche des oberen Stempels eine einheitliche Fläche, die ggf. in einer leichten Schräge eine stumpfe Spitze bildet.

Beim Absenken des oberen Stempels wird die Deckelplatte (Deckelschicht) des Formteils ausgebildet und das gesamte Formteil zu seiner fertigen Höhe verpresst.

Ein Nachteil dieses Verfahrens ist, dass der obere Stempel leicht angespitzt sein muss, um das Material des ersten Füllvorgangs teilweise zu den Seitenwänden zu verdrängen und um die Seitenwände oben anzuschrägen, damit darauf verbliebenes Kernmaterial nach innen rutschen kann.

Dadurch wird die Formgestaltung des Formteils eingeschränkt.

Außerdem ist dadurch keine gleichmäßige Stärke der Bodenplatte möglich, da bei einer Anschrägung des unteren Stempels in gleichem Maße wie der des oberen Stempels die seitliche Verdrängung des Hüllmaterials beim ersten Pressschritt nicht in ausreichendem Maße erfolgt.

Des Weiteren wird das Rohmaterial unter dem Innenstempel stärker komprimiert als unter dem Außenstempel, so dass sich die Eigenschaften der Bodenplatte und der Seitenwände unterscheiden (z.B. Festigkeit, Auflösungsverhalten, Dichtigkeit etc.).

Ein weiterer Nachteil ist, dass ggf. zwei Pressschritte für das Kernmaterial erforderlich sind.

Ein weiterer Nachteil ist, dass Innen- und Außenstempel des oberen Stempels separat geführt werden müssen, was eine komplizierte Führung der Stempel erfordert.

Ein weiteres Verfahren, ein Kernformteil herzustellen, ist in EP1302304B1 und EP1440790A1 beschrieben.

Dort werden sowohl oberer als auch unterer Stempel als Innen- und Außenstempel beschrieben.

Diese Stempel sind ähnlich aufgebaut wie in "A Novel Compression-Coated Tablet Dosage Form", jedoch nicht notwendigerweise mit der abgeschrägten Spitze. Für die ersten und zweiten Füll- und Pressvorgänge wird der Außenstempel des unteren Stempels vertikal angehoben, so dass er möglichst bündig mit der Oberkante der Matrize abschließt.

Dadurch wird ein in lateraler Dimension kleinerer Füllraum geschaffen.

Ebenfalls für die ersten und zweiten Füll- und Pressvorgänge wird der Außenstempel des oberen Stempels vertikal angehoben, so dass er beim Absenken des oberen Stempels nicht in den Füllraum eintritt. Es wird somit ein zweischichtiges Teilformteil erzeugt, bei dem die untere Schicht aus Hüllmaterial und die obere Schicht aus Kernmaterial besteht.

Vor dem dritten Füllschritt wird der Außenstempel des unteren Stempels vertikal abgesenkt, so dass er möglichst bündig mit dem Innenstempel abschließt.

Dadurch wird beim dritten Füllschritt, der dann mit Hüllmaterial erfolgt, sowohl der Füllraum oberhalb, als auch der nun frei gewordene Raum seitlich des Teilformteils mit Hüllmaterial gefüllt.

Für den dritten Pressschritt wird der Außenstempel des oberen Stempels vertikal abgesenkt, so dass er möglichst bündig mit dem Innenstempel abschließt.

Beim Absenken des oberen Stempels werden der Deckel (die Deckelplatte) des Formteils und die Seitenwände ausgebildet und das gesamte Formteil zu seiner fertigen Höhe verpresst.

Ein Nachteil dieses Verfahrens ist, dass zwei Stempel mit Innen- und Außenstempel eingesetzt werden müssen, welche separat geführt werden müssen, was eine sehr komplizierte Führung der Stempel erfordert. Diese kann üblicherweise nur bei sehr aufwendig konstruierten Pressen realisiert werden. Außerdem wird dadurch die maximale Geschwindigkeit und somit die Fertigungskapazität pro Zeiteinheit limitiert.

Ein Verfahren zur Herstellung eines Formteils mit einem Kern unter Verwendung einer Formpressvorrichtung gemäß dem Oberbegriff des Anspruchs 1 ist beispielsweise aus EP 0 470 679 A2 bekannt.

Beschreibung der Erfindung:
Die Erfindung stellt ein optimiertes Verfahren bereit, das durch die Merkmale des Anspruchs 1 gekennzeichnet ist. Ebenfalls stellt die Erfindung einen Stempel bereit, der zur Ausführung einer bevorzugten Ausführung des Verfahrens geeignet ist.

Durch Verwendung eines unteren Stempels (1), bei dem der Außenstempel (1B) durch Druck von oben nach unten verschoben werden kann, ist es möglich, auch beim ersten und zweiten Pressschritt einen oberen Stempel (2) einzusetzen, der einteilig ist, bzw. bei dem sich Innen- (2A) und Außenstempel (2B) nicht gegeneinander verschieben lassen.

Beim Absenken des oberen Stempels weicht der untere Außenstempel nach unten zurück, sobald der obere Stempel Druck auf ihn ausübt. Dadurch kann der obere Stempel das im Füllraum (4) befindliche Rohmaterial (5) verpressen, ohne dass hierzu ein zweiteiliger oberer Stempel mit zurückgezogenem (angehobenem) Außenstempel benötigt wird.

Da der untere Außenstempel bei diesen Schritten nicht aktiv nach unten gezogen wird, sondern passiv dem oberen Stempel folgt, können keine durch nicht völlig synchrone Bewegungen auftretenden Lücken zwischen den Stempeln entstehen und das Rohmaterial kann während dem Pressschritt nicht seitlich entweichen.

In einer bevorzugten Ausführungsform wird vorgeschlagen, dass der obere Stempel eine leicht erhabene Oberfläche aufweist, deren laterale Abmessungen dem unteren Innenstempel entsprechen.

Dadurch kann der Verschluss des Füllraums zur Seite hin verbessert werden. Die dadurch entstehende Stufe in der Oberseite des Formteils ist üblicherweise tolerierbar.

In einer bevorzugten Ausführungsform wird vorgeschlagen, den oberen Stempel zweiteilig auszuführen und den oberen Innenstempel (2A) gegenüber dem oberen Außenstempel (2B) leicht nach unten abzusenken um eine solche Verbesserung des Füllraumverschlusses zu erreichen. Der obere Stempel wird vorzugsweise so ausgeführt, dass der obere Innenstempel durch Druck von unten so weit nach oben verschoben werden kann, dass er wieder plan mit dem Außenstempel ist (z.B. federnd gelagert mit Anschlag, siehe oberer Stempel in Zeichnung 38). Dadurch kann sowohl ein verbesserter Verschluss des Füllraums als auch eine ebene Oberfläche des Formteils erreicht werden. In einer bevorzugten Ausführungsform beträgt die Verschiebung des Innenstempels nach unten maximal 20% der angestrebten Höhe des Formteils, mehr bevorzugt maximal 10% und besonders bevorzugt maximal 5%. Die Federkraft wird vorzugsweise deutlich geringer gewählt als die Presskraft der ersten beiden Pressschritte. Vorzugsweise ist sie geringer als 30% der geringeren der beiden Presskräfte (bei Verzicht auf Pressschritt 1 jedoch gemessen an Pressschritt 2), mehr bevorzugt geringer als 10% und noch mehr bevorzugt geringer oder gleich 5%.

Eine Möglichkeit, die Verschiebbarkeit des unteren Außenstempels durch Druck von oben zu realisieren, ist die federnde Lagerung der Führungsschienen (Führungskurven, Unterstempelbahnen, Niederzugschinen etc.), welche die Position des Außenstempels bestimmen. Dabei können diese Führungsschienen beispielsweise nur dort federnd gelagert sein, wo sie den Außenstempel gegenüber dem Innenstempel anheben, damit dort, wo er nicht angehoben ist, z.B. beim letzten Pressschritt, eine Verpressung auch der Seitenwände erreicht werden kann.

Sie können in einer weiteren Ausführungsform aber auch auf ihrer gesamten Länge federnd gelagert sein. Dann erfolgt beim letzten Pressschritt eine Unterstützung der Führung durch zusätzliche Andruckrollen.

In einer weiteren Ausführungsform ist die Federung in den Außenstempel integriert (Siehe Zeichnungen 15 bis 18, Elemente 6), so dass auf eine Federung der Führungsschienen (Stempelführung/Führungskurven) verzichtet werden kann.

In einer weiteren Ausführungsform ist die Federkraft so gewählt, dass sie größer ist, als der Druck von oben, dem der untere Außenstempel beim letzten Pressschritt ausgesetzt ist (Pressdruck pro Fläche des Formteils mal obere Fläche des unteren Außenstempels). Dadurch wird erreicht, dass der vor dem letzten Füllschritt abgesenkte Außenstempel beim letzten Pressschritt nicht noch weiter absinkt. Bei den vorhergehenden Pressschritten muss der obere Stempel dann zwar mit einer höheren Kraft nach unten bewegt werden. Da bei diesen Pressschritten aber üblicherweise ohnehin ein geringerer Pressdruck auf das Rohmaterial benötigt wird, und die Oberfläche des Außenstempels üblicherweise weniger als 50% der Grundfläche des Formteils beträgt, ist diese Kraft mit üblichen Andrucksystemen realisierbar.

Diese Ausführungsform bezüglich der Wahl der Federkraft ist sowohl bei gefederten Führungsschienen als auch bei einer in den Außenstempel integrierten Federung möglich.

In einer weiteren Ausführungsform bei einer in den Außenstempel integrierten Federung ist der Federweg des oberen (gefederten) Teils des Außenstempels gegenüber dem unteren Teil des Außenstempels nach oben hin begrenzt. Dies kann beispielsweise durch eine begrenzte Länge der Feder oder der verwendeten Federn im druckfreien Zustand realisiert werden oder durch einen entsprechenden Anschlag (Zeichnung 20, Element 10).

Dadurch ist eine genauere Ausrichtung der Oberkante des Außenstempels möglich, beispielsweise zur Oberkante der Matrize (beispielsweise sinnvoll bei den ersten beiden Füllschritten) oder zur Oberkante des Innenstempels (beispielsweise beim letzten Füllschritt).

In einer weiteren Ausführungsform ist der Federweg des Außenstempels (bzw. bei Außenstempeln mit integrierter Federung der Federweg des oberen Teils des Außenstempels) gegenüber der Matrize nach oben hin begrenzt, beispielsweise durch einen entsprechenden Anschlag (siehe Zeichnung 22, Element 9). Dies erleichtert die Ausrichtung der Oberkante des Außenstempels bei den ersten beiden Füllschritten.

In einer weiteren Ausführungsform bei einer in den Außenstempel integrierten Federung ist der Federweg des oberen (gefederten) Teils des Außenstempels gegenüber dem unteren Teil des Außenstempels nach unten hin begrenzt. Dies kann beispielsweise durch eine begrenzte Zusammendrückbarkeit der Feder oder einer der verwendeten Federn realisiert werden oder durch einen entsprechenden Anschlag (Zeichnung 19, Element 8).

Dadurch kann z.B. die Federkraft der gefederten Lagerung des Oberstempels geringer gewählt werden, als bei den Ausführungsformen, bei denen die Federkraft so gewählt ist, dass sie größer ist, als der Druck von oben, dem der untere Außenstempel beim letzten Pressschritt ausgesetzt ist, um die Position des Außenstempels beim letzten Pressschritt halten zu können.

Beispielsweise wird bei einer Ausführungsform mit einem nach unten hin begrenzten Federweg des oberen (gefederten) Teils des Außenstempels gegenüber dem unteren Teil des Außenstempels nach dem letzten Füllschritt zuerst der obere Stempel so weit abgesenkt, dass genügend Druck von oben auf das eingefüllte Rohmaterial ausgeübt wird, um den unteren Teil des unteren Außenstempels bis zum Anschlagspunkt (oder der maximalen Zusammendrückbarkeit der Feder) anheben zu können, ohne dass der obere Teil sich dadurch wesentlich anhebt. Danach kann der obere Stempel mit dem vollen Pressdruck belastet werden und das Rohmaterial verpressen, ohne dass der obere Teil des unteren Außenstempels nach unten zurückweichen kann.

In einer weiteren Ausführungsform ist die Verschiebbarkeit des Außenstempels (bzw. bei Außenstempeln mit integrierter Federung der Federweg des oberen Teils des Außenstempels) gegenüber dem Innenstempel nach unten hin begrenzt. Dies kann beispielsweise durch einen Anschlag realisiert sein (siehe Zeichnungen 21 und 22, Element 11). Die Position des Anschlags ist vorzugsweise in der Höhe verstellbar. Dies kann beispielsweise durch Einlegen von Unterlegscheiben oder durch über Schraubgewinde verstellbare Anschläge erfolgen. Vorzugsweise wird die Anschlagsposition so eingestellt, dass beim Anschlag die Oberkanten des Innenstempels und des Außenstempels an denen einander zugewandten Kanten auf gleicher Höhe sind. Hierdurch können z.B. bei Verwendung von Führungsschienen für den Außenstempel diese überall federnd gelagert sein, ohne zusätzliche Andruckrollen zu benötigen. Der benötigte Pressdruck wird vom Innenstempel über den Anschlag an den Außenstempel weitergeleitet.

Damit bei einer Ausführungsform, bei welcher der Federweg des (oberen Teils des) Außenstempels gegenüber der Matrize nach oben hin durch einen Anschlag begrenzt ist, dieser Anschlag beim Ausstoßschritt, bei dem üblicherweise zumindest einer der unteren Stempel über die Oberkante der Matrize hinaus angehoben wird, nicht stört, wird er in einer weiteren Ausführungsform gefedert ausgeführt (siehe Zeichnung 24, Element 7). Vorzugsweise ist die dafür verwendete Feder stärker als diejenige, welche den Außenstempel (bzw. seinen oberen Teil) nach oben drückt. Dann bewirkt vorzugsweise erst ein Anheben des Innenstempels über den Anschlagspunkt zum Außenstempel hin hinaus oder eine über den maximalen Federweg des oberen Teil des Außenstempels hinausgehende Anhebung des unteren Teils des Außenstempels eine weitere Anhebung des Außenstempels.

Die in den vorgenannten Ausführungsformen beschriebenen Anschlagskonstruktionen können auf verschiedene Arten realisiert werden. Hierzu sind dem Fachmann vielfältige Möglichkeiten bekannt. Die Anschläge können nicht nur innerhalb der lateralen Abmessungen des Außenstempels realisiert werden (wie in Zeichnung 19 dargestellt), sondern können beispielsweise auch außerhalb (beispielsweise wie in Zeichnung 20 dargestellt) oder im verdickten Führungsschaft des Stempels untergebracht werden.

Eine weitere Möglichkeit, die Verschiebbarkeit des unteren Außenstempels durch Druck von oben zu realisieren, ist die federnde Lagerung des Außenstempels gegenüber dem Innenstempel (siehe Zeichnung 25, Elemente 12).

In einer weiteren Ausführungsform mit einem solchen Stempel mit integrierter Federung ist dessen Führung so ausgeführt, dass der Außenstempel nur dann über außerhalb des unteren Stempels angeordnete Führungen geführt wird, wenn er gegenüber dem Innenstempel nicht komplett angehoben ist. Dies wird beispielsweise dadurch erreicht, dass die Führung so ausgelegt ist, dass sie nur von oben auf den Führungsbolzen (bzw. das Führungselement) des Außenstempels einwirkt, beispielsweise durch eine nur oberhalb des Führungsbolzens angeordnete Führungsschiene.

Eine Limitierung des Federweges gegenüber dem Innenstempel oder der Matrize kann wie bei den vorstehend beschriebenen Ausführungsformen realisiert werden.

In einer weiteren Ausführungsform mit einem solchen Stempel mit integrierter Federung ist dieser so ausgeführt, dass der Außenstempel auch dann nach unten verschiebbar ist, wenn er über seine außerhalb des unteren Stempels angeordnete (bzw. auf den unteren Teil des Außenstempels einwirkende) Führungen ungefedert geführt ist. Diese Führung limitiert dann nur seine Verschiebbarkeit nach oben hin. Nach unten hin wird sie durch einen Anschlag gegenüber dem Innenstempel limitiert. Hierfür wird der Außenstempel vorzugsweise mindestens zweiteilig ausgeführt (siehe Zeichnung 26, Elemente 1B und 1B2). Diese Ausführungsform gestattet beispielsweise die Verwendung von Rundlaufpressen, welche für zweiteilige Unterstempel ausgelegt sind, ohne dass die Stempelführungen modifiziert werden müssten. Auch bei dieser Ausführungsform kann eine Limitierung des Federweges gegenüber dem Innenstempel oder der Matrize wie bei den vorstehend beschriebenen Ausführungsformen realisiert werden.

In einer weiteren Ausführungsform mit einem Stempel mit integrierter Federung ist der Stempel mit einem Rastmechanismus versehen (Zeichnung 23, Element 13). Durch diesen Rastmechanismus kann die Verschiebbarkeit des Außenstempels gegenüber dem Innenstempel begrenzt werden. Alternativ kann die Verschiebbarkeit des Außenstempels in ähnlicher Weise gegenüber der Matrize begrenzt werden.

In einer weiteren Ausführungsform wird die Verschiebbarkeit des Außenstempels gegenüber dem Innenstempel oder der Matrize durch den Rastmechanismus nur nach oben begrenzt. Nach unten ist der Außenstempel weiterhin durch Druck von oben verschiebbar, zumindest bis zu einem eventuell vorhandenen Anschlag gegenüber dem Innenstempel, der Matrize oder einem eventuell vorhandenen unteren Teil des Außenstempels.

Das Ein- und/oder Ausrasten des Rastmechanismus kann über entsprechende Anschläge (Zapfen/Betätigungselemente) z.B. an den Führungsschienen des unteren Stempels gesteuert werden. So kann vor dem zweiten Pressschritt der Rastmechanismus aktiviert (scharf gestellt) werden, so dass der Außenstempel beim zweiten Pressschritt in der nicht angehobenen Position einrastet. Vorzugsweise wird der Rastmechanismus nach dem letzten Pressschritt oder nach dem Ausstoßschritt gelöst, so dass der Außenstempel beim nächsten ersten Füllschritt wieder durch die federnde Lagerung angehoben sein kann.

In einer weiteren Ausführungsform mit einem Stempel mit integrierter Federung und Rastmechanismus ist der Rastmechanismus so ausgeführt, dass er durch die Bewegungen des Außenstempels gegenüber dem Innenstempel aktiviert und/oder gelöst wird. Dies kann beispielsweise durch einen Push-Push-Mechanismus realisiert sein, wie er auch bei Kugelschreibern eingesetzt wird, um die Kugelschreibermine durch mehrmaliges Drücken des Betätigungsknopfes abwechselnd in eine ausgefahrene oder eingefahrene Position zu bringen (siehe z.B. http://www.lehrerfreund.de/technik/1s/kugelschreiber/3078, am 30.05.2013 archiviert bei http://www.webcitation.ora/6H0dgMhHv). Der Rastmechanismus ist beispielsweise so konstruiert, dass der Außenstempel (z.B. mit einem darin integrierten Zahnring 16) dem Griffrohr des Kugelschreibers entspricht und die Teile des Innenstempels dem Betätigungsknopf (unterer Teil 1A3) und Mine (mittlerer Teil 1A2 und oberer Teil 1A1), wobei die Druckhülse (17) dazwischen liegt (siehe Zeichnungen 27 bis 36). Durch entsprechende Ausführung der Verzahnung ist es möglich, den Rastmechanismus so auszuführen, dass er nur bei jeder dritten oder höherzahligen Betätigung (durch die vom oberen Stempel ausgelöste Vertikalbewegung des unteren Außenstempels) einrastet, um zwei oder mehr Füllschritte ausführen zu können, bevor beim letzten Füllschritt die Seitenwände mit verfüllt werden. Bei einer bevorzugten Ausführungsform ist der Rastmechanismus so ausgeführt, dass er bei mehreren Betätigungen an zwei oder mehr individuellen Positionen einrasten kann, je nachdem wie viele Füll- und Pressschritte für die Herstellung des Formteils erforderlich sind. Dadurch ist es beispielsweise möglich, durch eine für jeden Füllschritt individuell eingestellte Höhe des Innenstempels für jeden Füllschritt individuelle Füllmengen einzustellen, während die Oberkante des Außenstempels in einer Ebene mit der Oberkante der Matrize liegt. Die Realisierung individueller Einrastpositionen kann durch unterschiedliche Höhen oder Positionen der Zähne im Zahnkranz des Rastmechanismus erreicht werden, der beispielsweise im Schaft des Außenstempels (analog des Griffrohres des Kugelschreibers) angeordnet wird. Die Anzahl der Positionen kann durch die Anzahl der Zähne und Nuten des Push-Push-Mechanismus und die Anzahl der Wiederholungen eingestellt werden. Beispielsweise ist der Rastmechanismus mit zwölf Zähnen/Nuten ausgeführt, wobei drei nacheinander folgende individuelle Positionen viermal wiederholt werden. Push-Push-Mechanismen bei Kugelschreibern sind üblicherweise mit acht Zähnen/Nuten ausgeführt, wobei zwei nacheinander folgende individuelle Positionen (Mine ein- und ausgefahren) viermal wiederholt werden).

In einer weiteren Ausführungsform mit einem Stempel mit integrierter Federung und Rastmechanismus ist der Rastmechanismus so ausgeführt, dass er durch die Bewegungen des Außenstempels gegenüber der Matrize aktiviert und/oder gelöst wird. Auch hier sind individuelle Positionen möglich, beispielsweise wie sie bei der Ausführungsform mit Aktivierung durch Bewegungen des Außenstempels gegenüber dem Innenstempel beschrieben sind.

In einer weiteren Ausführungsform werden zwei oder mehr Rastmechanismen eingesetzt, die entweder durch Bewegungen des Außenstempels gegenüber dem Innenstempel oder durch die Bewegungen des Außenstempels gegenüber der Matrize aktiviert und/oder gelöst werden, wobei nicht alle Rastmechanismen durch den gleichen Bewegungsvorgang ausgelöst werden müssen.

In einer solchen Ausführungsform werden beispielsweise zwei Rastmechanismen eingesetzt, von denen einer durch Bewegungen des Außenstempels gegenüber dem Innenstempel und der andere durch die Bewegungen des Außenstempels gegenüber der Matrize aktiviert und/oder gelöst wird.

Wird der Rastmechanismus so ausgeführt, dass der Außenstempel gegenüber dem Innenstempel einrastet, nachdem er durch den vom oberen Stempel ausgeführten Druck relativ zum Innenstempel nach unten bewegt wurde, so kann er möglicherweise nicht so weit unten einrasten, wie es erforderlich wäre, um die seine Oberkante mit der Oberkante des Innenstempels auf eine Höhe zu bringen. Für den ersten Füll- und Pressschritt und die Füll- und Pressschritte, bei denen Kernmaterial eingefüllt wird, ist das normalerweise unkritisch. Beim letzten Füll- und Pressschritt, bei dem normalerweise auch die Seitenwände verfüllt und verpresst werden, kann dies allerdings problematisch sein, weil hierdurch das Rohmaterial beim Füllschritt nicht neben dem Kern bis auf die Höhe des unteren Stempels gelangen kann. Bei Deckelschichten mit großer Schichtstärke wird allerdings ein Teil des eingefüllten Rohmaterials durch den oberen Stempel auch entlang der Seiten des Kerns nach unten gepresst, da der Außenstempel durch den Pressdruck weiter nach unten gedrückt wird. Allerdings wird dann, je nach Geometrie des oberen Stempels, das Rohmaterial seitlich des Kerns eventuell weniger stark komprimiert (verpresst) als unterhalb und oberhalb des Kerns. Bei vielen Anwendungen ist dies jedoch unkritisch und kann ggf. durch ein Verhältnis von Seitenwandstärke zu Bodenplattenstärke von größer als 1:1 kompensiert werden.

Damit der Füllraum auch seitlich des bisher verpressten Teilformteils befüllt werden kann, ist es vorteilhaft, den Außenstempel gegenüber dem Innenstempel weiter abzusenken, als nur bis zur Oberkante des bis dahin verpressten Teilformteils, weshalb eine weitere Ausführungsform dies vorschlägt.

Eine Ausführungsform erreicht diese weitere Absenkung dadurch, dass der obere Stempel ebenfalls als mehrteiliger Stempel ausgeführt ist, und der obere Außenstempel gegenüber dem oberen Innenstempel nach unten verschoben ist, sich aber durch Druck von unten wieder nach oben verschieben lässt, bis eine vordefinierte Verschiebung gegenüber dem oberen Innenstempel erreicht ist, die null sein kann, aber auch leicht negativ, beispielsweise um den vorab beschriebenen besseren Abschluss des Füllraumes beim Pressschritt zu realisieren (siehe Zeichnung 37). Die Federkraft, mit der der obere Außenstempel nach unten gedrückt wird, ist vorzugsweise höher als diejenige Federkraft, welche den unteren Außenstempel nach oben drückt, so dass der untere Außenstempel durch den oberen Außenstempel so weit nach unten gedrückt werden kann, dass beispielsweise beim zweiten Pressschritt ein Einrasten in etwa auf Höhe des unteren Innenstempels oder auch darunter erreicht werden kann. Vorzugsweise ist sie aber geringer als der Pressdruck beim letzten Pressschritt, so dass die erwähnte vordefinierte Verschiebung gegenüber dem Innenstempel erreicht ist, bevor das Formteil in seine endgültige Form gepresst ist.

Eine Ausführungsform erreicht diese weitere Absenkung dadurch, dass der obere Stempel ebenfalls als mehrteiliger Stempel ausgeführt ist, und der obere Innenstempel gegenüber dem oberen Außenstempel durch Druck von unten um ein bestimmtes Maß nach oben verschiebbar ist (siehe Zeichnung 38). Hierdurch kann die Unterkante des oberen Außenstempels beim Pressschritt um diese Maß weiter nach unten kommen als die Unterkante des oberen Innenstempels und dabei den unteren Außenstempel weiter nach unten drücken.

Die Verschiebbarkeit des oberen Innenstempels nach oben kann gemäß einer weiteren Ausführungsform durch einen Rastmechanismus oder ähnliches blockiert werden, so dass beim letzten Pressschritt mit unverschobenem oberen Stempel gepresst werden kann. Ein entsprechender Rastmechanismus kann ähnlich realisiert werden wie die Rastmechanismen bei den unteren Stempeln.

Eine weitere Ausführungsform erreicht die weitere Absenkung des unteren Außenstempels dadurch, dass der obere Teil eines mindestens zweiteiligen unteren Innenstempels gegenüber seinem unteren Teil auf Druck von oben um ein bestimmtes Maß nach unten verschiebbar ist (siehe Zeichnungen 28-36). Dies kann beispielsweise durch Verwendung einer Feder (Element 14) und zweier Anschläge (Element 15 und die von oben und unten daran anschlagenden Teile des oberen Teils des unteren Innenstempels) realisiert sein. Durch die Verschiebbarkeit unter Druck von oben kann der Außenstempel durch einen planen oberen Stempel weiter nach unten verschoben werden, da durch die Verschiebbarkeit auch der obere Teil des Innenstempels und somit die Oberkante des Teilformteils weiter nach unten verschoben werden kann. Nachdem der Rastmechanismus eingerastet ist und den Außenstempel gegenüber dem unteren Teil des Innenstempels (oder bei alternativen Ausführungsformen gegenüber der Matrize oder der Matrizenscheibe) fixiert hat, wird der obere Teil des Innenstempels bei der folgenden Anhebung des Oberstempels durch die Feder wieder nach oben verschoben. Wird das Maß dieser Verschiebung entsprechend der Höhe des Teilformteils (und der mechanischen Hysterese des Rastmechanismus) eingestellt, befinden sich danach die Oberkanten des Außen- und des Innenstempels auf der gewünschten relativen Höhe zueinander, so dass beim nächsten Füllschritt das Rohmaterial für die Seitenwände auch neben dem Kern eingefüllt werden kann. Die Federkraft, mit welcher der obere Teil des unteren Innenstempels nach oben gedrückt wird, ist vorzugsweise ähnlich der Federkraft, mit welcher der Außenstempel (bzw. dessen oberer Teil) nach oben gedrückt wird, wenn seine Oberfläche mit der des Innenstempels auf einer Ebene liegt. Hierdurch ergeben sich beim letzten Pressschritt eine möglichst gleichmäßige Absenkung beider unteren Teilstempel und eine möglichst ebene Unterseite des Formteils. Bei Ausführungsformen, bei denen der Außenstempel vor dem letzten Füllschritt etwas tiefer einrastet als der Innenstempel, beispielsweise um mehr Rohmaterial für die Seitenwände einfüllen zu können, werden beide Federkräfte und Federcharakteristiken (z.B. Progressivität) vorzugsweise so gewählt, dass beide unteren Teilstempel ihre endgültige relative Position zueinander eingenommen haben, bevor die Seitenwände um mehr als die Quadratwurzel ihres finalen Komprimierungsfaktors verpresst sind. Wird das Material der Seitenwände beispielsweise um einen Faktor von 1,44:1 komprimiert, sollten beide unteren Teilstempel ihre endgültige relative Position zueinander eingenommen haben, bevor die Seitenwände um mehr als Faktor 1,2:1 verpresst sind.

Eine weitere Ausführungsform erreicht die weitere Absenkung dadurch, dass der Rastmechanismus den Außenstempel gegenüber der Matrize fixiert. Wird der Innenstempel vor oder beim zweiten Pressschritt um etwa die Höhe des dabei erzeugten Teilformteils weiter abgesenkt, als es für den nächsten Füllschritt erforderlich ist (ggf. noch etwas weiter um die mechanischen Hysterese des Rastmechanismus zu kompensieren), kann der Außenstempel durch den Rastmechanismus in der gewünschten Position fixiert werden. Durch die anschließende Anhebung des Innenstempels um das entsprechende Maß befinden sich die Oberkanten des Außen- und des Innenstempels auf der gewünschten relativen Höhe zueinander, so dass beim nächsten Füllschritt das Rohmaterial für die Seitenwände auch neben dem Kern eingefüllt werden kann.

Eine weitere Ausführungsform erreicht die weitere Absenkung dadurch, dass der Rastmechanismus den Außenstempel nur temporär gegenüber der Matrize fixiert. Bei dieser Ausführungsform wird der Innenstempel vor oder beim zweiten Pressschritt um etwas mehr als die Höhe des dabei erzeugten Teilformteils weiter abgesenkt, als es für den nächsten Füllschritt erforderlich ist, wobei der Außenstempel dann gegenüber der Matrize fixiert wird. Bei der anschließenden Anhebung des Innenstempels rastet der Außenstempel nun in der gewünschten Position gegenüber dem Innenstempel ein, wobei vorzugsweise gleichzeitig die Rastung gegenüber der Matrize gelöst wird. Diese Ausführungsform hat den Vorteil, dass die Position des unteren Stempels beim zweiten Füllschritt nicht durch die Fixierung des Außenstempels an der Matrize eingeschränkt ist.

Bei den Ausführungsformen mit Rastmechanismus wird der eingerastete Rastmechanismus vorzugsweise nach dem letzten Pressschritt oder bei oder nach dem Ausstoßschritt gelöst (ausgerastet). Dies kann beispielsweise durch Betätigungselemente geschehen, welche an oder in der Nähe der Stempelführungen angebracht sind, und die beim Passieren der Stempel den Rastmechanismus lösen.

Bei einer weiteren Ausführungsform wird der Rastmechanismus gelöst, wenn der untere (Außen-)Stempel weiter in die Matrize eingedrungen ist, als er beim dritten Füll- und Pressschritt eindringt. Vorzugsweise dann, wenn er so weit in die Matrize eindringt, dass seine Oberkante mindestens die Höhe der Oberkante der Matrize erreicht (wie beim Ausstoßvorgang der Fall).

Eine Variante dieser Ausführungsform löst den Rastmechanismus erst dann, wenn der Stempel nach dem Ausstoßvorgang wieder abgesenkt wird, damit das Formteil nicht durch die plötzlich wieder auf den Außenstempel einwirkenden Federkräfte zu hoch ausgestoßen wird.

Bei einer weiteren Ausführungsform wird der Rastmechanismus gelöst, wenn der untere Stempel nach dem vorherigen Einrasten des Rastmechanismus erneut mit einem Druck von oben belastet wird, der ein bestimmtes Maß übersteigt. Beispielsweise wird bei der Ausführungsform mit dem mindestens zweigeteilten unteren Innenstempel, dessen Oberteil federnd gelagert ist, der Rastmechanismus gelöst, wenn der Außenstempel gegenüber dem unteren Innenstempel erneut um ein bestimmtes Maß abgesenkt wird (z.B. durch einen Push-Push-Rastmechanismus ähnlich wie vorstehend beschrieben).

Bei einer weiteren Ausführungsform wird der Rastmechanismus gelöst, wenn der untere Stempel um ein bestimmtes Maß nach unten abgesenkt wird. Dies kann vorteilhaft damit verbunden werden, dass der untere Stempel nach dem Ausstoßvorgang so weit nach unten abgesenkt wird, dass seine Oberkante unter die Unterkante der Matrize abgesenkt wird, damit die Stempeloberfläche durch Bürsten, Abblasen oder sonstige Reinigungsvorgänge von restlichem Rohmaterial gereinigt werden kann. Beispielsweise kann der Rastmechanismus dadurch gelöst werden, dass ein Hebel ausgelöst wird, wenn dieser beim Absenken des Stempels gegen die Lagerung der Stempel bewegt wird.

Bei einer weiteren Ausführungsform wird der Außenstempel beim letzten Füllschritt gegenüber dem Innenstempel so weit verschoben, dass seine Oberkante unter dessen Oberkante liegt. Hierdurch kann mehr Rohmaterial für die Seitenwände eingefüllt werden. Dies ist vorteilhaft, wenn der geringeren Komprimierbarkeit des vorkomprimierten Kerns Rechnung getragen und/oder eine stärkere Komprimierung der Seitenwände erreicht werden soll. Beim nachfolgenden Pressschritt wird der Außenstempel relativ zum Innenstempel dann wieder soweit angehoben, dass die Oberkante des Innenstempels und die des Außenstempels in einer Ebene liegen, so dass das Formteil eine ebenmäßige Unterseite erhält. Die relative Anhebung des Außenstempels kann auch durch eine relative Absenkung des Innenstempels erreicht werden. Bevorzugte Maßnahmen hierzu, wie beispielsweise die Auswahl der Federkräfte, sind in vorab beschriebenen Ausführungsformen beschrieben.

Bei einer weiteren Ausführungsform ist der Außenstempel gegen Innenstempel auch weiter absenkbar als es für den letzten Press- und Füllschritt erforderlich ist, allerdings nur bei einem Druck, der höher ist, als der Pressdruck beim letzten Pressschritt. Dies kann dadurch realisiert werden, dass der Anschlag, der die Verschiebbarkeit des Außenstempels gegenüber dem Innenstempel nach unten begrenzt, ebenfalls federnd gelagert ist, allerdings mit einer entsprechend hohen Federkraft. Durch eine solche Ausführungsform kann beispielsweise ein Ausstoßschritt realisiert werden, bei dem das Formteil durch Anhebung der Oberkante des unteren Innenstempels über die Oberkante der Matrize hinaus aus der Matrize ausgestoßen wird, obwohl die Maximalhöhe der Oberkante des Außenstempels, beispielsweise für die ersten Füllschritte, auf die Höhe der Oberkante der Matrize limitiert ist.

Bei Ausführungsformen, bei denen der Außenstempel vor dem letzten Füllschritt etwas tiefer einrastet als der Innenstempel (bemessen an deren Oberkanten), beispielsweise um mehr Rohmaterial für die Seitenwände einfüllen zu können, beträgt dieses Maß, um den der Außenstempel tiefer einrastet, bevorzugt maximal 40% der Gesamthöhe des fertigen Formteils, mehr bevorzugt maximal 20% der Gesamthöhe des fertigen Formteils, noch mehr bevorzugt maximal 10% der Gesamthöhe des fertigen Formteils.

Je nach Elastizität und Plastizität der verwendeten Pulvermischungen oder Granulate kann es zu einer mehr oder weniger starken Haftung des Formteils oder der Teilformteile an der Matrizenwand bzw. der Innenwand des unteren Außenstempels kommen. Dies kann insbesondere dann nachteilig sein, wenn dis beim ersten Pressschritt auftritt, bzw. bei Pressschritten, bei denen der untere Außenstempel nach unten bewegt wird und nach denen er wieder nach oben bewegt wird, weil dann evtl. das Teilformteil mit dem unteren Außenstempel ebenfalls nach oben bewegt wird und beim nächsten Füllschritt der Füllraum nicht korrekt befüllt werden kann.

Um dies zu vermeiden wird in einer weiteren Ausführungsform bei solchen Pressschritten ein möglichst geringer Pressdruck verwendet, so dass zwar eine Einebnung des bereits eingefüllten Materials erfolgt um eine Vermischung mit dem danach einzufüllenden Material zu vermeiden, das bereits eingefüllte Material jedoch nicht so stark an die Innenwand des unteren Außenstempels angepresst wird, dass sich das Teilformteil bei der nachfolgenden Aufwärtsbewegung des unteren Außenstempels mit diesem nach oben bewegt.

In einer weiteren Ausführungsform wird auf einen oder mehrere Pressschritte verzichtet, und zwar auf Pressschritte, bei denen der untere Außenstempel nach unten bewegt und nach denen er wieder nach oben bewegt werden würde. Zum Beispiel wird auf die Verpressung der ersten Schicht des Hüllmaterials verzichtet.

Eine weitere Ausführungsform der Erfindung sieht vor, dass ein oder mehrere Zwischenstempel (38) eingesetzt werden. Ein Zwischenstempel ist ein Stempel, der zwischen dem oberen und dem unteren Stempel eingefügt wird, vorzugsweise oberhalb der Matrize bzw. Matrizenscheibe.

In einer Ausführungsform weist zumindest ein Zwischenstempel an seinem unteren Ende eine Form auf, die in ihren lateralen Abessungen im Wesentlichen dem oberen Ende des unteren Innenstempels entspricht, so dass er bei einer Abwärtsbewegung ein innerhalb des unteren Außenstempels eingefülltes Material gegen den unteren Innenstempel verpressen kann, ohne dass der untere Außenstempel dabei nach unten bewegt werden muss.

An seinem oberen Ende weist der Zwischenstempel eine Struktur auf, die vorzugsweise dazu geeignet ist, dass der obere Stempel bei einer Abwärtsbewegung auf diese aufsetzt und bei einer weiteren Abwärtsbewegung den Zwischenstempel nach unten drückt.

Die Struktur kann auch so ausgestaltet sein, dass der obere Stempel zumindest teilweise in sie eindringt, beispielsweise um eine bessere Führung des Zwischenstempels zu ermöglichen.

Diese Struktur kann aus einer ebenen Fläche bestehen. Vorzugsweise ist sie an die Geometrie des oberen Stempels angepasst. Beispielsweise kann die obere Struktur des Zwischenstempels in dem Bereich, in dem der obere Stempel auf ihr aufsetzt, konvex ausgeführt sein, wenn der obere Stempel eine konkave Pressfläche aufweist.

Diese Struktur kann aus einem Material bestehen, das weicher ist, als der obere Stempel, oder mit solch einem Material beschichtet sein, damit die Pressfläche des oberen Stempels beim Auftreffen auf die Struktur möglichst nicht beschädigt wird.

Die vertikale Kraftübertragung vom oberen Stempel zum Zwischenstempel kann auch durch andere Strukturen als die Pressfläche des oberen Stempels erfolgen, beispielsweise durch Nuten, Federn, Stege und andere Arten der mechanischen Kopplung stattfinden. Hierdurch kann beispielsweise die Stempelfläche des Oberstempels geschont werden, da sie nicht in Kontakt mit dem Zwischenstempel kommen muss.

Bei einer weiteren Ausführungsform wird hierzu (zur vertikalen Kraftübertragung) die Ringförmige horizontale Fläche (Zeichnungen 39 und 40, 30, Ansicht von unten gesehen) verwendet, die an der Stelle auftritt, an welcher der obere Stempel eine stufige Verkleinerung seines Durchmessers aufweist (Zeichnungen 39 und 40, 31). Oberhalb dieser Verkleinerung des Durchmessers weist der Stempel einen größeren Durchmesser auf, der in etwa dem Durchmesser der Führungshülsen der Stempelhalterung entspricht. Dieser Teil entspricht dem Führungsschaft (Zeichnungen 39 und 40, 29, Ansicht des oberen Stempels im Schnitt von der Seite gesehen)

Unterhalb dieser Verkleinerung des Durchmessers weist der Stempel einen kleineren Durchmesser auf, der in etwa dem Durchmesser des zu produzierenden Formteils entspricht.

Der Zwischenstempel (Zeichnung 40, 38) weist am oberen Ende eine rohrförmige Struktur auf, in deren Hohlraum der obere Stempel mit seiner Pressfläche (Zeichnungen 39 und 40, 28) und dem entsprechend dimensionierten unteren Teil eintaucht, und auf deren oberer Ringförmigen Fläche (Zeichnung 40, 33, Ansicht von oben gesehen) die Ringförmige Fläche des oberen Stempels aufsetzt (Zeichnung 40, Ansicht 26, Schnitt von der Seite, oberer Stempel sitzt auf Zwischenstempel auf).

Bei einer weiteren Ausführungsform weist die röhrförmige Struktur eine seitliche Öffnung auf (Zeichnung 41, 34, Ansichten von unten, von der Seite und von oben). Vorzugsweise besteht diese seitliche Öffnung in einem vertikalen Schlitz, der sich bis zur ringförmigen oberen Fläche erstreckt und der eine Breite aufweist, die mindestens so groß ist, wie der Durchmesser des unteren, verjüngten Teils des oberen Stempels.

Hierdurch ist es möglich, den Zwischenstempel von der Seite her unter der ringförmigen Fläche des oberen Stempels zu positionieren, ohne den oberen Stempel so weit anheben zu müssen, dass seine Pressfläche vollständig oberhalb des Zwischenstempels sein muss.

Bei Ausführungsformen mit oder ohne seitlicher Öffnung kann die rohrförmige Struktur an ihrem unteren Ende vorzugsweise verjüngt ausgeführt sein, insbesondere konisch zulaufend nach unten hin verjüngend (Zeichnung 42, 35). Besonders bevorzugt erfolgt dabei ab dem Erreichen des Außendurchmessers des unteren Endes des oberen Stempels keine weitere Verjüngung.

Vorzugsweise befindet sich die Pressfläche des oberen Stempels, bei der Positionierung des Zwischenstempels unterhalb des oberen Stempels von der Seite her, oberhalb der Stelle mit der größten Verjüngung und nach der Absenkung des oberen Stempels bis zum Kontakt beider ringförmiger Flächen unterhalb der Stelle mit der größten Verjüngung.

Besonders bevorzugt erstreckt sich eine vorhandene seitliche Öffnung nicht bis unterhalb der Stelle mit der größten Verjüngung. Bei solchen Ausführungsformen kann eine besonders gute Zentrierung von oberem Stempel und Zwischenstempel erreicht werden.

Ist ein Zwischenstempel unterhalb des oberen Stempels eingefügt, wird er bei einer Abwärtsbewegung des oberen Stempels durch diesen nach unten gedrückt und ebenfalls abwärts bewegt. Er taucht dann in die Matrizenöffnung ein und kann das dort befindliche Material unterhalb seiner Pressfläche (z.B. dargestellt in Zeichnung 40, 27, Ansicht von unten gesehen) verpressen. Bei oder nach einer Aufwärtsbewegung des oberen Stempels wird auch der Zwischenstempel wieder nach oben bewegt.

Dies kann durch eine in der Senkrechten federnde Lagerung/Halterung (21 und 22) des Zwischenstempels erfolgen oder durch andere geeignete Maßnahmen wie beispielsweise durch einen Pneumatik- oder Hydraulikzylinder, eine Steuerkurve ähnlich der Kurve für die oberen und unteren Stempel.

Ein Zwischenstempel wird vorzugsweise nur für bestimmte Teilschritte unterhalb des oberen Stempels eingefügt.

Zeichnungen 30 und 31 zeigen die Anordnung des Zwischenstempels (38) unterhalb des oberen Stempels (2) in seiner Lagerung/Halterung (21), in der eine Federung (22) den Zwischenstempel oben hält, wenn er nicht durch den oberen Stempel nach unten gedrückt wird, bzw. den Zwischenstempel wieder anhebt, nachdem er vom oberen Stempel nach unten gedrückt worden war und der obere Stempel keinen Druck mehr auf den Zwischenstempel ausübt. Hier wird der Pressschritt der ersten Lage des Hüllmaterials dargestellt.

Die Mechanik zur Positionierung der Lagerung/Halterung des Zwischenstempels ist in diesen Zeichnungen nicht dargestellt. Sie kann beispielsweise wie vorstehend beschrieben mittels Rotor, Kette oder auf andere Art und Weise ausgeführt werden.

Vorzugsweise wird ein Zwischenstempel unterhalb des oberen Stempels eingefügt, wenn die erste Schicht des Hüllmaterials verpresst werden soll.

Bei einer weiteren Ausführungsform wird ein Zwischenstempel unterhalb des oberen Stempels eingefügt, wenn die erste Schicht des Hüllmaterials verpresst werden soll und wenn eine oder mehrere Schichten aus Kernmaterial verpresst werden sollen und wenn zwischen diesen Schichten weitere Schichten verpresst werden sollen.

Bei einer weiteren Ausführungsform wird ein Zwischenstempel unterhalb des oberen Stempels eingefügt, wenn nur Material innerhalb des unteren Außenstempels verpresst werden soll.

Bei einer weiteren Ausführungsform wird ein Zwischenstempel unterhalb des oberen Stempels immer dann eingefügt, wenn nur Material innerhalb des unteren Außenstempels verpresst werden soll, außer beim letzten dieser Pressschritte. Dadurch kann bei diesem Schritt erreicht werden, dass der untere Außenstempel beim Pressschritt ebenfalls nach unten bewegt wird. Hierdurch kann auf separate Führungsbahnen für den unteren Außenstempel verzichtet werden.

Bei weiteren Ausführungsformen wird ein Zwischenstempel so ausgeführt, dass seine Pressfläche (die lateralen Abmessungen) den gemeinsamen Pressflächen von unterem Innen- und Außenstempel entspricht und der obere Stempel so ausgeführt, dass dessen Pressfläche der Pressfläche des unteren Innenstempels entspricht. Bei diesen Ausführungsformen wird dieser Zwischenstempel nur bei den Schritten eingefügt, bei denen bei den vorstehend beschriebenen Ausführungsformen kein Zwischenstempel eingefügt wird. Im Prinzip sind hierbei die Pressflächenabmessungen und Pressschritte von oberem Stempel und Zwischenstempel vertauscht. Dies kann besonders dann vorteilhaft sein, wenn eine größere Anzahl von Pressschritten bei angehobenem unterem Außenstempel durchgeführt werden soll.

Bei weiteren Ausführungsformen werden für verschiedene Pressschritte mehrere unterschiedliche Zwischenstempel verwendet. Beispielsweise wird zum Verpressen der ersten Schicht des Hüllmaterials ein Zwischenstempel verwendet, der eine konvexe Pressfläche aufweist, die einen ähnlichen oder etwas kleineren Wölbungsradius aufweist, wie die konkave Pressfläche des unteren Innenstempels. Zum Verpressen des Kernmaterials wird ein Zwischenstempel verwendet, der eine konkave Pressfläche aufweist, die einen ähnlichen oder etwas kleineren Wölbungsradius aufweist, wie die konkave Pressfläche des oberen Stempels. Vorzugsweise setzt sich die konkave Wölbung des unteren Innenstempels in der Pressfläche des unteren Außenstempels fort. Durch solch eine Ausführungsform, bei der die Pressfläche des unteren Außenstempels nicht plan ist, sondern nach außen hin ansteigt, kann erreicht werden, dass das Hüllmaterial, das um den Kern eingefüllt wird, beim Verpressen durch die nach außen ansteigende Pressfläche des unteren Außenstempels gegen das vorgepresste Hüllmaterial der ersten Schicht gedrückt wird und so ein besserer Einschluss des Kernmaterials erreicht wird.

Die Einfügung bzw. Positionierung des Zwischenstempels unterhalb des oberen Stempels erfolgt vorzugsweise durch eine laterale Bewegung des Zwischenstempels.

Beispielsweise wird bei einer Formpresseinrichtung, bei der oberer und unterer Stempel nicht lateral bewegt werden, z.B. einer Excenterpresse (Korsch XP1) gegenüber dem Füllschuh, der zum Einfüllen des zu verpressenden Materials lateral über die Matrizenöffnung bewegt und danach wieder zurück bewegt wird, eine Vorrichtung angeordnet, die ähnlich der Bewegung des Füllschuhes den Zwischenstempel über die Matrizenöffnung positioniert, wo dieser dann vom oberen Stempel nach unten bewegt werden kann. Vorzugsweise ist der Zwischenstempel nach oben federnd (bedeutet, dass die Lagerung eine Federkraft auf den Zwischenstempel ausübt, die ihn nach oben drückt) in einer Halterung gelagert, die mit dem Zwischenstempel über die Matrizenöffnung bewegt wird. Eine entsprechende Halterung (21) mit Zwischenstempel (38) zeigen die Zeichnungen 30 und 31. Nachdem der obere Stempel und der Zwischenstempel wieder angehoben sind, wird der Zwischenstempel dann wieder von der Matrizenöffnung weg bewegt, vorzugsweise zusammen mit seiner Halterung.

Statt einer linearen Bewegung des Zwischenstempels und seiner Lagerung kann auch eine Bewegung auf einer Teilkreisbahn erfolgen, z.B. wenn Zwischenstempel und Halterung an einem Ausleger montiert sind, der an einer Achse befestigt ist, die parallel zur Matrizenöffnung verläuft. Durch Drehung der Achse oder Drehung des Auslegers um die Achse werden Zwischenstempel und Lagerung lateral unter den oberen Stempel oder wieder darunter weg bewegt.

Bei Formpresseinrichtungen, bei denen oberer und unterer Stempel auch lateral bewegt werden, wie beispielsweise bei einer Rundlauf- oder Rundläuferpresse, bei der die laterale Bewegung vorzugsweise einer Kreisbahn entspricht, werden der oder die Zwischenstempel vorzugsweise über einen Teilkreis dieser Kreisbahn mit den oberen und unteren Stempeln unterhalb des entsprechenden oberen Stempels positioniert und dort synchron mitgeführt.

Während der Bewegung über diesen Teilabschnitt der Kreisbahn erfolgen dann vorzugsweise die Absenkung des oberen Stempels und damit auch eine Absenkung des entsprechenden Zwischenstempels und eine Verpressung des innerhalb der Matrizenöffnung, bzw. innerhalb des unteren Außenstempels befindlichen Materials.

Auch bei dieser Positionierung unterhalb des oberen Stempels ist der Zwischenstempel vorzugsweise nach oben federnd in einer Halterung gelagert, die ebenfalls synchron mitgeführt wird.

Die synchrone Mitführung des Zwischenstempels und/oder seiner Lagerung über einen bestimmten Teilabschnitt (Teilkreis) der Kreisbahn von oberem und unterem Stempel (Stempelkreisbahn) kann über verschiedene Ausgestaltungen erreicht werden.

In manchen Ausführungsformen erfolgt die synchrone Mitführung über eine Art Kette, wie sie auch in EP2165826A2 zur Anwendung kommt um die dort verwendeten Halter für die Tablettenkerne (dort bezeichnet als "core retention elements" 52) synchron mitzuführen. In EP2165826A2 werden zwar keine Zwischenstempel, wie sie in der vorliegenden Erfindung verwendet werden, synchron mit den oberen und unteren Stempeln mitgeführt, sondern "core retention elements", die jedoch zur Ausführung der vorliegenden Erfindung durch Kernstempel mit Lagerungen ersetzt werden können, so dass nach einem entsprechenden Austausch im Prinzip die in EP2165826A2 beschriebene Konstruktion der Mitführung auch für die vorliegende Erfindung verwendet werden kann.

Auf EP2165826A2 und die entsprechenden Ausführungen darin wird hiermit ausdrücklich Bezug genommen. Insbesondere wird Bezug genommen auf die Absätze [0035] bis [0037] und [0041] bis [0043], sowie die darin benannten Zeichnungen.

Vorzugsweise wird der dort beschriebene "core push pin" (58) durch einen Zwischenstempel ersetzt. Der dort beschriebene "core holder" (57) wird vorzugsweise durch eine nach oben federnde Lagerung für den Zwischenstempel ersetzt. Diese kann beispielsweise aus einem Stapel Tellerfedern bestehen. Die äußere Form des "core retention elements" kann dabei weitgehend beibehalten werden, so auch das "transfer cog" (56), das zur synchronen Mitführung des Zwischenstempels mit dem oberen Stempel geeignet ist.

Vorzugsweise ist das "transfer cog" an der Unterseite jedoch nicht komplett offen, sondern weist dort lediglich eine Öffnung auf, die geringfügig größer als der untere Teil (der Schaft am unteren Ende) des Zwischenstempels ist, so dass dieser darin lateral geführt wird. Die ringförmigen Fläche der Unterseite des "transfer cog", welche die Öffnung aufweist, in welcher der untere Teil des Zwischenstempels geführt wird, dient vorzugsweise auch als Widerlager der nach oben federnden Lagerung des Zwischenstempels. Eine entsprechende Zeichnung solch einer Ausführungsform sieht aus wie Zeichnung 9 der EP2165826A2, außer dass:
1. der zylindrische "core holder" (57) durch einen zylindrischen Tellerfederstapel ersetzt ist und
2. das "transfer cog" an seiner Unterseite eine Fläche aufweist, auf welcher der Tellerfederstapel aufliegt, wobei die Fläche auf der Unterseite des "transfer cog" eine Öffnung aufweist, die geringfügig größer ist, als der untere Teil des Zwischenstempels, so dass dieser hindurchragen kann und
4. der "core push pin" durch den Zwischenstempel ersetzt ist, der in etwa auch die Form des "core push pin" aufweist, an seiner Unterseite jedoch eine Pressfläche aufweist, mit welcher Hüll- und/oder Kernmaterial verpresst werden kann.

In weiteren Ausführungsformen erfolgt die synchrone Mitführung über einen Rotor, wie er auch in EP0349777A1 zur Anwendung kommt (dort mit der Nummer 10 gekennzeichnet) um die dort verwendeten Kernstempel mit den durch sie gehaltenen Tablettenkernen synchron mitzuführen. In EP0349777A1 werden zwar keine Zwischenstempel, wie sie in der vorliegenden Erfindung verwendet werden, synchron mit den oberen und unteren Stempeln mitgeführt, sondern Übergabeköpfe (8) mit Kernstempeln, die jedoch durch Kernstempel mit Lagerungen ersetzt werden können, so dass nach einem entsprechenden Austausch im Prinzip die in EP0349777A1 beschriebene Konstruktion der Mitführung auch für die vorliegende Erfindung verwendet werden kann.

Auf EP0349777A1 und die entsprechenden Ausführungen darin wird hiermit ausdrücklich Bezug genommen. Insbesondere wird Bezug genommen auf die Beschreibung von Seite 3, Spalte 3, Zeile 25 bis Seite 4, Spalte 5, Zeile 24, Seite 4, Spalte 6, Zeilen 1 bis 37, Seite 5, Spalte 7, Zeile 42 bis Seite 5, Spalte 8, Zeile 20.

Ähnlich wie bei der zweiten Ausführungsform, die in EP0349777A1 beschrieben ist (Seite 5, Spalte 8, Zeile 21 bis Seite 7, Spalte 11, Zeile 44), können Zwischenstempel auch über die Absenkung ihrer Halterungen nach unten bewegt werden, wie es in EP0349777A1 mit den Zylinderkolbeneinheiten (111) realisiert ist. Insbesondere wenn keine hohen Presskräfte gefordert sind, wie es z.B. beim Verpressen der ersten Schicht aus Hüllmaterial der Fall sein kann, stellt dies eine praktikable Ausführungsform dar. Vorzugsweise wird die synchrone Mitführung auch über mehr als zwei benachbarte Matrizen ausgeführt.

Im Prinzip können beide Ausführungsformen der EP0349777A1 abgewandelt werden, so dass mit ihnen Ausführungsformen der vorliegenden Erfindung ausgeführt werden können.

Die Kernstempel werden durch Zwischenstempel ersetzt, die an ihrer Unterseite Pressflächen aufweisen, mit denen Hüll- und/oder Kernmaterial verpresst werden kann.

Auf die in EP0349777A1 ausgeführte Vakuumtechnik kann hierbei verzichtet werden, da keine Kerne zugeführt werden müssen. Ebenso kann die Führungseinrichtung entfallen.

Statt Tablettenkerne in die Matrizenöffnungen einzufüllen, wird durch die Abwärtsbewegung der synchron mitgeführten Zwischenstempel ein zuvor in die Matrizenöffnung bzw. innerhalb des unteren Außenstempel eingefülltes Material verpresst.

Eine weitere Möglichkeit, einen oder mehrere Zwischenstempel mit einem oder mehreren Oberstempeln synchron mitzuführen, besteht darin, die Halterung oder die Halterungen für den oder die Zwischenstempel an der Halterung oder den Halterungen für die Oberstempel oder die Unterstempel oder an der Matrize oder ihrer Halterung zu befestigen. Hierdurch bewegen sich der oder die Zwischenstempel grundsätzlich synchron zu den Oberstempeln.

Über eine Möglichkeit, einen Zwischenstempel seitlich zu bewegen, kann er bei Bedarf unterhalb des Oberstempels positioniert werden oder seitlich daneben (bei einer Rundlaufpresse beispielsweise außerhalb oder innerhalb des vom Oberstempel beschriebenen Umlaufkreises oder im Zwischenraum zwischen zwei benachbarten Oberstempeln).

Eine bevorzugte Ausführungsform realisiert dies, indem am Umfang der Scheibenförmigen Oberstempelführung vertikal ausgerichtete Profile angebracht sind, die sich nach unten bis auf die Höhe des Zwischenraumes zwischen der Matrize und der Pressfläche der angehobenen Oberstempel erstrecken. Vorzugsweise wird pro Oberstempel ein solches Profil angebracht.

Von unten ist an jedem dieser Profile ein drehbar gelagertes, vorzugsweise waagerecht angeordnetes Profil angebracht, wobei vorzugsweise das senkrechte Profil, bzw. die Befestigung am senkrechten Profil, die Drehachse für das waagerechte Profil darstellt. An diesem Profil, vorzugsweise an dessen Ende) ist die Halterung für den Zwischenstempel angebracht, bzw. in dieses eingearbeitet. Durch Drehung des waagerechten Profils kann der Zwischenstempel unter den Oberstempel eingeschwenkt oder herausgeschwenkt werden. Vorzugsweise ist die Drehbewegung durch Anschläge limitiert, beispielsweise auf einen Winkelbereich, der 60 Grad umfasst. Vorzugsweise wird das waagerecht angeordnete Profil durch eine Feder in einer der beiden Positionen (eingeschwenkt oder ausgeschwenkt) fixiert, so dass zum Wechseln der Positionen nur eine Krafteinwirkung in eine Richtung (oder eben das Ausbleiben dieser Krafteinwirkung) erforderlich ist.

Die Positionierung (ein- oder ausgeschwenkt) kann auf verschiedene Arten erfolgen. Beispielsweise kann das Profil als L-förmiger Winkel ausgeführt sein, dessen einer Schenkel die Halterung des Zwischenstempels trägt und dessen anderer Schenkel (Führungsschenkel) in einem Winkel von etwa 120 Grad zum ersten Schenkel steht. Wird das Profil durch die Feder in der ausgeschwenkten Position fixiert, ist der Schenkel mit der Halterung für den Zwischenstempel tangential zur Matrizenscheibe ausgerichtet und der andere Schenkel radial nach außen. Durch eine größtenteils kreisförmige Führungsbahn, die auf der Höhe des waagerechten Profils angeordnet ist, und einen nominalen Durchmesser aufweist, der um die Länge des Führungsschenkels größer ist, als der Durchmesser des Umlaufkreises der senkrechten Profile, und deren Durchmesser an bestimmten Stellen verkleinert ist, kann das waagerechte Profil für einen Teilkreis des Umlaufkreises in die eingeschwenkte Position gedreht werden (Zeichnung 46).

Statt einer Anbringung am Umfang der scheibenförmigen Oberstempelführung ist auch eine Anbringung an der Unterseite der Oberstempelführung möglich, beispielsweise auch weiter innen als die Oberstempel geführt werden.

Die Führungsbahn kann dann ggf. auch weiter innen angebracht werden und stört dann weniger bei der Bedienung der Presse.

Das vorstehend beschriebene waagerechte Profil kann auch an einer drehbaren Welle fixiert sein.

Diese Welle kann beispielsweise durch die vorab beschriebenen senkrechten Profile nach oben geführt sein. Vorzugsweise wird die Position (eingeschwenkt oder ausgeschwenkt) durch Drehung dieser Welle bestimmt. Die Drehung kann beispielsweise durch eine Führungsbahn bestimmt werden, die sich vorzugsweise oberhalb der Oberstempelführung befindet.

Eine Führung der Welle nach unten ist natürlich ebenso möglich. Sie kann auch durch die Matrizenscheibe hindurch geführt sein. Vorzugsweise wird sie dann entsprechend einer Führungsbahn gedreht, die sich unterhalb der Matrizenscheibe befindet.

Alternativ kann statt eines drehbar gelagerten Profils auch ein radial verschiebbar gelagertes Profil verwendet werden, das dann beispielsweise eine gerade Form aufweist.

In einer weiteren Ausführungsform werden waagerechte Profile radial unterhalb der Oberstempelführung oder oberhalb des Matrizentisches bzw. der Matrizenscheibe angebracht, vorzugsweise ein Profil pro Oberstempel. Diese Profile rotieren mit der Oberstempelführung oder dem Matrizentisch bzw. der Matrizenscheibe um die vertikale Achse der Rundläuferpresse, sind diesen gegenüber aber radial verschiebbar gelagert.

Die radiale Position der Profile wird durch eine Kurvenbahn bestimmt. Die Kurvenbahn kann auch außerhalb der von den Stempeln beschriebenen Kreisbahn angebracht sein. Vorzugsweise ist die Kurvenbahn innerhalb dieser Kreisbahn angebracht. Die Kurvenbahn ist vorzugsweise stationär, dreht sich also vorzugsweise nicht mit Oberstempelführung oder Matrizentisch bzw. der Matrizenscheibe um die vertikale Achse der Rundläuferpresse. Vorzugsweise ist die Kurvenbahn drehbar gegenüber der vertikalen Achse der Rundläuferpresse gelagert, so dass die laterale Position gegenüber dieser Achse fixiert ist. Eine Mitdrehung mit der Achse wird durch eine Befestigung an einem stationären Bauteil der Presse verhindert. In einer bevorzugten Ausführungsform erfolgt diese Befestigung an einem oder mehreren Füllschuhen, bzw. an dessen Halterung oder deren Halterungen.

Die waagerechten Profile sind vorzugsweise gegenüber dem Matrizentisch/der Matrizenscheibe erhöht angebracht, so dass sie bei der Drehung um die Achse nicht mit dem oder den Füllschuhen oder deren Befestigung kollidieren. Deshalb erfolgt die Befestigung bzw. Lagerung der Profile in einer bevorzugten Ausführungsform an der Oberstempelhalterung.

Durch entsprechende Aufnehmer, welche die Kontur der Kurvenbahn aufnehmen, wird die radiale Position der Kurvenbahn auf die Profile übertragen. Beispielsweise besteht die Kurvenbahn aus einer Scheibe, die an ihrer Oberseite eine umlaufende Nut aufweist, in welche Zapfen eingreifen, die am der Achse zugewandten Ende der Profile befestigt sind. Dadurch, dass der Abstand der Nut zur Achse je nach Winkelposition unterschiedlich ist, werden die Profile entsprechend diesem Abstand radial positioniert. Hierdurch kann ein am der Achse abgewandten Ende des jeweiligen Profils angeordneter Zwischenstempel zwischen Oberstempel und Matrize angeordnet und auch entsprechend wieder aus dieser Position entfernt werden. Bei einer weiteren Ausführungsform sind an dem Profil mehrere Zwischenstempelhalterungen mit Zwischenstempeln angebracht. Somit kann durch den Abstand der Nut zur Achse und daraus folgend die radiale Position des jeweiligen Profils jeweils einer von mehreren vorzugsweise verschiedenen Zwischenstempeln zwischen Oberstempel und Matrize positioniert werden.

In einer weiteren Ausführungsform werden die Profile durch eine entsprechend ausgeführte Kurvenbahn kurz vor der Annäherung an die Füllschuhe bis nach der Passage derselben so weit in Richtung der Achse verschoben, dass eine Kollission mit dem oder den Füllschuhen ausgeschlossen ist. Somit können die waagerechten Profile auch auf dem Matrizentisch/der Matrizenscheibe angebracht werden.

In manchen Ausführungsformen werden die waagerechten Profile in einer Scheibe geführt, welche radiale Nuten oder Schlitze aufweist, in denen die waagerechten Profile geführt werden.

Der Radius der Scheibe ist vorzugsweise nicht größer als der Radius des Raumes zwischen der Achse und jeglichen Bauteilen welche in den Raum innerhalb des Umlaufkreises der Oberstempel hinein ragen.

Der Radius kann auch größer sein, dann aber vorzugsweise nicht größer als der Radius des Raumes zwischen der Achse und jeglichen stationären Bauteilen welche in den Raum innerhalb des Umlaufkreises der Oberstempel hinein ragen, beispielsweise den Füllschuhen oder dem Abstreifer. An den Stellen, an denen mit umlaufende Bauteile weiter in diesen Raum hinein ragen, ist die Scheibe vorzugsweise ausgespart.

Da die Scheibe ebenfalls um die Achse umläuft, kann sie in manchen Ausführungsformen auch mit anderen umlaufenden Bauteilen verbunden werden.

In bevorzugten Ausführungsformen umfasst ein Rotor eine Oberstempelhalterung, ein Mittelteil mit integrierter oder daran angebrachter Matrizenscheibe und eine Unterstempelhalterung.

Oberhalb der Matrizenscheibe weist das Mittelteil pro Stempelpaar zwei übereinander liegende radiale Bohrungen auf. In den Bohrungen werden zwei radiale Teil-Arme geführt, welche radial verschiebbar in den Bohrungen gelagert sind. Die der Achse abgewandten Enden dieser Teil-Arme sind mit einer oder mehreren Zwischenstempelhalterungen verbunden und an dieser vorzugsweise so befestigt, dass sie sich nicht um ihre Längsachse drehen können. Die eine oder mehrere Zwischenstempelhalterungen sind dadurch so fixiert, dass sie nicht um eine ihrer Achsen gekippt werden können und die darin gehaltenen Zwischenstempel immer senkrecht ausgerichtet sind. Sie drehen sich lediglich mit dem Rotor um dessen Achse und sind über die radial verschiebbar gelagerten radialen Teil-Arme ebenfalls radial verschiebbar.

Über Führungselemente, vorzugsweise über Zapfen, die an einem der radialen Teil-Arme befestigt sind, werden die Zwischenstempelhalterungen abhängig von der Position auf ihrem Umlauf um die Achse (Winkelposition) an einer bestimmten radialen Position positioniert. Beispielsweise werden sie direkt unterhalb des jeweiligen Oberstempels positioniert, wenn an dieser Winkelposition ein Zwischenstempel zum Verpressen eingesetzt werden soll. Endsprechend werden sie weiter in Richtung Achse positioniert, wenn an dieser Winkelposition kein Zwischenstempel zum Verpressen eingesetzt werden soll. Sind mehrere Zwischenstempelhalterungen an einem radialen Arm befestigt, wird durch die radiale Positionierung bestimmt, ob ein, und ggf. welcher Zwischenstempel an dieser Winkelposition zum Einsatz kommt.

Vorzugsweise ist zwischen der Matrizenscheibe und der vertikalen Position der unteren Bohrung im Mittelteil ein bestimmter Abstand, so dass zwischen der Matrizenscheibe und dem unteren Teil-Arm Raum für nicht mit dem Rotor umlaufende Einbauten befindet.

Vorzugsweise ist in diesem Raum eine Kurvenscheibe angeordnet. Dies ist beispielsweise eine Scheibe, die gleitend, mit Rollen, Kugeln oder sonstwie gelagert auf der Matrizenscheibe aufliegt, und so befestigt ist, dass sie nicht mit dem Rotor umläuft. Beispielsweise ist die Kurvenscheibe an den Füllschuhen befestigt. Sie kann, statt auf der Matrizenscheibe aufzuliegen, auch in einer umlaufenden Nut des Mittelteils in einer definierten Höhe gehalten werden. Auch kann sie durch eine entsprechende mechanische Konstruktion unterhalb der Oberstempelhalterung gehalten werden.

Diese Kurvenscheibe hat in bestimmten bevorzugten Ausführungsformen auf ihrer Oberseite eine Nut, welche abhängig von der Winkelposition einen Definierten Abstand zur Achse hat. In diese Nut greifen die Führungselemente der radial verschiebbaren Arme. Vorzugsweise bestehen diese Führungselemente aus Zapfen, welche entweder in den radialen Armen drehbar gelagert sind oder an ihrem in die Nut der Kurvenscheibe eintauchenden Ende ein drehbar gelagertes Rad aufweisen (ggf. in Form eines einfachen Kugellagers), so dass die Reibung zwischen den Führungselementen und den Seitenwänden der Nut minimiert wird.

In anderen Ausführungsformen weist die Kurvenscheibe statt einer Nut einen nicht kreisfürmigen Umfang auf. Der Radius der Kurvenscheibe ist dabei abhängig von der Winkelposition. Die Führungselemente greifen bei solchen Ausführungsformen nicht in eine Nut, sondern liegen am Umfang der Kurvenscheibe an. Dazu sind sie ähnlich der vorstehend bewschriebenen Ausführungsformen entweder in den radialen Armen drehbar gelagert oder weisen an ihrem am Umfang der Kurvenscheibe entlang geführten Ende ein drehbar gelagertes Rad auf.

Vorzugsweise ragen die Führungselemente nur zu einem Teil der Stärke der Kurvenscheibe in diese hinein, bzw. werden nur auf einem Teil der vertikalen Umfangsfläche geführt, so dass die Nut nicht so tief sein muss, wie die Kurvenscheibe stark ist, bzw. die vertikale Umfangsfläche nicht auf ihrer gesamten Höhe freigestellt sein muss.

Beispielsweise entspricht die Tiefe der Nut in der Kurvenscheibe zwei Dritteln ihrer Stärke und die Führungselemente tauchen zu einer Hälfte der Kurvenscheibenstärke in die Nut ein.

Vorzugsweise ist die Kurvenscheibe gleitend oder auf andere Weise drehbar gegenüber dem Mittelteil gelagert, so dass sie seitlich nicht gegenüber diesem verschiebbar ist.

Vorzugsweise ist sie zwei- oder mehrteilig ausgeführt. Beispielsweise besteht sie aus zwei halbkreisförmigen Scheiben mit halbkreisförmigen Ausschnitten für das Mittelteil, welche zur Montage von zwei gegenüber liegenden Seiten an das Mittelteil herangeführt und dann aneinander befestigt werden.

Die Kurvenscheibe kann in weiteren Ausführungsformen auch in einem Hohlraum innerhalb des Rotors angeordnet sein. Beispielsweise ist sie zwischen der Achse, um die sich der Rotor dreht, und dem Mittelteil des Rotors angeordnet. Die radialen Arme, an deren der Achse abgewandten Seite die Zwischenstempelhalterung angeordnet ist, werden in radialen Bohrungen durch die Wandung des Rotors in diesen Hohlraum geführt und dort abhängig vom Umfang der Kurvenscheibe, die vorzugsweise an der Achse befestigt ist, radial verschoben. Dazu werden die radialen Arme mithilfe von Federn so weit in Richtung der Achse gedrückt, bis sie dort anstoßen. Vorzugsweise wird eine Reibung der Arme an der Kurvenscheibe durch Stützrollen vermieden.

In weiteren Ausführungsformen weisen die radialen Arme einen nicht kreisförmigen Querschnitt auf. Dadurch sind sie in den Führungsbohrungen auch dann nicht verdrehbar, wenn sie aus einzelnen Profilen bestehen. Somit sind in solchen Ausführungsformen keine zwei oder mehr Teilarme erforderlich.

Dem Fachmann sind auch andere Methoden bekannt, wie er die Arme verdrehsicher ausführen und lagern kann.

Eine solche Ausführungsform der Erfindung ist nachfolgend anhand eines in den Zeichnungen dargestellten Ausführungsbeispieles näher erläutert. Es zeigen:

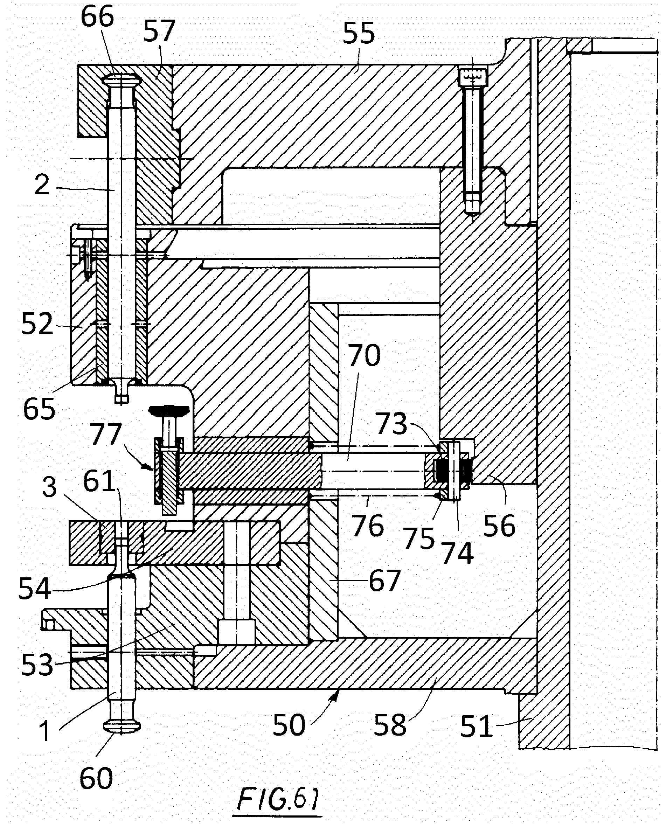

- Fig. 61 einen Vertikalschnitt durch die Formpressvorrrichtung zur Herstellung von Kernformteilen mit zurückgezogenem Zwischenstempel,

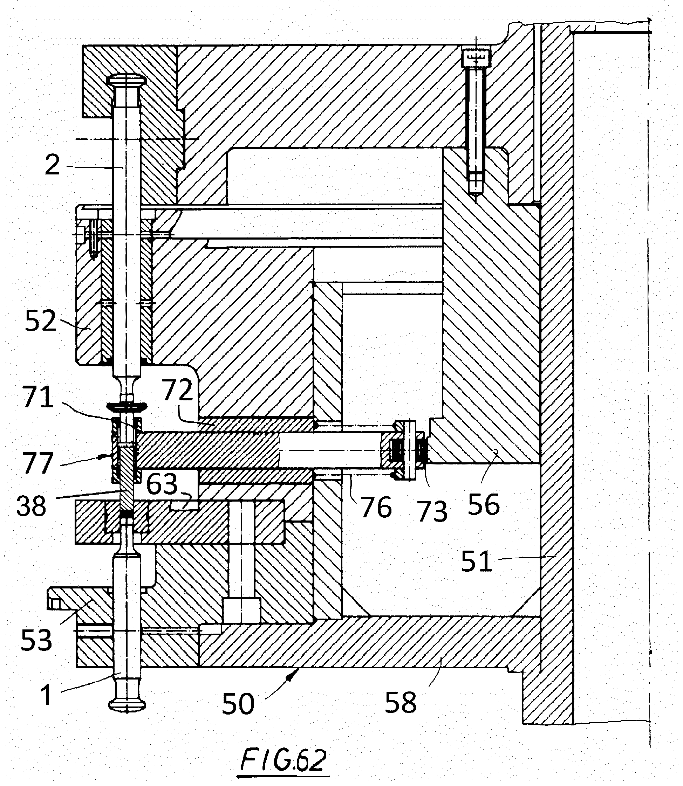

- Fig. 62 einen Vertikalschnitt durch die Formpressvorrrichtung zur Herstellung von Kernformteilen mit unterhalb des Oberstempels angeordneten und von diesem nach unten bewegten Zwischenstempel,
- Fig. 63 einen prinzipiellen horizontalen Querschnitt durch die Presse, bei einer Ausführungsform mit jeweils 2 Zeischenstempeln und ihren Halterungen.

Die Presse zur Herstellung von Kernformteilen besteht aus einem Rotor 50, der an einer rotierenden Antriebswelle 51 festgelegt ist und aus einem Oberteil 52, einem Unterteil 53 und einem dazwischen befestigten Matrizentisch 54 gebildet ist, und aus einer feststehenden Kurvenglocke 55 mit Kurvenscheibe 56 und Oberstempelkurve 57. Innerhalb der gestellfesten Kurvenglokke 55 ist die Antriebswelle 51 mittels nicht näher dargestellter Lagerungen gelagert.

Der Rotor 50 umfaßt eine auf die Antriebswelle 51 aufgesetzte, kreisrunde Trageplatte 58, auf welche das kreisringförmige Unterteil 53 zur Führung der Unterstempel 1 fest aufgesetzt ist. Die Köpfe 60 der Unterstempel 1 werden in nicht näher dargestellten Kurven geführt, um eine vertikale Auf- und Abbewegung der Stempel 1 zu bewirken. Auf das Unterteil 53 ist der kreisscheibenförmige Matrizentisch 54 aufgesetzt. Dieser umfaßt einerseits einen Teilkreis 61 für die Matrizen 3 und einen im Durchmesser kleiner als der Teilkreis 61 für die Matrizen 3 ausgebildeten radialen Freiraum 63.

Oberhalb des Unterteiles 53 und des Matrizentisches 54 ist das mit dem Rotor 50 befestigte Oberteil 52 angeordnet, das der Führung der Oberstempel 2 dient, die in Führungsbuchsen 65 innerhalb des Oberteiles 52 gelagert sind. Die Köpfe 66 der Oberstempel 2 sind in der Oberstempelkurve 57 geführt, die an der gestellfesten Kurvenglocke 55 befestigt ist. Die an der Antriebswelle 51 angeschweißte Trageplatte 58 ist mit einem konzentrisch zur Achse der Antriebswelle 51 angeordneten, angeschweißten Tragezylinder 67 fest verbunden, der eine innere Abstützung für das Oberteil 52 und das Unterteil 53 des Rotors 50 bildet.

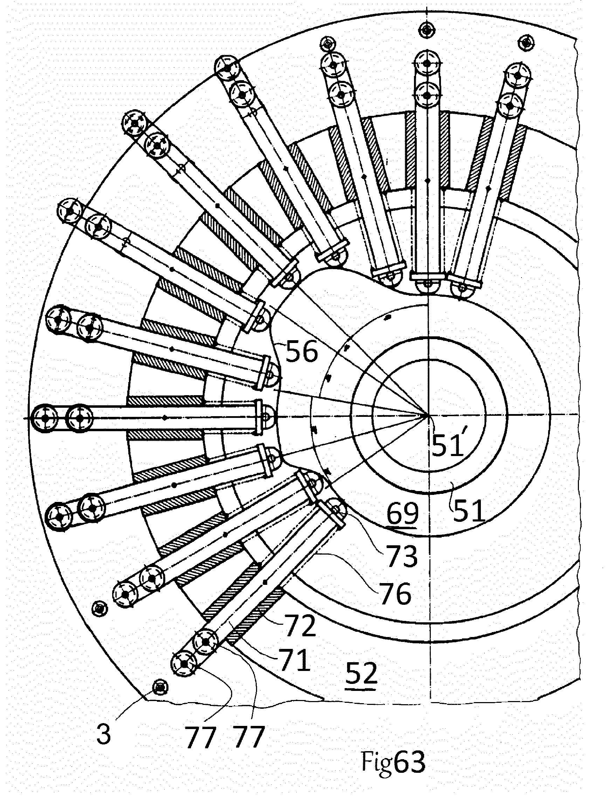

An der Kurvenglocke 55 ist die Kurvenscheibe 56 mittels Gewindeschrauben festgelegt. Der Grundkörper, der an seinem unteren Ende die Kurvenscheibe 56 bildet, ist konzentrisch zur Antriebswelle 51 angeordnet. Die Form der Kurvenscheibe 56 ergibt sich aus dem Querschnitt gemäß der Figur 63. Von der Kurvenscheibe 56 werden Radialarme 70 betätigt, die als mit einem Polygonprofil 71 versehene Kolben ausgebildet sind, die innerhalb von in das Oberteil 52 radial eingesetzten, ein Polygonprofil aufweisenden Buchsen 72 radial zur Achse der Antriebswelle 51 gelagert sind. Zur Durchführung der Kolben weist der Tragezylinder 67 des Rotors 50 radiale Durchgangsöffnungen auf. Am radial innen gelegenen Ende der Kolben sind in Aufnahmeschlitzen Stützrollen 73 über Wälzlager, insbesondere Nagellager, gelagert, die auf einem quer zur Längsachse der Kolben angeordneten Lagerstift 74 drehbar sind. Auf der radial auswärts gelegenen Seite eines jeden Lagerstiftes 74 ist eine Begrenzungsscheibe 75 für eine Druckfeder 76 gelagert, die sich auf der radial einwärts gerichteten Stirnseite der Polygon-Buchsen 72 abstützt und eine ständige Anpreßkraft für die Stützrolle 73 an der Außenkurve der Kurvenscheibe 56 bewirkt.

Die in den Figuren dargestellte Presse trägt auf dem Tisch 54 vierundzwanzig Matrizen 3 mit zugehörigen Unter- und Oberstempeln 1, 2 und mit zugehörigen Radialarmen 70 in Form der Kolben. Jeder Kolben trägt an seinem radial äußeren, freien Ende eine Zwischenstempelhalterung 77 (Figur 63 zeigt eine Ausführungsform mit jeweils zwei Zwischenstempelhalterungen). Jede Zwischenstempelhalterung 77 umfaßt ein Kopfstück, das mit einer Bohrung zur Aufnahme des Kolbens versehen ist. Das Kopfstück ist am Kolben drehfest und unverschiebbar festgelegt. Quer zur Längsachse des Kolbens und parallel zur Achse 51' der Antriebswelle 51 erstreckt sich ein Zwischenstempel 38, der der Verpressung von erstem Hüllmaterial und Kernmaterial dient. Der Zwischenstempel 38 durchdringt eine untere Bohrung des Kopfstückes mit einer Gleitführung und ist ferner durch einen im oberen Bereich des Kopfstückes in einer Innenbohrung gleitenden Bund geführt. In der Innenbohrung des Kopfstückes ist am oberen Ende eine Kreisringscheibe mittels eines Sprengringes befestigt. Zwischen dem Bund des Kernstempels 38 und dem Boden der Innenbohrung des Kopfstückes erstreckt sich eine Druckfeder, welche einen ständigen Anschlag des Bundes des Zwischenstempels 38 an der Kreisringscheibe bewirkt.

Die Wirkungsweise der voranstehend beschriebenen Presse zur Herstellung von Kernformteilen wird nachstehend näher erläutert.

Nach Befüllung der Matrize mit dem ersten Hüllmaterial wird über die Kurvenscheibe 56 und die dieser zugeordnete radial wirkende Druckfeder 76 eine radiale Auswärtsbewegung der Zwischenstempelhalterung 77 in den Teilkreis 61 der Matrizen 3 durchgeführt. Der Zwischenstempel befindet sich nunmehr zur Verpressung unterhalb des Oberstempels und oberhalb einer Matrize 3. Das innerhalb der Matrize 3 auf dem Unterstempel 1 befindliche erste Hüllmaterial wird nun mittels des Zwischenstempels verpresst. Dazu wird der Zwischenstempel 38 unter Wirkung des Oberstempels 2 gegen die Wirkung der Druckfeder eingedrückt. Anschließend wird der Oberstempel 2 unter Wirkung der Oberstempelkurve 57 angehoben und gleichzeitig der Zwischenstempel 38 unter Wirkung der Druckfeder aus der Matrize 3 herausgeführt. Anschließend erfolgt unter Wirkung der Kurvenscheibe 56 und der den Kolben radial einwärts drückenden Druckfeder 76 ein radiales Einfahren der Zwischenstempelhalterung 77 in den Freiraum 63, der sich radial einwärts außerhalb des Bereiches der im Teilkreis 61 gelegenen Matrizen 3 befindet. In dieser Stellung der Zwischenstempelhalterung 77 kann nun die Befüllung der Matrize mit dem Kernmaterial erfolgen. Anschließend erfolgt unter Wirkung der Kurvenscheibe 56 und der den Kolben radial einwärts drückenden Druckfeder 76 ein radiales Ausfahren der Zwischenstempelhalterung 77 in den Bereiches der im Teilkreis 61 gelegenen Matrizen 3. Das innerhalb der Matrize 3 auf dem bereits verpressten ersten Hüllmaterial befindliche Kernmaterial wird nun mittels des Zwischenstempels unter Einwirkung des Oberstempels verpresst. Bei Ausführungsformen mit jeweils mehreren Zwischenstempelhalterungen pro Radialarm kommt bei diesem Pressschritt ein anderer Zwischenstempel zum Einsatz als bei der vorhergehenden Verpressung des ersten Hüllmaterials. Dazu wird beispeilsweise der Radialarm entsprechend mehr oder weniger ausgefahren um den gewünschten Zwischenstempel unterhalb des Oberstempels zu positionieren. Anschließend erfolgt unter Wirkung der Kurvenscheibe 56 und der den Kolben radial einwärts drückenden Druckfeder 76 ein erneutes radiales Einfahren der Zwischenstempelhalterung 77 in den Freiraum 63. In dieser Stellung der Zwischenstempelhalterung 77 kann nun die Befüllung der Matrize mit dem zweiten Hüllmaterial und danach unter Wirkung von Unter- und Oberstempel 1, 2 der letzte Pressvorgang der Formpressvorrichtung erfolgen.

Die beschriebenen radialen Arme können in auch so angeordnet sein, dass sie nicht genau radial verlaufen, sondern leicht schräg, also quasi tangential eines konzentrischen Kreises um die Achse des Rotors, der kleiner ist als der Umlaufkreis der Oberstempel. Dadurch kann eine längere Führung der Arme ermöglicht werden, z.B. wenn der Durchmesser des Mittelteils relativ klein oder der Durchmesser der Achse relativ groß.

Vorzugsweise umfassen die waagerechten Profile bzw. die radialen Arme mehrere Halterungen für Zwischenstempel und sind auf mehr als zwei Positionen positionierbar. So können mehrere verschiedene Zwischenstempel zum Einsatz kommen.

Das vorab beschriebene L-förmige Profil kann beispielsweise Y-förmig sein und zwei Halterungen mit Zwischenstempeln aufweisen. Abhängig von der Führungsbahn (bzw. von ihrem Durchmesser auf bestimmten Teilkreisen) wird dann der erste, der zweite oder keiner der Zwischenstempel unterhalb des Oberstempels positioniert.

Eine Positionierung des Zwischenstempels (egal ob per Drehung oder sonstiger Bewegung) kann auch durch Magnete, Hydraulik, Pneumatik oder sonstige Mechanik erfolgen.

Bei Ausführungsformen mit Zwischenstempel kann bevorzugt auf die gefederte Lagerung des unteren Außenstempels verzichtet werden, insbesondere wenn der untere Außenstempel zur Verfüllung und Verpressung der zweiten Schicht des Hüllmaterials durch andere Maßnahmen nach unten bewegt werden kann, beispielsweise durch separate Führungsbahnen.

Es kann vorteilhaft sein, den Zwischenstempel nach dem Pressschritt nicht nur über die federnde Lagerung wieder nach oben bewegen zu lassen. Zusätzlich zur oder anstatt der federnden Lagerung kann die Anhebung des Zwischenstempels auch durch andere Maßnahmen erfolgen.

In weiteren Ausführungsformen, vorzugsweise solchen, die ansonsten der vorab beschriebenen Ausführungsform mit der synchronen Mitführung der Zwischenstempel durch eine Kette entsprechen (angelehnt an die in EP2165826A2 verwendete Mitführung der "core retention elements"), umfasst die Lagerung des Zwischenstempels zwei Ringe, die flach aufeinander liegen. Beide Ringe weisen an ihrer dem jeweils anderen Ring zugewandten axialen Seitenfläche Sägezähne ähnlich einer Hirth-Verzahnung auf, die komplementär zueinander sind und somit ineinander greifen können. Einer der Ringe, vorzugsweise der untere Ring, ist um seine vertikale Achse drehbar gelagert. Vorzugsweise liegt dieser Ring mit seiner flachen axialen Seite auf dem Boden des "transfer cog" auf. Der zweite Ring liegt mit seiner gezahnten axialen Seite auf dem ersten Ring auf und ist gegenüber dem transfer cog nicht drehbar. Beispielsweise weist er an seinem Umfang radiale Führungsstifte auf, die in senkrechte Nuten auf der Innenseite des transfer cog greifen. Somit ist er nach oben hin verschiebbar.

Wird der untere, drehbar gelagerte Ring gedreht, so stehen die Zähne beider Ringe nicht mehr komplementär zueinander, so dass der obere Ring nach oben hin verschoben wird. Diese Verschiebung basiert auf der gleichen Mechanik wie bei Hubelement (4) aus EP1158207B1 auf die hiermit bezüglich der Hubmechanik Bezug genommen wird.

Durch diese Verschiebung des oberen Rings nach oben wird der Zwischenstempel zwangsweise angehoben, bzw. die Hubwirkung der federnden Lagerung unterstützt.

Vorzugsweise werden federnde Lagerung und Dreh-Hub-Mechanik kombiniert. Beispielsweise weisen die Ringe einen wesentlich größeren Innendurchmesser auf als der Zwischenstempeldurchmesser am unteren Ende. Dadurch ergibt sich zwischen Innenstempel und den Ringen ein zylinderförmiger Hohlraum in dem die Feder angeordnet werden kann.

Vorzugsweise weist der untere Ring an seinem Umfang ebenfalls Zähne auf, die durch ein Loch im transfer cog mittels einem von außen eindringenden Stift dazu benutzt werden können, den Ring in Drehung zu versetzen. Entsprechende Stifte befinden sich beispielsweise in den Aussparungen des hinteren (von der Kette zuletzt umlaufenen) Zahnrades, welches die Kette an den Teilkreis des Matrizentisches andrückt und zur Umlenkung der Kette dient.

Erreicht ein transfer cog das Umlenkzahnrad, sticht dessen Stift durch das Loch, dreht den unteren Ring und hebt damit den Zwischenstempel an.

In einer weiteren Ausführungsform ist vor dem letzten Umlenkzahnrad ein weiteres Zahnrad angeordnet, das entsprechende Stifte aufweist und somit den Zwischenstempel bereits vor dem Umlenkzahnrad anhebt.

Eine weitere Möglichkeit, den Zwischenstempel anzuheben, besteht darin, dass er über eine Führungsbahn, die sich nicht mit dem Rotor dreht, angehoben wird. Vorzugsweise werden dafür an den Stellen des Umlaufkreises, an dene eine Anhebung erforderlich ist, entsprechende schräge Rampen angeordnet. Der Zwischenstempel weist dann eine Führungsnase auf, die, wenn der Zwischenstempel an der Rampe vorbei geführt wird, auf dieser aufliegt und von ihr nach oben bewegt wird.

Eine weitere Möglichkeit, den Zwischenstempel anzuheben, besteht darin, ihn während des Teilkreises, den er synchron mit dem entsprechenden Oberstempel durchläuft, mit diesem so mechanisch zu koppeln, dass er bei dessen Aufwärtsbewegung ebenfalls angehoben wird.

In einer weiteren Ausführungsform wird zur Möglichkeit des Anhebens des Zwischenstempels durch den oberen Stempel eine umlaufende Nut (Zeichnung 43, 36) in den Oberstempel eingebracht. Eine Feder am Zwischenstempel (Zeichnung 43, 37) greift in diese Nut ein und ermöglicht es, dass der Oberstempel den Zwischenstempel sowohl bei Abwärts-, als auch bei Aufwärtsbewegungen mitnimmt.

Vorzugsweise erfolgt die Mitnahme des Zwischenstempels nach unten nicht über Nut und Feder, sondern über die vorab beschriebene Ringförmige Fläche an der Stelle, an der sich der obere Stempel verjüngt. Hierdurch kann eine größere Kontaktfläche und somit eine bessere Kraftübertragung beim Pressen erreicht werden.

Alternativ kann auch eine Feder um den Oberstempel umlaufen, die in eine Nut am Zwischenstempel passt.

Alternative Passungen sind möglich und können vom Fachmann ausgeführt werden, ohne erfinderisch tätig werden zu müssen.

Ein Ausführungsbeispiel ist in Zeichnung 43 gezeigt.

Der obere Stempel weist oberhalb des unteren Endes seines Führungsschaftes eine umlaufende Nut (36) auf.

Der Zwischenstempel (38) weist an seinem oberen Ende eine rohrförmige Struktur auf, deren Innendurchmesser geringfügig größer ist als der Durchmesser des Führungsschaftes des oberen Stempels.

Eine ringförmige, bzw. aufgrund der seitlichen Öffnung eher halbringförmige Feder (37) ermöglicht die vertikale Anhebung des Zwischenstempels durch den oberen Stempel, in dessen Nut (36) die Feder eingreift. Zeichnung 43 zeigt den zentralen Schnitt von der Seite mit der seitlichen Öffnung aus gesehen. Der obere Stempel ist abgesenkt, zentriert, und kann nun den Zwischenstempel nach unten bewegen.

Zeichnung 44 zeigt den Zwischenstempel von der Seite mit der seitlichen Öffnung aus gesehen.

Zeichnung 45 zeigt den oberen Stempel und den Zwischenstempel von der Seite mit der seitlichen Öffnung aus gesehen, mit dem oberen Stempel in der noch nicht abgesenkten Position, in welcher der Zwischenstempel von der Seite her (in dieser Ansicht von hinten) unter dem oberen Stempel positioniert und auch wieder entfernt werden kann..

In weiteren Ausführungsformen, insbesondere bei solchen Ausführungsformen, bei denen die Pressfläche des Zwischenstempels größer ist als die Pressfläche des oberen Stempels, kann die Pressfläche des Zwischenstempels eine Aussparung aufweisen, in die von oben der untere Teil des oberen Stempels ragt. Somit bilden dann Pressfläche des oberen Stempels und Pressfläche des Zwischenstempels eine gemeinsame Pressfläche. Bei solchen Ausführungsformen ragt der untere Teil des oberen Stempels vorzugsweise nicht so weit in die Aussparung des Zwischenstempels hinein, dass seine Pressfläche an ihrem äußeren Umfang tiefer liegt, als die innere Kante der Pressfläche des Zwischenstempels.

Vorzugsweise tritt die Pressfläche des oberen Stempels jedoch mit dem zu verpressenden Material in Kontakt, wenn ein Zwischenstempel unterhalb des oberen Stempels positioniert ist. Besonders bevorzugt wird dies dadurch sichergestellt, dass der obere Stempel nicht durch eine Aussparung in einem Zwischenstempel hindurch ragen kann.

Ausführungsformen mit einem oder mehreren Zwischenstempeln können sowohl zusammen mit federnd gelagerten unteren Stempeln ausgeführt werden, mit oder ohne eigene Stempelführungsbahnen, als auch mit ungefederten Unterstempeln, die dann vorzugsweise über eigene Stempelführungsbahnen geführt werden.

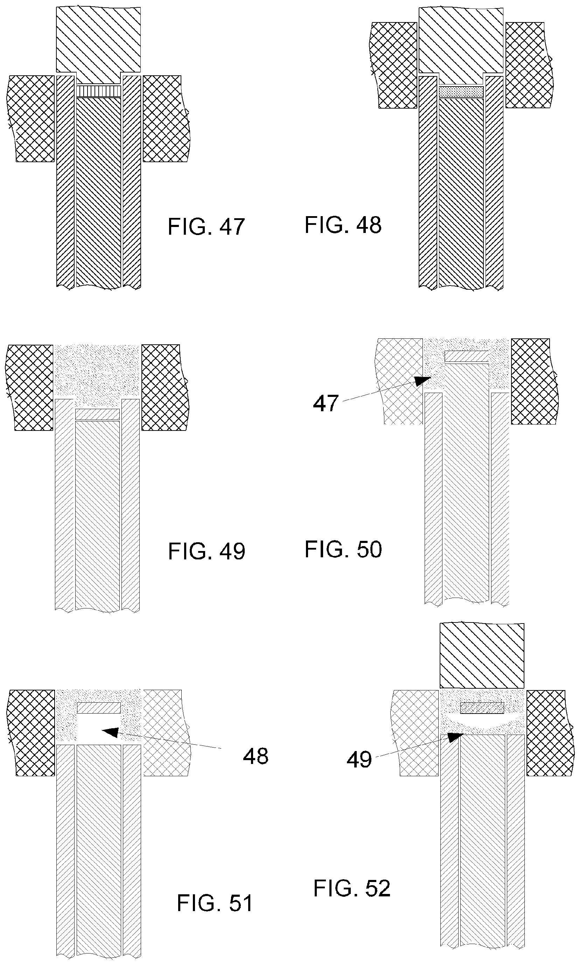

In weiteren Ausführungsformen wird auf das Einfüllen und verpressen einer ersten Schicht aus Hüllmaterial verzichtet. Es wird also zuerst bei angehobenem äußerem unteren Stempel und abgesenktem inneren unteren Stempel Kernmaterial eingefüllt und verpresst (Fig. 47 und 48).

Dann wird der untere Außenstempel bei, vor oder nach dem Einfüllen des Hüllmaterials so weit abgesenkt, dass seine Oberkante bis unter die Oberkante des unteren Innenstempels abgesenkt wird, oder/und der Innenstempel vor, während oder nach diesem Einfüllschritt entsprechend weit angehoben. Bevorzugt wird der untere Innenstempel nach dem Einfüllschritt (Fig. 49) angehoben (Fig. 50). Vorzugsweise findet das Anheben statt, so lange sich die Fülleinrichtung noch über der Matrizenöffnung befindet, so dass aus der Matrize austretendes Material in die Fülleinrichtung zurückgedrückt wird und nicht verloren geht. Dadurch befindet sich Hüllmaterial seitlich des unteren Innenstempels auch unterhalb des verpressten Kernmaterials, wodurch eine Art Schürze 47 aus Hüllmaterial entsteht. Wird sodann der innere untere Stempel abgesenkt, entsteht unterhalb des bereits verpressten Kernmaterials ein Hohlraum (48, Fig. 51), da das verpresste Kernmaterial durch das um es herum eingefüllte Hüllmaterial daran gehindert ist, dem Stempel nach unten zu folgen. Damit sich das bereits verpresste Kernmaterial nicht zusammen mit dem unteren Innenstempel absenkt, kann es erforderlich sein, dass das sich seitlich davon befindende Hüllmaterial vorher leicht verdichtet und dadurch an das Kernmaterial herangedrückt wird. Dazu wird in einer bevorzugten Ausführungsform vor dem Absenken des unteren Innenstempels der untere Außenstempel angehoben, vorzugsweise um ein Drittel, mehr bevorzugt um die Hälfte der Höhe der Schürze. Um ein Ausweichen des Hüllmaterials nach oben zu verhindern wird in einer weiter bevorzugten Ausführungsform der Oberstempel währdenddessen auf das Hüllmaterial aufgesetzt. Vorzugsweise wird der innere untere Stempel so weit abgesenkt, dass seine Pressfläche mit der des äußeren unteren Stempels auf einer Ebene liegt.

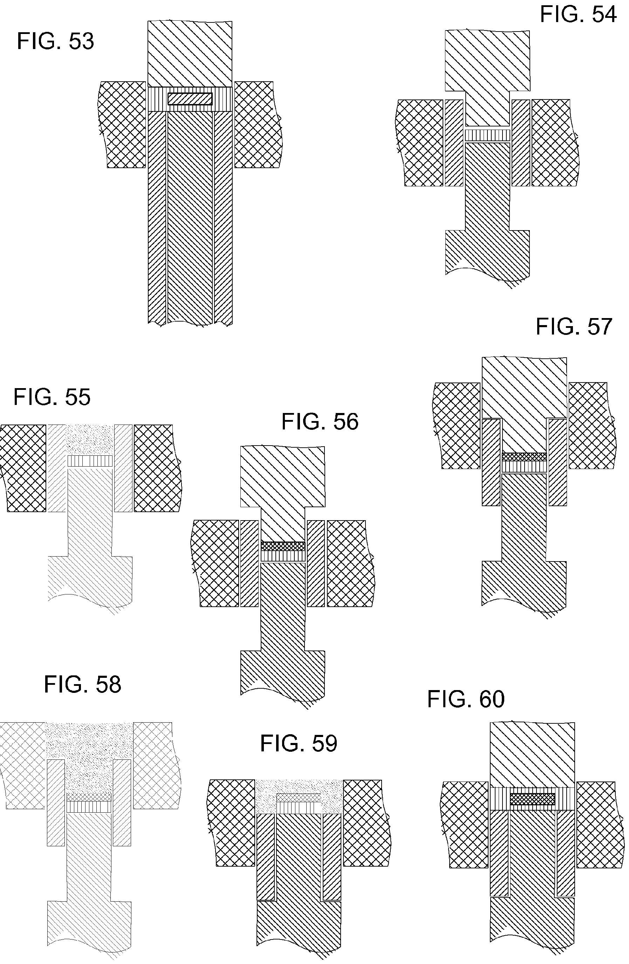

Dann wird der untere Stempel angehoben (Fig. 52). Dabei bricht die aus unverpresstem Hüllmaterial bestehende Schürze während dieser Aufwärtsbewegung des unteren Stempels auseinander und das auseinandergefallene Hüllmaterial 49 verteilt sich auf der Pressfläche des unteren Stempels, wodurch sich auch unterhalb des verpressten Kernmaterials eine Schicht verpressbaren Hüllmaterials befindet und beim weiteren Anheben des unteren Stempels ein Kernformteil erzeugt wird (Fig. 53). Alternativ zum oder gemeinsam mit dem Anheben des unteren Stempels kann auch der obere Stempel abgesenkt werden um das Material der Schürze brechen zu lassen und in den Hohlraum zu drücken.

Anschließend wird die gesamte Tablette verpresst und ausgestoßen.

In weiteren Ausführungsformen wird ein mindestens dreiteiliger koaxialer unterer Stempel verwendet, der zusätzlich zum zweiteiligen unteren Stempel noch einen äußersten unteren Stempel aufweist. Mit solchen Ausführungsformen können Kernformteile hergestellt werden, bei denen der Kern doppelt umhüllt ist.

Der äußerste Stempel bleibt so lange angehoben und seine Pressfläche in einer Ebene mit der Oberkante der Matrize, bis mittels einer der vorstehend beschriebenen Verfahren ein Kernformteil hergestellt ist, welches als inneres Kernformteil bezeichnet wird.

Danach fungieren der innere untere Stempel und der äußere untere Stempel wie der innere untere Stempel in der unmittelbar vorstehenden Ausführungsform, bei der auf das Einfüllen und verpressen einer ersten Schicht aus Hüllmaterial verzichtet wird. Der äußerste untere Stempel fungiert entsprechend wie der äußere untere Stempel in der unmittelbar vorstehenden Ausführungsform. Das bereits hergestellte innere Kernformteil fungiert entsprechend dem bereits verpressten Kernmaterial. Nach dem Verpressen des inneren Kernformteils wird der äußerste untere Stempel bei, vor oder nach dem Einfüllen des Hüllmaterials für die äußerste Umhüllung so weit abgesenkt, dass seine Oberkante bis unter die Oberkante des inneren und des äußeren unteren Stempels abgesenkt wird, oder/und der innere und der äußere unteren Stempel vor, während oder nach diesem Einfüllschritt entsprechend weit angehoben. Dadurch befindet sich Hüllmaterial seitlich des äußeren unteren Stempels auch unterhalb des inneren Kernformteils, wodurch eine Art Schürze aus Hüllmaterial entsteht. Werden sodann innerer und äußerer unterer Stempel abgesenkt, entsteht unterhalb des inneren Kernformteils ein Hohlraum, da das innere Kernformteil durch das um es herum eingefüllte Hüllmaterial daran gehindert ist, dem Stempel nach unten zu folgen. Vorzugsweise werden innerer und äußerer unterer Stempel so weit abgesenkt, dass ihre Pressflächen mit der des äußersten unteren Stempels auf einer Ebene liegen.

Dann wird der gesamte untere Stempel angehoben. Dabei fällt bzw. bricht die aus unverpresstem Hüllmaterial bestehende Schürze während dieser Aufwärtsbewegung des unteren Stempels auseinander und das Hüllmaterial verteilt sich auf der Pressfläche des unteren Stempels, wodurch sich auch unterhalb des inneren Kernformteils eine Schicht verpressbaren Hüllmaterials befindet. Alternativ zum oder gemeinsam mit dem Anheben des unteren Stempels kann auch der obere Stempel abgesenkt werden um das Material der Schürze brechen zu lassen und in den Hohlraum zu drücken. In einer bevorzugten Ausführungsform ist die Pressfläche des unteren Stempels konkav ausgeführt oder zumindest die Pressfläche des äußersten unteren Stempels nach innen hin (zum inneren Stempel hin) abfallend. Dadurch kann die Verteilung eines Teils des Hüllmaterials, das die Schürze gebildet hatte, unter das innere Kernformteil unterstützt werden.

Anschließend wird die gesamte Tablette verpresst und ausgestoßen.

Hierdurch lassen sich auch Formteile mit doppelt umhüllten Kernen herstellen.

Beispielsweise werden als Kernmaterial ein Wirkstoffgranulat oder wirkstoffhaltige Mikropellets verwendet, als erstes Hüllmaterial ein Granulat aus Polyvinylacetal-Diethylaminoacetat (AEA Sankyo) ggf. mit Hilfsstoffen, als zweites Hüllmaterial ein Granulat aus Schellack, ggf. mit Hilfsstoffen und als letztes Hüllmaterial ein Granulat aus Eudragit L ggf. mit Hilfsstoffen. Die so hergestellten Tabletten setzen den oder die Wirkstoffe im Dickdarm eines Menschen frei.

Werden das Wirkstoffgranulat bzw. die wirkstoffhaltigen Mikropellets zuvor mit einem Polymer beschichtet, das oberhalb von pH 7 unlöslich ist, jedoch unterhalb von pH 6,5 löslich, wie beispielsweise Chitosan, kann eine Freisetzung vor Erreichen des Dickdarms noch sicherer verhindert werden, weshalb dies eine bevorzugte Ausführungsform darstellt.

Bei Ausführungsformen mit dreiteiligem koaxialem unterem Stempel wird die vertikale Positionierung der drei Teilstempel beispielsweise mithilfe von drei separaten Führungsbahnen (Führungskurven) realisiert.

Bei bevorzugten Ausführungsformen mit dreiteiligem koaxialem unterem Stempel wird die vertikale Positionierung mindestens eines der drei Teilstempel mithilfe einer gefederten Lagerung mit Rastvorrichtung ausgeführt, so dass vorzugsweise nicht mehr als zwei Führungsbahnen für die unteren Stempel benötigt werden.

In einem Ausführungsbeispiel weist der obere Stempel, bzw. der entsprechende Zwischenstempel, der beim letzten Press-Schritt für das innere Kernformteil zur Anwendung kommt, eine Pressfläche auf, deren laterale Abmessungen den lateralen Abmessungen denen des äußersten unteren Stempels entspricht. Zusätzlich zum Verpressen des inneren Kernformteils bewegt er somit auch den äußersten unteren Stempel, der federnd gelagert ist, nach unten. In dieser nach unten Bewegten Position rastet der äußerste untere Stempel ein, beispielsweise relativ zur Matrize.

Nach dem Einfüllen des Materials für die äußerste Umhüllung werden der innere und der äußere untere Stempel angehoben, um das innere Kernformteil nach oben in das eingefüllte Material zu positionieren. Der äußerste untere Stempel bleibt abgesenkt, so dass das oberhalb von ihm eingefüllte Material nicht mit dem inneren Kernformteil nach oben bewegt wird, sondern seitlich desselben und seitlich des äußeren unteren Stempels verbleibt. Ein Teil des oberhalb des inneren Kernformteils befindlichen eingefüllten Materials fließt zur Seite hin und sorgt dafür, dass auch seitlich des angehobenen inneren Kernformteils eingefülltes Material ist. Danach werden der innere und der äußere untere Stempel wieder abgesenkt, um unter dem inneren Kernformteil einen Hohlraum zu schaffen. Bei dieser Abwärtsbewegung wechselt die Rastung des äußersten unteren Stempels von einer Rastung relativ zur Matrize zu einer Rastung relativ zu innerem und äußerem unteren Stempel. Vorzugsweise bilden die Pressflächen der drei Teilstempel in dieser Rastposition eine gemeinsame Pressfläche, die der gewünschten Geometrie der unteren Seite des Kernformteils entspricht.

Falls dies nicht bereits beim Absenken des inneren und des äußeren unteren Stempels geschehen ist, fällt beim anschließenden Anheben des unteren Stempels das seitlich des Hohlraums befindliche Material nach innen auf die Pressfläche des unteren Stempels und wird beim weiteren Anheben zum unteren Teil der äußersten Umhüllung verpresst. Das seitlich vom inneren Kernformteil befindliche Material wird zum seitlichen Teil der äußersten Umhüllung verpresst, das oberhalb des inneren Kernformteils befindliche Material wird zum oberen Teil der äußersten Umhüllung verpresst

Beispielbeschreibung einer Ausführungsform der Rastmechanismen:
Der äußerste Stempel (St1) rastet gegen ein Rohr (Ro2), das über eine Feder (Fe1) in Richtung Matrize (Ma) gedrückt wird. Eine stärkere, vorgespannte Feder (Fe2) hält einen bestimmten Abstand zwischen Rohr Ro2 und der Matrize, so lange die Kraft, die das Rohr Ro2 nach oben drückt, die Vorspannkraft der Feder Fe1 nicht überschreitet. Zwischen der vorgespannten Feder Fe1 und der Matrize ist noch eine Scheibe Sc1 angeordnet, die an einem weiteren Rohr Ro2 befestigt ist, welches über den Stempelschaft (Sh1) ragt und dort so eingehakt ist, dass der Weg des Rohres Ro2, der vorgespannten Feder Fe1 und des äußersten Stempels St1 nach oben begrenzt ist, wenn der untere Stempel nach unten bewegt wird.

Durch Druck von oben wird der äußerste Stempel nach unten gedrückt und rastet dort ein, so dass er unten bleibt, wenn der Druck von oben weggenommen wird. Auf erneuten Druck von oben rastet der äußerste Stempel wieder aus, bzw. der Rotor in eine witer nach oben verschiebbare Position, und kann sich wieder weiter nach oben bewegen. Dies wird beispielsweise durch einen Rastmechanismus erreicht, wie er auch in Kugelschreibern zum Einsatz kommt, beispielsweise durch einen Rastmechanismus mit einem Rotor. Der Rotor wird über eine Feder nach oben gedrückt und drückt somit auch den äußersten Stempel nach oben, wobei die Höhe eben durch das Einrasten des Rotors in den Führungsnuten auf der Innenseite des Rohres Ro2 bestimmt wird, und diese Höhe zwischen zwei vorgegebenen Höhen alterniert.

Der Weg des Äußersten Stempels nach oben ist mittels eines Anschlages durch die Matrize bzw. die Scheibe Sc1 begrenzt, so dass die Oberkante des äußersten Stempels nicht über die Oberkante der Matrize hinaus ragen kann.

Wird Rohr Ro2 mit einer höheren Kraft als der Vorspannkraft der vorgespannten Feder Fe1 nach oben gedrückt, wird die vorgespannte Feder Fe1 komprimiert und der äußerste Stempel St1 bewegt sich nun relativ zu Rohr Ro2 nach unten, da er wegen der Scheibe S1 nicht weiter mit Ro2 nach oben kann. Dadurch wird der Rastmechanismus zwischen dem äußersten Stempel und Rohr Ro2 wieder gelöst.

Rohr Ro2 ist gegenüber dem äußeren Stempel federnd gelagert (mit Federdruck auf Ro2 nach oben). Ro2 kann durch den äußeren Stempel nach oben bewegt werden. Dies geschieht über eine zweite Rastung. Im Grundzustand ist diese zweite Rastung so eingerastet, dass die Pressfläche des äußeren Stempels über die Pressfläche des äußersten Stempels hinaus angehoben werden kann. Dies ermöglicht es, das innere Kernformteil nach dem Einfüllen des Füllmaterials für die äußerste Hüllschicht in dieses Hüllmaterial hinein anzuheben und eine Art Schürze aus Hüllmaterial um den äußeren Stempel herum zu generieren, da ein Teil des Hüllmaterials dabei seitlich am inneren Kernformteil vorbei rutscht und nur ein Teil davon oberhalb des inneren Kernformteils verbleibt. Bei diesem Anheben wird die zweite Rastung voraktiviert. Wird nun der äußere Stempel wieder abgesenkt um unter dem inneren Kernformteil einen Hohlraum zu erzeugen, so rastet die zweite Rastung so ein, dass das Rohr Ro2 dem äußeren Stempel beim Anheben so folgt, dass der äußerste Stempel im unten gerasteten Zustand der ersten Rastung mit seiner Pressfläche auf der Ebene der Pressflächen des äußeren und inneren Stempels ist und mit diesen eine gemeinsame Pressfläche bildet.

Beim anschließenden Anheben des äußeren Stempels folgt der äußerste Stempel diesem. Die Schürze aus Hüllmaterial fällt zusammen, da sie von unten durch die sich nach oben bewegenden unteren Stempel mechanisch belastet wird und aufgrund des Hohlraums unterhalb des inneren Kernformteils seitlich nicht unterstützt wird, und kann als untere Schicht der äußersten Umhüllung dienen. Ist das Kernformteil fertig verpresst, wird es ausgestoßen, indem der äußere Stempel so weit angehoben wird, dass seine Pressfläche über die Matrizenoberkante hinaus angehoben wird. Da der äußerste Stempel nicht so weit nach oben bewegt werden kann, dass seine Pressfläche über die Matrize hinaus stehen würde, wird er relativ zu dem sich mit dem äußeren Stempel nach oben bewegenden Rohr R2 nach unten bewegt, was seinen Rastmechanismus löst und dafür sorgt, dass er auch nach dem Absenken des äußeren Stempels mit der Oberkante der Matrize bündig bleibt, da er dann wieder durch die Feder Fe3 nach oben gedrückt werden kann.

Bei weiterem Anheben des äußeren Stempels wird der zweite Rastmechanismus für die Ausrastung voraktiviert, so dass er beim Absenken des äußeren Stempels unter den äußersten Stempel wieder ausrastet.

Bei weiteren Ausführungsformen wird ein ungeteilter unterer Stempel verwendet. Ähnlich wie in "A Novel Compression-Coated Tablet Dosage Form", Madhusudan Hariharan und Vishal K. Gupta, Pharmaceutical Technology YEARBOOK 2001, beschrieben, wird zuerst ein Tassenförmiges Formteil aus Hüllmaterial erzeugt. Hierzu wird allerdings kein zweiteiliger koaxialer oberer Stempel verwendet, dessen innerer Stempel gegenüber dem äußeren Stempel nach unten verschoben ist, sondern ein Zwischenstempel , der eine entsprechende Form aufweist (Außendurchmesser der kompletten Pressfläche entsprechend dem Außendurchmesser des unteren Stempels mit einer um die Wandstärke des zu formenden tassenförmigen Formteils verjüngten um die Öffnungstiefe des zu formenden tassenförmigen Formteils nach unten vorspringenden Teil-Pressfläche). Die untere Teil-Pressfläche des Zwischenstempels kann kegelförmig (leicht spitz zulaufend) geformt sein, um einen Teil des eingefüllten Hüllmaterials nach außen zu drücken, damit die Seitenwände des zu formenden tassenförmigen Formteils gebildet werden können. Ebenso kann die obere ringförmige Teil-Pressfläche leicht spitz zulaufend geformt sein, um die Oberkante der genannten Seitenwände etwas schräg nach innen unten zulaufen zu lassen, damit nachfolgend eingefülltes Kernmaterial von diesen abrutscht und in die Mulde des tassenförmigen Formteils fällt.

Dann wird das Kernmaterial eingefüllt und vorzugsweise mit einem weiteren Zwischenstempel in die Mulde gepresst.Danach wird Hüllmaterial für die obere Hüllschicht eingefüllt.

Dieses wird mit einem weiteren Zwischenstempel oder dem oberen Stempel verpresst.

Da beim Verpressen des nach dem Erzeugen des tassenförmigen Formteils eingefüllten Kernmaterials ein anderer Zwischenstempel eingesetzt werden kann, kann dessen Pressfläche anders geformt sein. Ebenso kann auch die Pressfläche des oberen Stempels anders geformt sein als die des oder der Zwischenstempel. Hierdurch ist es möglich, diese Pressflächen flach oder auch leicht konkav oder hohlkegelförmig zu formen. Dies ist Gegenstand einer weiteren Ausführungsform der Erfindung. Im Gegensatz zu dem Verfahren, wie es in "A Novel Compression-Coated Tablet Dosage Form", Madhusudan Hariharan und Vishal K. Gupta, Pharmaceutical Technology YEARBOOK 2001, beschrieben ist, bei dem Tabletten erzeugt werden, die auf der einen Seite konvex bzw. kegelförmig sind, aber auf der anderen Seite konkav oder hohlkegelförmig, können hierdurch plane, plankonvexe oder bikonvexe, bzw. beidseitig kegelförmige Tabletten erzeugt werden. Zusätzlich zur freieren Formgestaltung können die unteren und oberen Lagen von Hüllmaterial (Boden und Deckel des Kernformteils) hierbei auch noch mit besonders einheitlicher Stärke hergestellt werden. Unter anderem wird beispielsweise der Abrieb an spitzwinkligen Tablettenkanten verringert, der z.B. an der Oberkante von Tabletten auftreten kann, die, wie in "A Novel Compression-Coated Tablet Dosage Form", beim letzten Press-Schritt mittels eines kegelförmig geformten Stempels verpresst wurden.

In weiteren Ausführungsformen kommt eine spezielle Matrize zum Einsatz, wodurch auf die Verwendung eines koaxialen Unterstempels verzichtet werden kann, bzw. bei einem ansonsten erforderlichen dreiteilig koaxialem Unterstempel ein zweiteilg koaxialer Unterstempel ausreichend ist. Diese Matrize weist innerhalb ihrer Öffnung einen Rohrförmigen Einsatz auf, der vertikal verschiebbar ist. Vorzugsweise ist die Verschiebbarkeit des rohrförmigen Einsatzes zumindest in eine Richtung begrenzt. Besonders bevorzugt ist die Verschiebbarkeit des rohrförmigen Einsatzes in beide Richtungen begrenzt. Die Begrenzung der Verschiebbarkeit ist vorzugsweise durch einen oder mehrere Anschläge realisiert. Vorzugsweise ist die spezielle Matrize als Matrizeneinsatz ausgeführt, der in eine entsprechende Bohrung in der Matrizenscheibe eingesetzt werden kann. Ähnliche Matrizeneinsätze sind im Stand der Technik bekannt. Sie bestehen üblicherweise aus einer zylinderförmigen Scheibe, welche in ihrer Mitte eine vertikale Bohrung aufweist, die als Matrize für das zu pressende Formteil dient.

Um die Scheibe umlaufend ist eine Nut vorhanden, welche mit einer Befestigungsbohrung in der Matrizenscheibe korrespondiert, durch die ein Haltestift eingeschraubt wird, der mit seiner Spitze in die Nut des Matrizeneinsatzes eingreift, den Matrizeneinsatz gegen die gegenüberliegende Wandung der Bohrung in der Matrizenscheibe presst, und den Matrizeneinsatz somit in der Matrizenscheibe fixiert.

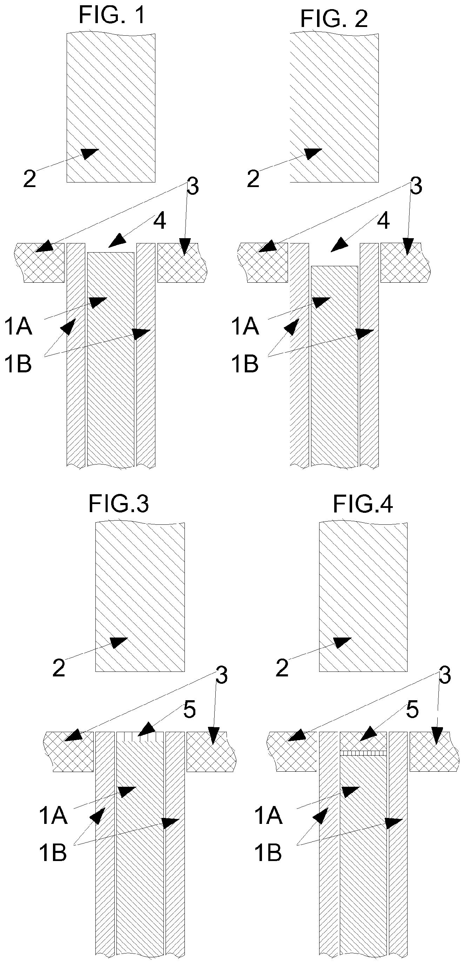

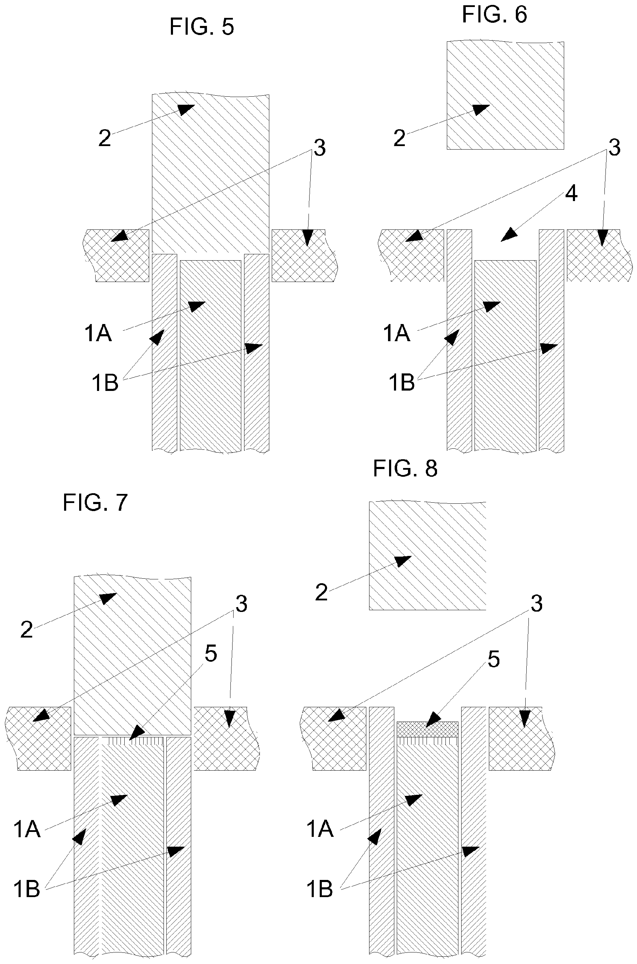

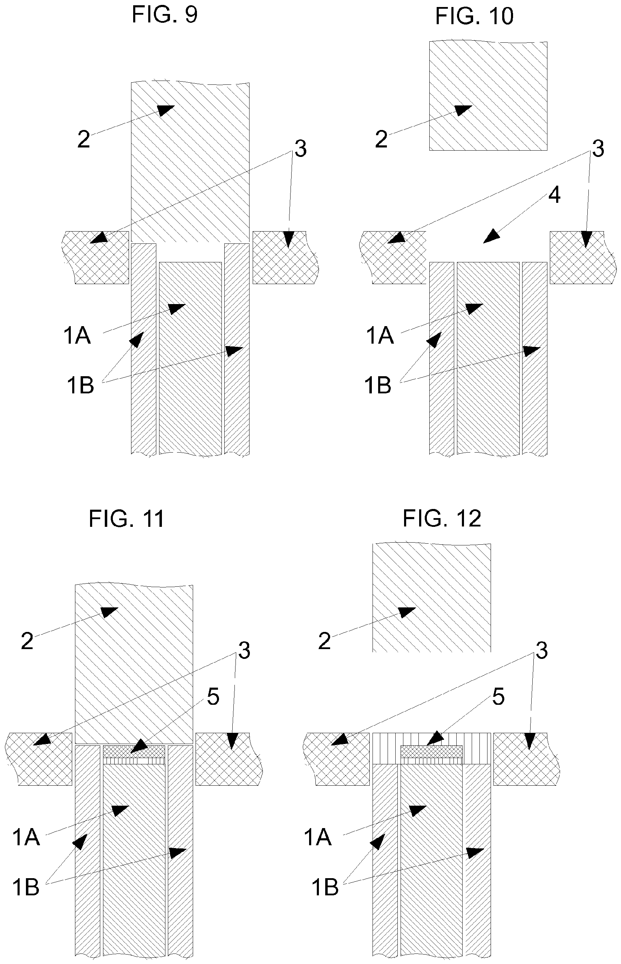

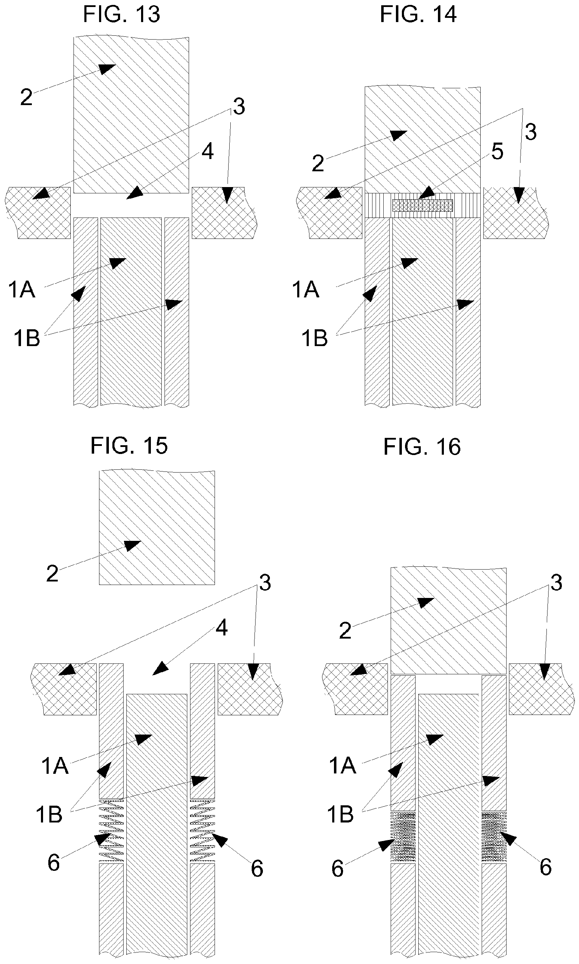

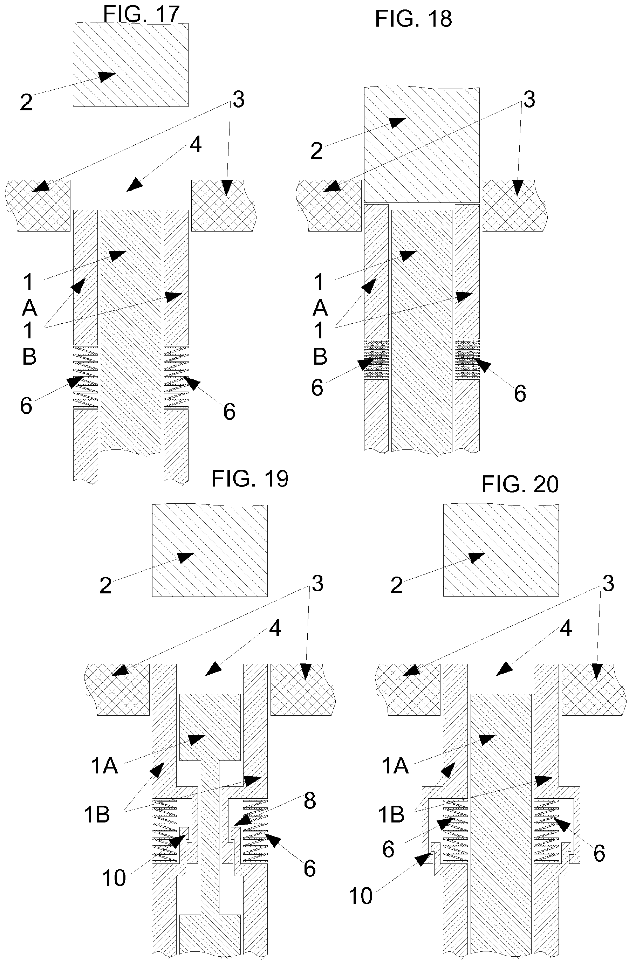

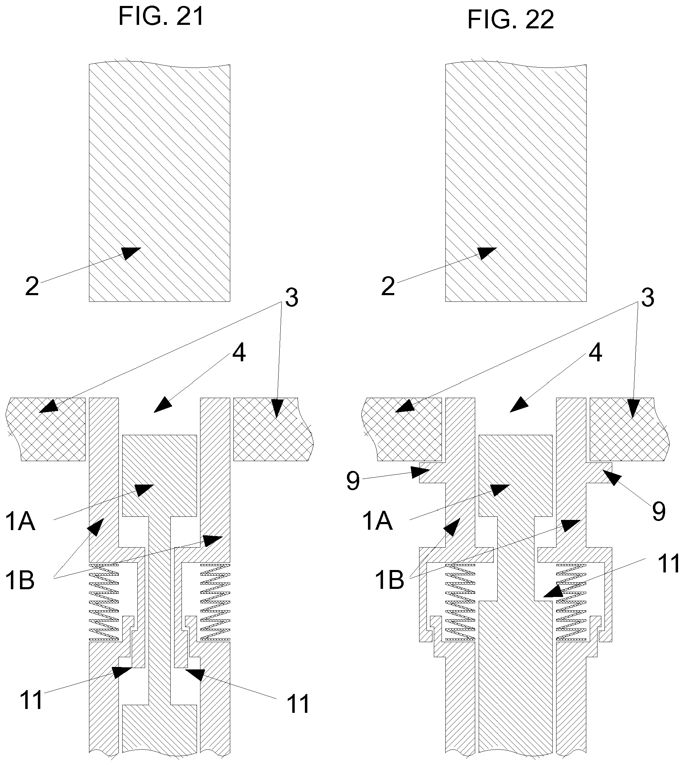

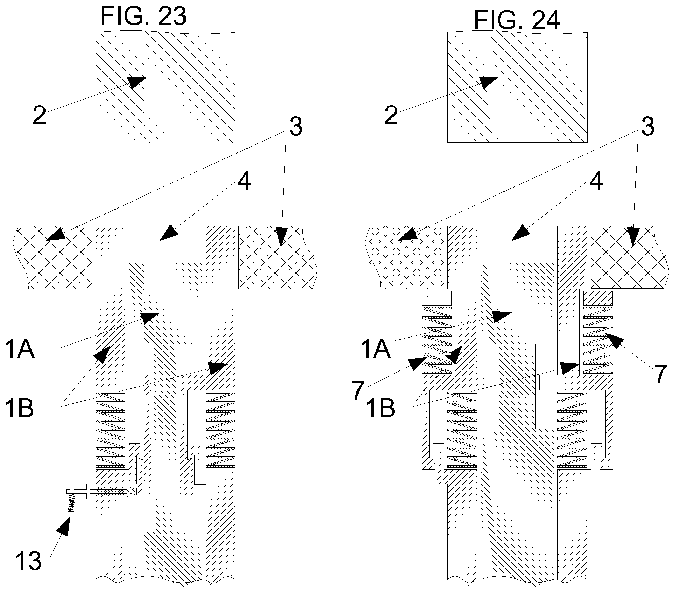

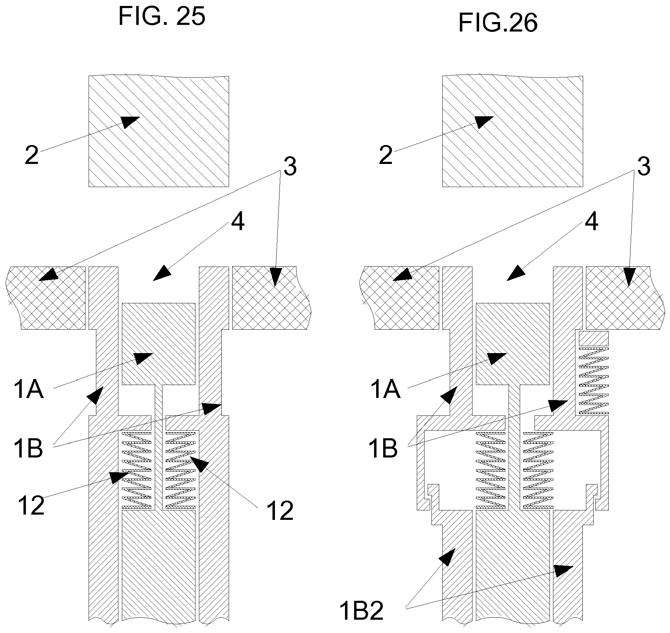

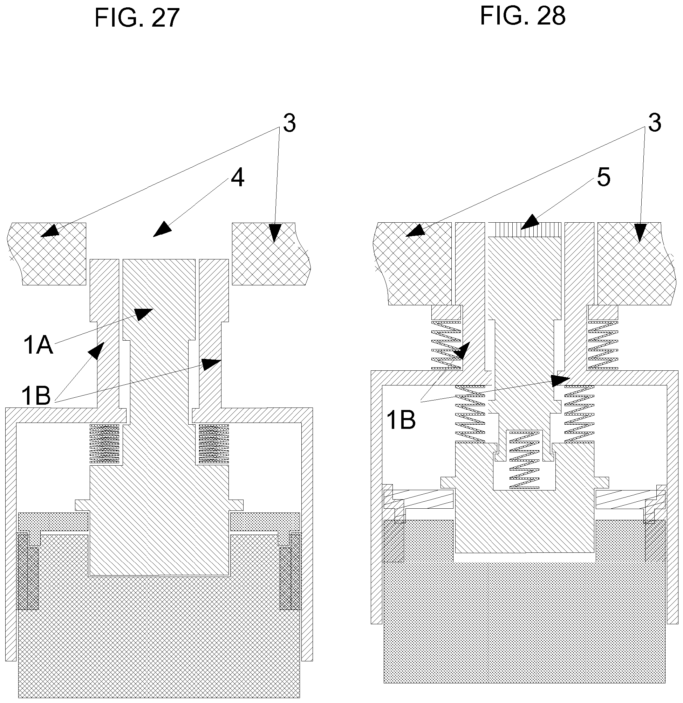

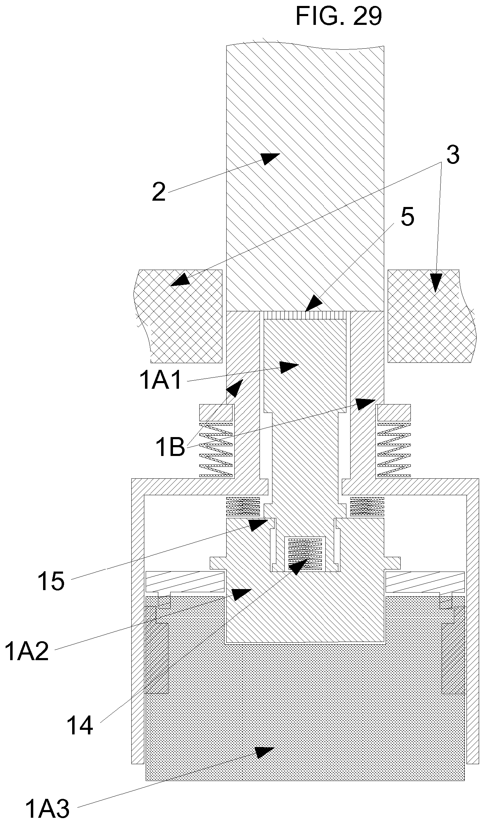

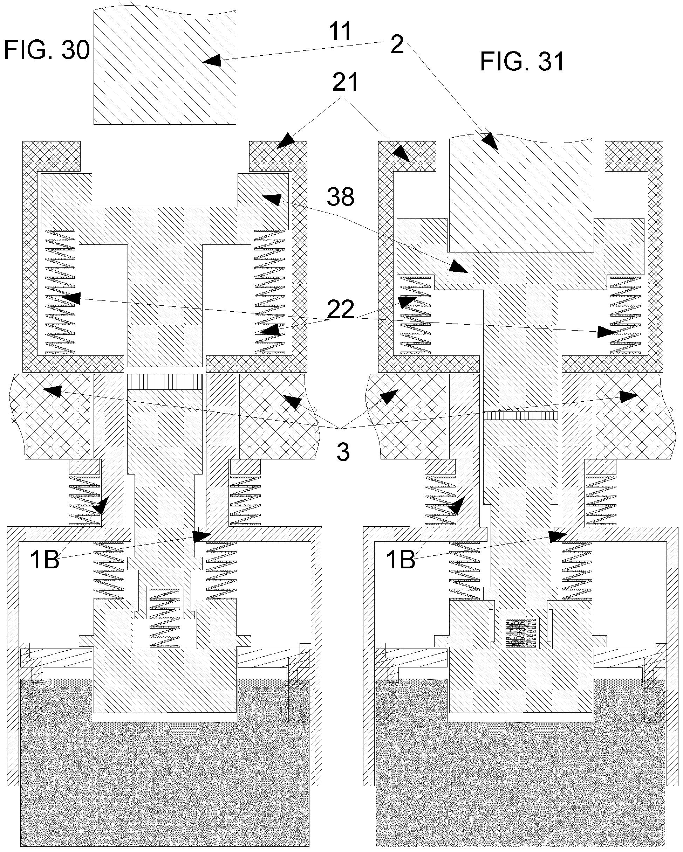

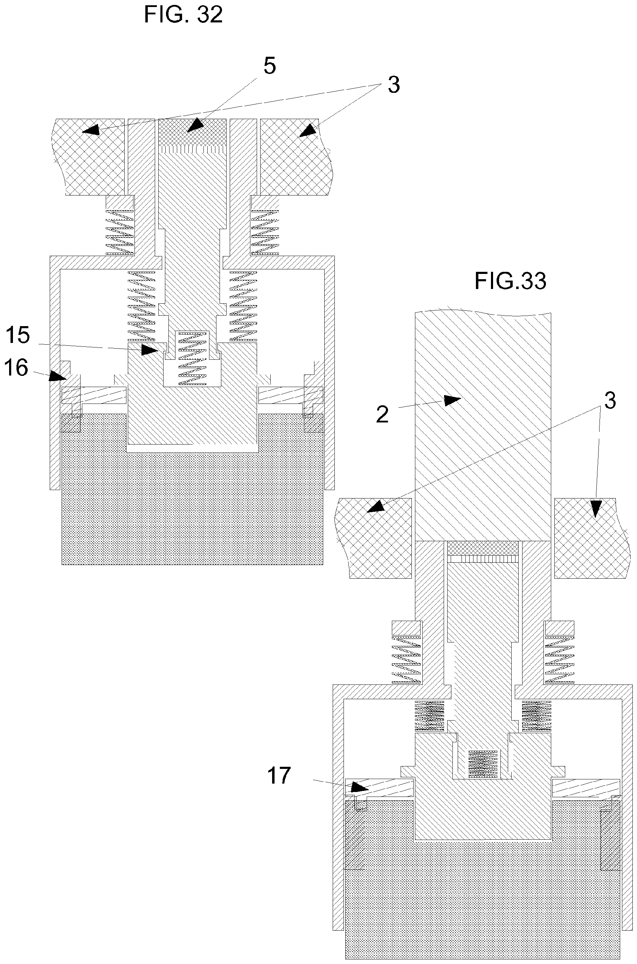

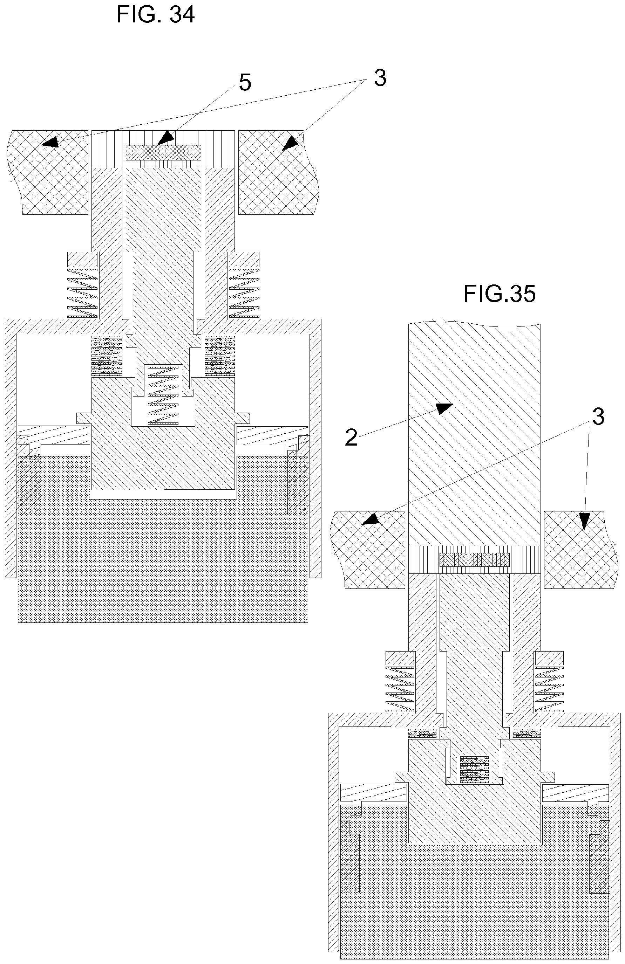

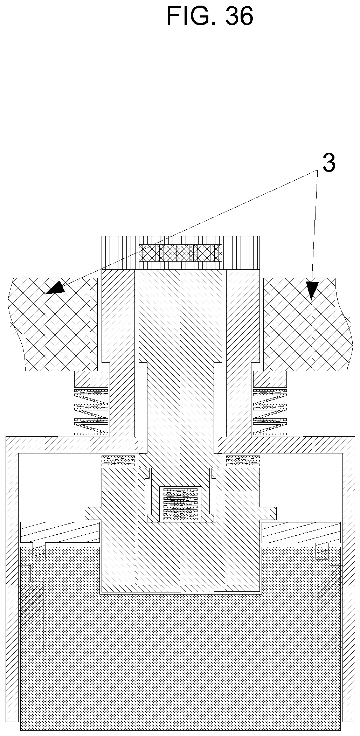

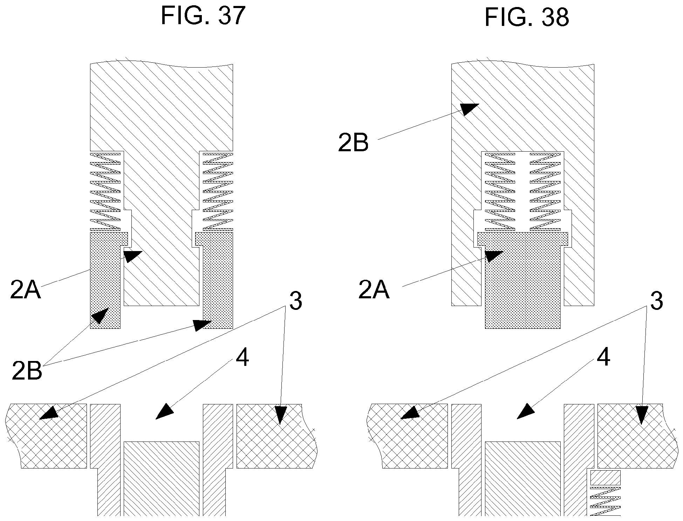

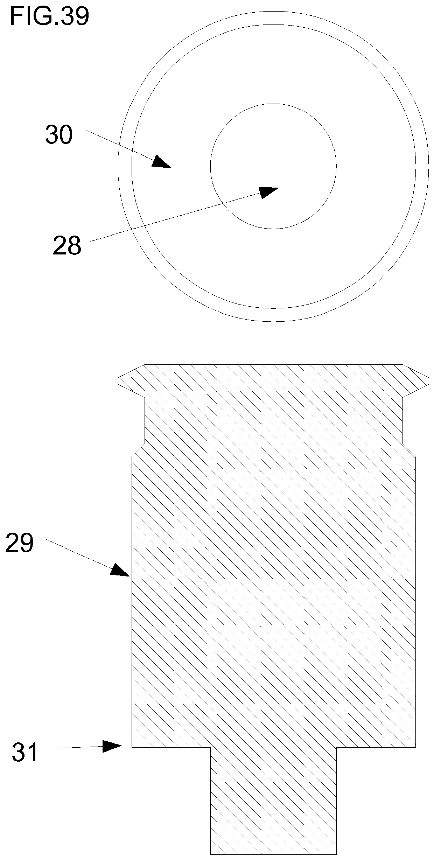

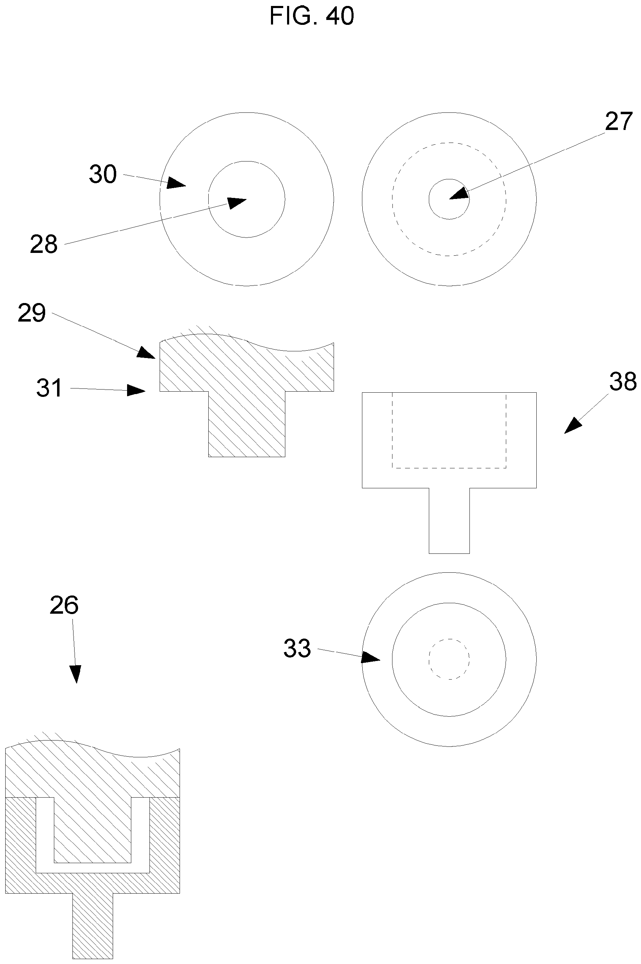

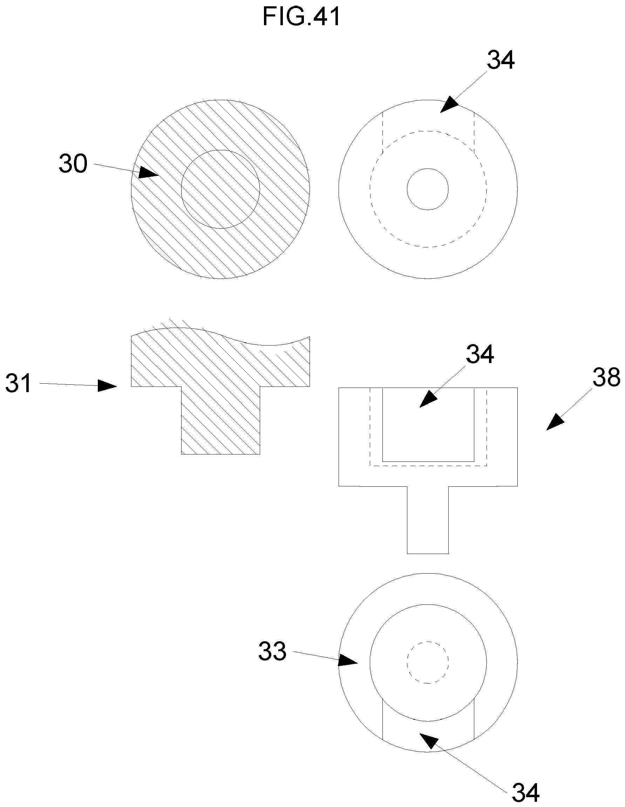

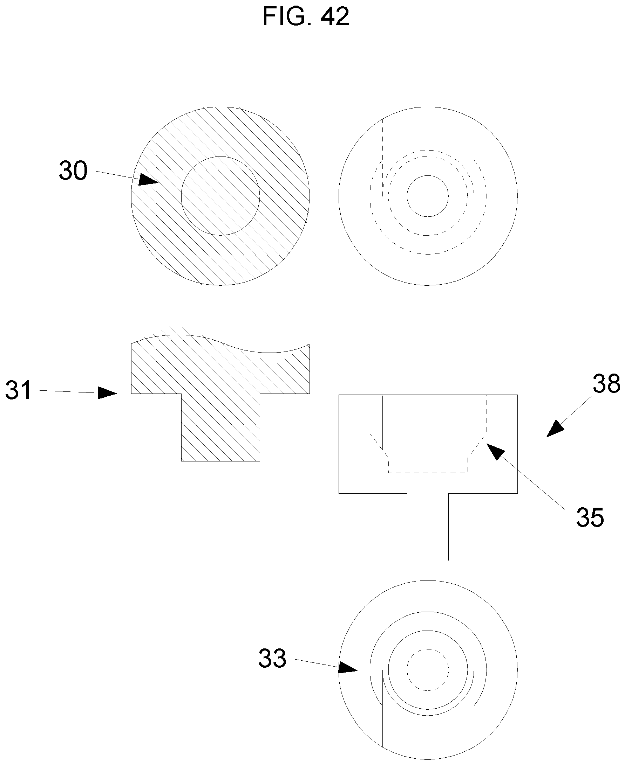

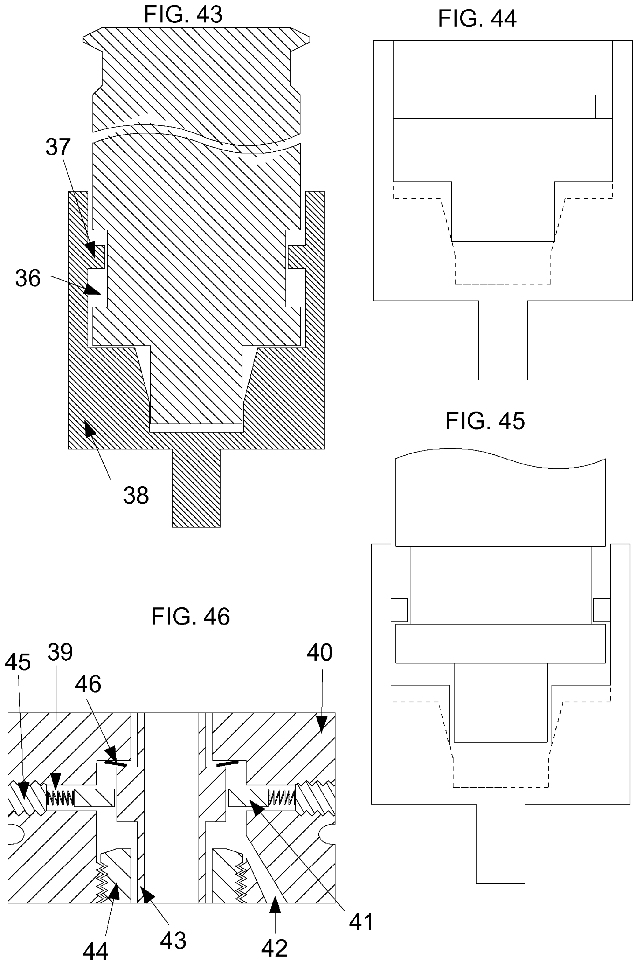

Der Spezielle Matrizeneinsatz (Fig. 46) entspricht von seiner äußeren Form den herkömmlichen Matrizeneinsätzen, besteht also aus einem Scheibenförmigen Körper 40. Der Durchmesser der Bohrung (Matrizenbohrung) und deren Form entsprechen dem Außendurchmesser und der Form des zu pressenden Formteils. Er weist jedoch einen rohrförmigen Einsatz 43 auf, dessen Außendurchmesser geringfügig kleiner ist, als der Durchmesser der Bohrung in dem Matrizeneinsatz, so dass er sich in diesem vertikal verschieben lässt. Der Innendurchmesser entspricht dem Kerndurchmesser des zu pressenden Formteils. Im Prinzip entspricht der rohrförmige Einsatz der Spitze des äußeren Unterstempels, wie er bei der Verwendung von koaxialen Unterstempeln verwendet wird. Der Unterstempel, der bei solchen Ausführungsformen verwendet wird, entspricht dem inneren Unterstempel, wie er bei der Verwendung von koaxialen Unterstempeln verwendet wird. Der Spalt zwischen der Innenwand der Matrizenbohrung und dem rohrförmigen Einsatz ist in Figur 46 breiter dargestellt, als er üblicherweise ausgeführt wird, damit er auf der Zeichnung zu erkennen ist.

Der Matrizeneinsatz ist vorzugsweise so ausgeführt, dass der rohrförmige Einsatz nur dann vertikal verschiebbar ist, wenn eine vertikale Kraft auf ihn ausgeübt wird, die ein bestimmtes Maß überschreitet. Dies kann durch verschiedene Maßnahmen realisiert sein. Beispielsweise ist der rohrförmige Einsatz bei Verwendung einer kreisrunden Bohrung im Matrizeneinsatz an seinem äußeren Umfang nicht ganz rund, sondern leicht oval, wobei er dann zwei Außendurchmesser aufweist, von denen einer geringfügig kleiner ist als die Bohrung im Matrizeneinsatz und der andere noch etwas geringfügiger größer. Auch der innere Umfang ist dann vorzugsweise leicht oval, wobei die Achsen der Ovale einander entsprechen, der Unterschied zwischen dem maximalen und minimalen Innendurchmesser aber weniger groß ist, als der zwischen dem maximalen und minimalen Außendurchmesser. Zum Einführen des rohrförmigen Einsatz in die Bohrung des Matrizeneinsatz wird dieser an den gegenüberliegenden zylindrischen Seitenflächen mit dem maximalen Durchmesser zusammengepresst, so dass er unter Spannung eine weitgehend runde Form annimmt und in die Bohrung eingeführt werden kann. Dort kehrt er soweit wie möglich in seine ovale Form zurück und liegt dann an den gegenüberliegenden zylindrischen Seitenflächen mit dem maximalen Durchmesser an der Innenwandung der Bohrung an und wird durch die entstehende Reibung in seiner vertikalen Position so fixiert, dass er nur dann vertikal gegenüber dem Matrizeneinsatz bewegt werden kann, wenn die auf ihn einwirkende Kraft die Haftreibung überschreitet. Die Haftreibungskraft kann über die Formgebung und die Materialeigenschaften eingestellt werden. In den Figuren 54 bis 59 sind einige Schritte des Verfahrens mit einem solchen rohrförmigen Einsatz gezeigt. Dort ist auch zu sehen, wie der rohrförmige Einsatz durch Oberstempel nach unten und durch Unterstempel nach oben bewegt wird, wenn diese mit ihrem im Durchmesser verbreiteten Bereich an den rohrförmigen Einsatz anstoßen und bei ihrer Weiterbewegung die Haftreibung überwinden.

In einer weiteren Ausführungsform weist der Matrizeneinsatz von seiner Unterseite her eine weitere Bohrung auf, die koaxial zu der Matrizenbohrung ist, jedoch einen größeren Durchmesser bzw. größere Abmaße hat. Diese Bohrung ist nur so tief, dass die Matrizenbohrung mindestens bis zur maximalen Fülltiefe an zu verpressendem Material in ihrem ursprünglichen Durchmesser erhalten bleibt. Vorzugsweise ist am unteren Ende dieser Bohrung eine Möglichkeit zum Einsatz bzw. zur Befestigung einer Verschlussscheibe 44 vorgesehen, z.B. ein Innengewinde. Dort kann eine Verschlusscheibe eingesetzt werden, welche ebenfalls eine Bohrung aufweist, die vorzugsweise der Matrizenbohrung entspricht. Der rohrförmige Einsatz weist einen Bereich auf, in dem der Außendurchmesser vergrößert ist, jedoch nicht größer als der Durchmesser der vorbeschriebenen Bohrung in der Unterseite des Matrizeneinsatzes. Dieser Bereich ist vorzugsweise so weit vom oberen Ende des rohrförmigen Einsatzes entfernt, dass die Oberkante des rohtförmigen Einsatzes bündig mit der Oberkante des Matrizeneinsatzes ist, wenn der rohrförmige Einsatz mit dem oberen Ende des Bereichs am Ende der vergrößerten Bohrung anschlägt, womit der Verscheibeweg des rohrförmigen Einsatzes nach oben begrenzt wird. Der Bereich ist vorzugsweise so weit vom unteren Ende des rohrförmigen Einsatzes entfernt, dass die Oberkante des rohrförmigen Einsatzes sich noch oberhalb des Endes der vergrößerten Bohrung befindet, wenn der rohrförmige Einsatz mit dem unteren Ende des Bereichs an der Verschlusscheibe anschlägt, womit der Verscheibeweg des rohrförmigen Einsatzes nach unten begrenzt wird.

Der Matrizeneinsatz ist vorzugsweise so ausgeführt, dass der rohrförmige Einsatz nur dann vertikal verschiebbar ist, wenn eine vertikale Kraft auf ihn ausgeübt wird, die ein bestimmtes Maß überschreitet. Dies kann durch verschiedene Maßnahmen realisiert sein. Beispielsweise kann wie bei der vorab beschriebenen Ausführungsform der rohrförmige Einsatz so verformt sein, dass er an der Wandung der Matrizenbohrung reibt.

In einer bevorzugten Ausführungsform ist in dem Spalt zwischen der Wandung der vergrößerten Bohrung im Matrizeneinsatz und dem Bereich des rohrförmigen Einsatzes, der einen vergrößerten Außendurchmesser aufweist, eine Art Reibungsbremse angeordnet. Diese besteht vorzugsweise aus einem streifen aus federndem Material, beispielsweise einem Streifen aus Federstahlblech, welcher wellenförmig geformt ist und zu einem offenen Ring geformt in den Spalt eingepresst wird.

In einer anderen Ausführungsform sind in dem Matrizeneinsatz eine oder mehrere Bohrungen eingebracht, die von der Umfangsfläche her radial zur vergrößerten Bohrung hin führen. In diese Bohrungen sind Bremselemente 41 eingesetzt, beispielsweise zylindrische Stäbe aus einem Material mit einer definierten Haftreibung gegenüber dem Material des rohrförmigen Einsatzes. Radial weiter außen angeordnet sind Federelemente 39, beispielsweise Spiralfedern und ein oder mehrere Verschlussstücke 45, beispielsweise Madenschrauben. Die durch die Verschlusstücke nach außen hin fixierten Federelemente drücken die Bremselemente gegen den im Durchmesser vergrößerten Bereich des rohrförmigen Einsatzes und erlauben dessen vertikale Bewegung erst dann, wenn die axial auf ihn einwirkende Kraft die Haftreibung überschreitet.

Die Haftreibung ist vorzugsweise so groß, dass der rohrförmige Einsatz nur dann bewegt wird, wenn er durch den Kontakt mit einem Oberstempel, Unterstempel oder Zwischenstempel und dessen weiter gehende Bewegung auf den rohrförmigen Einsatz zu bewegt wird.

Der Rohrförmige Einsatz wird also nur durch einen Ober- oder Zwischenstempel nach unten bewegt, dessen Durchmesser größer ist, als der Innendurchmesser des rohrförmigen Einsatzes.

Er wird nur durch einen Unterstempel nach oben bewegt, dessen Durchmesser größer ist, als der Innendurchmesser des rohrförmigen Einsatzes, bzw. durch einen im Durchmesser verbreiterten Bereich des Unterstempels, falls der Durchmesser des Unterstempels an sich bzw. dessen Pressfläche nicht größer ist.

Üblicherweise weist der Unterstempel, der in solchen Ausführungsformen mit einem speziellen Matrizeneinsatz zur Anwendung kommt, einen Bereich auf, der im Durchmesser gegenüber seiner Pressfläche vergrößert ist. Der vertikale Abstand dieses Bereiches zur Außenkante seiner Pressfläche ist vorzugsweise gleich der Länge des rohrförmigen Einsatzes. Somit wird der rohrförmige Einsatz immer dann vom Unterstempel nach oben bewegt, wenn die Pressfläche des Unterstempels auf einer Höhe mit der Oberkante des rohrförmigen Einsatzes liegt, und der Unterstempel sich noch weiter nach oben bewegt.

In einer bevorzugten Ausführungsform ist zwischen dem Ende der vergrößerten Bohrung und dem oberen Ende des Bereiches des rohrförmigen Einsatzes mit einem vergrößerten Durchmesser eine Federung 46 eingebracht, vorzugsweise in Form einer Tellerfeder. Vorzugsweise ist die Vorspannung dieser Federung so stark, dass sie nicht bei normalen Pressvorgängen beispielsweise durch Zusammenwirkung von einer Aufwärtsbewegung des Unterstempels mit der Haftung des Formpresslings an der Innenwand des rohrförmigen Einsatzes überschritten wird. Wird jedoch der Unterstempel so weit angehoben, dass der Bereich, in dem sein Durchmesser gegenüber der Pressfläche vergrößert ist, einen geringeren Abstand zur Oberkante des Matrizeneinsatzes hat, als der rohrförmige Einsatz lang ist, so wird die Federung zusammengepresst und die Oberkante des rohrförmigen Einsatzes erhebt sich vorzugsweise mit der Pressfläche des Unterstempels über die Oberkante des Matrizeneinsatzes hinaus. Dies kann vorteilhaft beim Ausstoßschritt sein, da bei diesem die Pressfläche des Unterstempels vorzugsweise mindestens bündig mit der Oberkante des Matrizeneinsatzes sein soll. Aufgrund unvermeidbarer Toleranzen kann es dazu kommen, dass der Oberstempel beim Ausstoßschritt so weit angehoben wird, dass seine Pressfläche etwas über die Oberkante des Matrizeneinsatzes hinaus steht. Wäre der Anschlag des rohrförmigen Einsatzes nicht entsprechend gefedert, könnte es zu einer übermäßigen Belastung des Anschlages, des rohrförmigen Einsatzes, des Unterstempels oder des Matrizeneinsatzes kommen. Unter Umständen würde der Unterstempel den Matrizeneinsatz aus seiner Befestigung lösen.

Durch die Federung des Anschlags kann der rohrförmige Einsatz der Bewegung des Unterstempels folgen und eine übermößige Belastung der mechanischen Komponenten kann vermieden werden.

Vorzugsweise sind in dem Matrizeneinsatz eine oder mehrere weitere Bohrungen 42 eingebracht, welche vom Zwischenraum zwischen der Matrizenbohrung oder der vergrößerten Bohrung und dem rohrförmigen Einsatz nach unten hin an die Unterseite des Matrizeneinsatzes verlaufen. Durch diese Bohrungen kann Füllmaterial, welches durch den Spalt zwischen der Matrizenbohrung und dem rohrförmigen Einsatz in den Matrizeneinsatz eindringt, nach unten entweichen.

In weiteren Ausführungsformen werden keine einzelnen Matrizeneinsätze verwendet, sondern die Bohrungen in der Matrizenscheibe sind entsprechen den vorbeschriebenen Bohrungen in den Matrizeneinsätzen ausgeführt und die rohrförmigen Einsätze oder sonstige Konstruktionen sind direkt in der Matrizenscheibe angeordnet bzw. in diese eingebaut.

Bei Ausführungsformen, bei denen ein spezieller Matrizeneinsatz entsprechend der vorbeschriebenen Ausführungsformen verwendet wird, kommt vorzugsweise kein koaxialer Unterstempel zur Anwendung. Bei solchen Ausführungsformen ist zu Beginn des Verfahrens zur Herstellung eines Formteils mit einem Kern der rohrförmige Einsatz mit seiner Oberkante bündig zur Oberkante des Matrizeneinsatzes. Entsprechend dem Verfahren mit koaxialem Unterstempel werden die erste Hüllschicht und die Kernschicht befüllt und verpresst. Ggf. werden weitere Kernschichten und Zwischenschichten eingefüllt und verpresst. Beim letzten dieser Pressschritte kommt ein Oberstempel oder Zwischenstempel zum Einsatz, dessen Pressfläche entweder direkt der Außenkontur des rohrförmigen Einsatzes entspricht oder der etwas oberhalb der Pressfläche, welche der Innenkontur des rohrförmigen Einsatzes entspricht, einen Bereich mit einer Vergrößerung des Durchmessers aufweist, welche der Außenkontur des rohrförmigen Einsatzes entspricht. Bei diesem Pressschritt wird dieser Oberstempel oder Zwischenstempel so weit nach unten bewegt, dass er mit seiner Pressfläche oder der unterseite des im Duchmesser vergrößerten Bereichs den rohrförmigen Einsatu nach unten bewegt. Der Unterstempel weicht dabei so weit nach unten zurück, dass die Verpressung vorzugsweise erst dann vollständig erfolgt ist, wenn die Oberkante des rohrförmigen Einsatzes so weit nach unten bewegt ist, dass die darüber einfüllbare Menge an zweitem Hüllmaterial ausreichend für die Ausbildung der seitlichen und oberen Hüllschicht ist. Nach Anhebung des Oberstempels oder Zwischenstempels wird der entstandene Hohlraum mit dem zweiten Hüllmaterial befüllt, vorzugsweise indem die Matrize unter einem Füllschuh entlang geführt wird. Während der Füllung wird der Unterstempel nach oben bewegt, so dass das zwei- oder Merhschichtige Teilformteil ebenfalls nach oben bewegt wird. Hierdurch verdrängt es einen Teil des bereits eingefüllten zweiten Hüllmaterials nach oben. Das sich seitlich davon befindende bereits eingefüllte zweite Hüllmaterial verhindert eine dezentrierung des Teilformteils. Der rohrförmige Einsatz bewegt sich dabei nicht nach oben, da er beispielsweise durch die Bremselemente daran gehindert ist. Sobald die Oberkante des Unterstempels bündig mit der Oberkante des rohrförmigen Einsatzes ist, wird der rohrförmige Einsatz gleichförmig mit nach oben bewegt. Beispielsweise weist der Unterstempel hierzu unterhalb seiner Pressfläche eine Vergrößerung seines Durchmessers auf. Der vergrößerte Durchmesser entspricht dem Außenmaß des nicht im Duchmesser vergrößerten Bereich des rohrförmigen Einsatzes. Der Abstand zwischen der Pressfläche und der Vergrößerung des Unterstempels entspricht vorzugsweise der Länge des rohrförmigen Einsatzes. Zuviel eingefülltes zweites Hüllmaterial wird dabei wieder in den Füllschuh zurückgeschoben. Durch die Höhe der Anhebung kann die Menge des in der Matrize verbleibenden zweiten Hüllmaterials eingestellt werden. Danach wird der Oberstempel dessen Pressfläche der Außenkontur des rohrförmigen Einsatzes entspricht oder ein entsprechender Zwischenstempel abgesenkt und das zweite Hüllmaterial wird oberhalb und seitlich um das Teilformteil herum verpresst. Durch den Kontakt mit dem im Durchmesser vergrößerten Bereich des Unterstempels kann der rohrförmige Einsatz nicht nach unten entweichen und somit bildet seine obere (beispielsweise ringförmige) Oberfläche zusammen mit der Pressfläche des Unterstempels eine gemeinsame untere Pressfläche in größe und Form des zu produzierenden Formteils. Beim anschließenden Ausstoßschritt wird der Unterstempel so weit angehoben, dass seine Pressfläche mindestens bis auf die Höhe der Oberkante des Matrizeneinsatzes angehoben wird, so dass das Kernformteil vorzugsweise mittels eines Abstreifers aus der Formpresseinrichtung entnommen werden kann. Dabei wird auch der rohrförmige Einsatz so weit angehoben, dass ein weiterer Pressvorgang beginnen kann.

Ausführungsformen, bei denen der Einsatz von Rastmechanismen beschrieben ist, können statt mit Rastmechanismen auch mit gezielter Ausnutzung von Reibungskräften ausgeführt werden, vorzugsweise mit Ausnutzung von Haftreibungskräften. Beispielsweise kann statt einer beschriebene Rastung des äußeren Unterstempels relativ zur Matrize auch eine Reibung des Schaftes des Unterstempels gegenüber der Unterstempelführung genutzt werden. Solche Ausnutzungen von Reibungskräften sind im Stand der Technik bekannt, beispielsweise wird die vertikale Beweglichkeit des Unterstempels gegenüber seiner Stempelführung gebremst um nach einem schnellen Anhebevorgang des Unterstempels ein Abheben des Stempelkopfes von seiner Steuerungsbahn zu verhindern. Hier werden ähnliche Konstruktionen und Methoden verwendet, wie sie bei der speziellen Matrize bzw. dem speziellen Matrizeneinsatz beschrieben sind. Die Reibungskräfte sind unter anderem durch die verwendeten Materialien und deren Oberflächenbeschaffenheit einstellbar. Vorzugsweise sind die eingestellten Haftreibungskräfte größer als die Reibungskräfte, die beim Verpressen von Hüll- oder Kernmaterial auf die per Haftreibung fixierten mechanischen Teile wie z.B. den äußeren Unterstempel oder den rohrförmigen Einsatz der speziellen Matrize einwirken.

Besonders bevorzugte Ausführungsformen sind Verfahren, bei denen folgende Formpressvorrichtungen verwendet werden:
Formpressvorrichtungen, die bei einem Press-Schritt einen Zwischenstempel unterhalb des oberen Stempels positionieren können, bei denen das untere Hüllmaterial und das (erste) Kernmaterial beim gleichen Press-Schritt mithilfe des Zwischenstempels verpresst werden können und bei denen der letzte Press-Schritt ausgeführt wird, ohne dass ein Zwischenstempel unterhalb des oberen Stempels positioniert wird.

Formpressvorrichtungen, die bei einem Press-Schritt einen Zwischenstempel unterhalb des oberen Stempels positionieren, bei denen der letzte Press-Schritt mithilfe des Zwischenstempels ausgeführt wird und bei denen die erste Schicht Hüllmaterial und das Kernmaterial in einzelnen Press-Schritten oder in einem gemeinsamen Press-Schritt verpresst werden, ohne dass ein Zwischenstempel unterhalb des oberen Stempels positioniert wird.

Formpressvorrichtungen, die bei zwei oder mehr Press-Schritten Zwischenstempel unterhalb des oberen Stempels positionieren, bei denen das untere Hüllmaterial und das Kernmaterial und ggf. weitere Schichten von Hüll- und Kernmaterialien) mithilfe des oder der Zwischenstempel verpresst werden und bei denen der letzte Press-Schritt ausgeführt wird, ohne dass ein Zwischenstempel unterhalb des oberen Stempels positioniert wird.

Formpressvorrichtungen, die bei zwei oder mehr Press-Schritten Zwischenstempel unterhalb des oberen Stempels positionieren, bei denen das untere Hüllmaterial und das Kernmaterial und ggf. weitere Schichten von Hüll- und Kernmaterialien) mithilfe des oder der Zwischenstempel verpresst werden und bei denen der letzte Press-Schritt ebenfalls mithilfe eines Zwischenstempels ausgeführt wird.

Vorgenannte Formpressvorrichtungen, bei denen der oder die unteren Stempel mindestens zweiteilig koaxial ausgeführt sind.

Vorgenannte Formpressvorrichtungen, bei denen der oder die unteren Stempel nicht koaxial ausgeführt sind, wobei der Stempel, der beim Press-Schritt für das als erstes eingefüllte Hüllmaterial verwendet wird, so ausgeführt ist, dass er beim Verpressen eine Mulde in das eingefüllte Hüllmaterial presst, in das nachfolgend das Kernmaterial eingefüllt werden kann.

Folgende Ausführungsformen sind ebenfalls besonders bevorzugt:
Verfahren zur Herstellung eines Formteils mit einem Kern unter Verwendung einer Formpressvorrichtung, die einen oberen Stempel und einen unteren Stempel umfasst, die in Vertikalrichtung einer Pressform angeordnet sind, wobei zumindest einer von dem oberen Stempel und dem unteren Stempel einen zweiteiligen Aufbau hat, der aus einem inneren Stempel und einem äußeren Stempel besteht, der den äußeren Rand des inneren Stempels umgibt,
bei denen der untere Stempel einen mindestens zweiteiligen Aufbau hat, der mindestens aus einem inneren Stempel und einem äußeren Stempel besteht, der den äußeren Rand des inneren Stempels umgibt, wobei der äußere Stempel gegenüber dem inneren Stempel, gegenüber der Matrize oder gegenüber seiner Führung federnd gelagert ist.

Verfahren wie vorstehend beschrieben, bei dem der Ablauf des Verfahrens folgende Schritte umfasst:
- einen Zufuhr-Schritt 1 der äußeren Schicht, bei dem Formmaterial für einen ersten Teil einer äußeren Schicht in eine Aussparung oberhalb des unteren mittleren Stempels zugeführt wird, die vom unteren äußeren Stempel umschlossen ist;
- einen Kern-Zufuhr-Schritt, bei dem das Formmaterial für den Kern in eine Aussparung oberhalb des Formmaterials für die erste äußere Schicht, das im vorangegangenen Schritt zugeführt wurde, und umschlossen vom unteren äußeren Stempel zugeführt wird;
- einen Press-Schritt der äußeren Schicht und des Kerns, bei dem das in den vorangegangenen Schritten zugeführte Formmaterial für die erste äußere Schicht und das Formmaterial für den Kern durch den Zwischenstempel und unteren inneren Stempel formgepresst werden;
- einen Zufuhr-Schritt 2 der äußeren Schicht, bei dem Formmaterial für einen zweiten Teil der äußeren Schicht in eine Aussparung in einer Pressform oberhalb und um die erste äußere Schicht und den Kern herum, die im vorangegangenen Schritt geformt wurden, zugeführt wird,
- einen Gesamtpress-Schritt, bei dem die erste äußere Schicht, der Kern und das Formmaterial für die zweite äußere Schicht, welche im vorangegangenen Schritt zugeführt wurden, vom unteren und oberen Stempel formgepresst werden; und
- einen Schritt des Entfernens des formgepressten Formteils, der nach dem Gesamtpress-Schritt durchgeführt wird.

Verfahren wie vorstehend beschrieben, bei dem zwischen dem Zufuhr-Schritt 1 und dem Kern-Zufuhr-Schritt mindestens einen Press-Schritt der äußeren Schicht umfasst, bei dem das Formteil für die erste äußere Schicht formgepresst wird.

Eine ebenfalls bevorzugte Ausführungsform der Erfindung ist ein Stempel zur Verpressung eines Formteils der einen mindestens zweiteiligen Aufbau hat, der mindestens aus einem inneren Stempel und einem äußeren Stempel besteht, der den äußeren Rand des inneren Stempels umgibt, wobei der äußere Stempel gegenüber dem inneren Stempel, gegenüber der Matrize oder gegenüber seiner Führung federnd gelagert ist.

Durch Verwendung eines oder mehrerer, vorzugsweise verschiedener Zwischenstempel ist es möglich, Durchmesser, Form, Kontur oder andere Eigenschaften der von oben auf die eingefüllten Materialien einwirkenden Pressfläche zu variieren, ohne den eigentlichen oberen Stempel wechseln zu müssen oder einen mehrteiligen koaxialen oberen Stempel einzusetzen.

Es kann bei einem, bei mehreren oder bei jedem der verschiedenen Press-Schritte jeweils ein individueller Zwischenstempel eingesetzt werden. Bei entsprechender Mitführung über einen größeren Teilabschnitt können Zwischenstempel auch für mehrere Press-Schritte eingesetzt werden.

Vorzugsweise wird zumindest bei einem Press-Schritt kein Zwischenstempel eingesetzt (unterhalb des oberen Stempels positioniert). Dadurch lässt sich zumindest für einen Press-Schritt die Mitführungsmechanik für den Zwischenstempel einsparen. Sind die erforderlichen Eigenschaften der Pressfläche (Durchmesser, Form, Kontur etc.) für mehrere Press-Schritte gleich, ist es vorteilhaft, für diese Press-Schritte keine Zwischenstempel einzusetzen, sondern direkt mit dem oberen Stempel zu verpressen, da so die Anzahl der erforderlichen Zwischenstempel minimiert werden kann.

Es ist vorteilhaft, für denjenigen Press-Schritt keinen Zwischenstempel einzusetzen, der die größte Press-Kraft erfordert. Dadurch wird die maximale mechanische Belastung der Zwischenstempel reduziert.

Mit den beschriebenen Ausführungsformen der Erfindung ist es unter anderem möglich, Formteile mit Kern herzustellen, ohne darauf angewiesen zu sein, dass der obere Stempel einen zweiteiligen Aufbau hat, der aus einem inneren Stempel und einem äußeren Stempel besteht und bei dem der äußere Stempel den äußeren Rand des inneren Stempels umgibt.

Die Ausführungsformen mit federnder Lagerung sind nicht auf die Verwendung von Federn wie z.B. Schrauben oder Tellerfedern beschränkt, sondern können mit jeder passenden Art von Federung ausgeführt werden. Sie können alternativ auch per Pneumatik oder Hydraulik ausgeführt werden. Beispielsweise können Tellerfederstapel durch entsprechende Luftdruckzylinder ersetzt werden.

Wortdefinitionen:
"oberer Stempel" bedeutet im Wesentlichen das gleiche wie "Oberstempel".
"unterer Stempel" bedeutet im Wesentlichen das gleiche wie "Unterstempel".
"oberer Außenstempel" und "oberer äußerer Stempel" bedeuten im Wesentlichen das gleiche wie "äußerer Oberstempel". "oberer Innenstempel" und "oberer innerer Stempel" bedeuten im Wesentlichen das gleiche wie "innerer Oberstempel". "unterer Außenstempel" und "unterer äußerer Stempel" bedeuten im Wesentlichen das gleiche wie "äußerer Unterstempel". "unterer Innenstempel" und "unterer innerer Stempel" bedeuten im Wesentlichen das gleiche wie "innerer Unterstempel". "äußere Schicht" bedeutet im Wesentlichen das gleiche wie "Hüllschicht", "Hülle" oder "Umhüllung".
"Ausführung" bedeutet im Wesentlichen das gleiche wie "Ausgestaltung".

Die Zeichnungen zeigen diverse Ausführungen der Erfindung.
Zeichnung Nr. 1 zeigt die Stellung beim ersten Füllschritt.
Zeichnung Nr. 3 zeigt zusätzlich das eingefüllte Hüllmaterial.
Zeichnung Nr. 5 zeigt die Stellung beim ersten Pressschritt.
Zeichnung Nr. 7 zeigt zusätzlich das Teilformteil.
Zeichnung Nr. 2 zeigt die Stellung beim zweiten Füllschritt.
Zeichnung Nr. 4 zeigt zusätzlich das eingefüllte Kernmaterial.
Zeichnung Nr. 9 zeigt die Stellung beim zweiten Pressschritt.
Zeichnung Nr. 11 zeigt zusätzlich das Teilformteil.
Zeichnung Nr. 10 zeigt die Stellung beim dritten Füllschritt.
Zeichnung Nr. 12 zeigt zusätzlich das eingefüllte Hüllmaterial.
Zeichnung Nr. 13 zeigt die Stellung beim dritten Pressschritt.
Zeichnung Nr. 14 zeigt zusätzlich das Formteil.

Die Zeichnungen zeigen jeweils Querschnitte von Rotationskörpern (zur Herstellung von kreisförmig zylindrischen Formteilen). Ausgenommen hiervon ist der seitlich in den Stempel eingesetzte Rastmechanismus Element 13 in Zeichnung 23).

Die dargestellten Federn können als Tellerfedern ausgebildet sein, es können aber auch Schraubenfedern verwendet werden. Die Federn können sowohl konzentrisch um die Stempelachse verlaufende Einzelfedern sein, als auch über den Umfang verteilte mehrfache Federelemente.

Wenn nicht direkt anders benannt, bezeichnet 1 den unteren Stempel (1A Innenstempel, 1B Außenstempel), 2 den oberen Stempel, 3 die Matrize, 4 den Füllraum, 5 das eingefüllte Rohmaterial bzw. das Formteil oder Teilformteil.

Die restlichen Zeichnungsinhalte sind im Text beschrieben.

## Patentansprüche

1. Verfahren zur Herstellung eines Formteils mit einem Kern unter Verwendung einer Formpressvorrichtung, die einen oberen Stempel (2) und einen unteren Stempel (1) umfasst, die in Vertikalrichtung einer Pressform (3) angeordnet sind, sowie einen Zwischenstempel (38) und eine Vorrichtung, mit welcher der Zwischenstempel zwischen dem oberen Stempel und der Pressform positioniert und aus dieser Position entfernt werden kann, **gekennzeichnet dadurch, dass** der Zwischenstempel an seiner Unterseite eine Pressfläche (27) aufweist, mit welcher Hüll- und/oder Kernmaterial verpresst werden kann, und dass
das Material, aus dem der Kern besteht, in die Pressform eingebracht wird, bevor es mit dem Zwischenstempel in Kontakt kommt,
und/oder
das Kernmaterial beim Einbringen in die Pressform aus mindestens zwei miteinander nicht fest verbundenen Partikeln besteht, beispielsweise aus Pulver, aus Granulat, mehreren Kristallen oder Mikropellets,
und/oder
der Zwischenstempel weder eine Haltevorrichtung für das Kernmaterial aufweist, noch das Kernmaterial aus einer Haltevorrichtung in die Pressform befördert.

2. Verfahren nach Anspruch 1, **gekennzeichnet dadurch, dass**
der Zwischenstempel weder eine Haltevorrichtung für das Kernmaterial aufweist, noch das Kernmaterial aus einer Haltevorrichtung in die Pressform befördert.

3. Verfahren nach Anspruch 1, **gekennzeichnet dadurch, dass**
das Material, aus dem der Kern besteht, in die Pressform eingebracht wird, bevor es mit dem Zwischenstempel in Kontakt kommt,
und
das Kernmaterial beim Einbringen in die Pressform aus mindestens zwei miteinander nicht fest verbundenen Partikeln besteht, beispielsweise aus Pulver, aus Granulat, mehreren Kristallen oder Mikropellets,
und
der Zwischenstempel weder eine Haltevorrichtung für das Kernmaterial aufweist, noch das Kernmaterial aus einer Haltevorrichtung in die Pressform befördert.

4. Verfahren nach einem der vorhergehenden Ansprüche, **gekennzeichnet dadurch, dass**
der untere Stempel einen mindestens zweiteiligen Aufbau hat, der einen inneren Stempel und einem äußeren Stempel umfasst, wobei der äußere Stempel den äußeren Rand des inneren Stempels umgibt, wobei sowohl der innere Stempel als auch der äußere Stempel sowohl Schiebebewegungen als auch Komprimierungsvorgänge ausführen können, und dass der untere Stempel vorzugsweise einen dreiteiligen Aufbau hat, welcher im Vergleich zum zweiteiligen Aufbau zusätzlich einen äußersten Stempel umfasst, wobei der äußerste Stempel den äußeren Rand des äußeren Stempels umgibt, und auch der äußerste Stempel sowohl Schiebebewegungen als auch Komprimierungsvorgänge ausführen kann.

5. Verfahren nach einem der vorhergehenden Ansprüche, **gekennzeichnet dadurch, dass**
der obere Stempel keinen zweiteiligen Aufbau, bestehend aus einem inneren Stempel und einem, den äußeren Rand des inneren Stempels umgebenden, äußeren Stempel, hat.

6. Verfahren nach einem der vorhergehenden Ansprüche, **gekennzeichnet dadurch, dass** der Ablauf des Verfahrens umfasst:
- einen Zufuhr-Schritt 1 einer äußeren Schicht, bei dem Formmaterial für einen ersten Teil der äußeren Schicht in eine Aussparung oberhalb des unteren inneren Stempels zugeführt wird, die vom unteren äußeren Stempel umschlossen ist;
- einen Kern-Zufuhr-Schritt, bei dem das Formmaterial für den Kern in eine Aussparung oberhalb des Formmaterials für den ersten Teil der äußeren Schicht, das im vorangegangenen Schritt zugeführt wurde, und umschlossen vom unteren äußeren Stempel zugeführt wird;
- einen Press-Schritt der äußeren Schicht und des Kerns, bei dem das in den vorangegangenen Schritten zugeführte Formmaterial für den ersten Teil der äußeren Schicht und das Formmaterial für den Kern formgepresst werden;
- einen Zufuhr-Schritt 2 der äußeren Schicht, bei dem Formmaterial für einen zweiten Teil der äußeren Schicht in eine Aussparung in einer Pressform oberhalb und/oder um den ersten Teil der äußeren Schicht und den Kern herum, die im vorangegangenen Schritt geformt wurden, zugeführt wird;
- einen Gesamtpress-Schritt, bei dem der erste Teil der äußeren Schicht, der Kern und das Formmaterial für den zweiten Teil der äußeren Schicht, welches im vorangegangenen Schritt zugeführt wurde, formgepresst werden; und
- einen Schritt des Entfernens des formgepressten Formteils, der nach dem Gesamtpress-Schritt durchgeführt wird;
und wobei zumindest bei einem Press-Schritt ein Zwischenstempel zwischen dem oberen Stempel und der Pressform positioniert wird,
und vorzugsweise, dass das Verfahren zwischen dem Zufuhr-Schritt 1 und dem Kern-Zufuhr-Schritt mindestens einen Press-Schritt der äußeren Schicht umfasst, bei dem das Formmaterial für den ersten Teil der äußeren Schicht formgepresst wird.

7. Verfahren nach einem der Ansprüche 1 bis 5, **gekennzeichnet dadurch, dass** der Ablauf des Verfahrens umfasst:
- einen Zufuhr-Schritt 1 einer äußeren Schicht, bei dem Formmaterial für einen ersten Teil der äußeren Schicht in eine Aussparung oberhalb des unteren Stempels zugeführt wird;
- einen Press-Schritt der äußeren Schicht, bei dem eine muldenförmige Vertiefung in den ersten Teil der äußeren Schicht formgepresst wird;
- einen Kern-Zufuhr-Schritt, bei dem das Formmaterial für den Kern in die muldenförmige Vertiefung des ersten Teils der äußeren Schicht zugeführt wird;
- einen Zufuhr-Schritt 2 der äußeren Schicht, bei dem Formmaterial für einen zweiten Teil der äußeren Schicht in die Pressform oberhalb des ersten Teils der äußeren Schicht und des Kerns zugeführt wird;
- einen Gesamtpress-Schritt, bei dem der erste Teil der äußeren Schicht, der Kern und das Formmaterial für den zweiten Teil der äußeren Schicht, welches im vorangegangenen Schritt zugeführt wurde, formgepresst werden; und
- einen Schritt des Entfernens des formgepressten Formteils, der nach dem Gesamtpress-Schritt durchgeführt wird; und
wobei zumindest bei einem Press-Schritt ein Zwischenstempel zwischen dem oberen Stempel und der Pressform positioniert wird, und vorzugsweise, dass in das Verfahren zwischen dem Kern-Zufuhr-Schritt und den Zufuhr-Schritt 2 der äußeren Schicht ein Press-Schritt der äußeren Schicht und des Kerns eingefügt ist, bei dem zumindest das im vorangegangenen Schritt zugeführte Formmaterial für den Kern gepresst wird.

8. Verfahren nach Anspruch 4 oder 5, **gekennzeichnet dadurch, dass** der untere Stempel einen dreiteiligen Aufbau hat, welcher im Vergleich zum zweiteiligen Aufbau zusätzlich einen äußersten Stempel umfasst, wobei der äußerste Stempel den äußeren Rand des äußeren Stempels umgibt, und auch der äußerste Stempel sowohl Schiebebewegungen als auch Komprimierungsvorgänge ausführen kann, und dass der Ablauf des Verfahrens umfasst:
- einen Zufuhr-Schritt 1 einer äußeren Schicht, bei dem Formmaterial für einen ersten Teil der äußeren Schicht in eine Aussparung oberhalb des unteren inneren Stempels zugeführt wird, die vom unteren äußeren Stempel umschlossen ist;
- einen Kern-Zufuhr-Schritt, bei dem das Formmaterial für den Kern in eine Aussparung oberhalb des Formmaterials für den ersten Teil der äußeren Schicht, das im vorangegangenen Schritt zugeführt wurde, und umschlossen vom unteren äußeren Stempel, zugeführt wird;
- einen Press-Schritt der äußeren Schicht und des Kerns, bei dem das in den vorangegangenen Schritten zugeführte Formmaterial für den ersten Teil der äußeren Schicht und das Formmaterial für den Kern formgepresst werden;
- einen Zufuhr-Schritt 2 der äußeren Schicht, bei dem Formmaterial für einen zweiten Teil der äußeren Schicht in eine Aussparung oberhalb und und ggf. seitlich des Formmaterials, das in den vorangegangenen Schritten zugeführt wurde, umschlossen vom unteren äußersten Stempel, zugeführt wird;
- einen Press-Schritt, bei dem der erste Teil der äußeren Schicht, der Kern und das Formmaterial für den zweiten Teil der äußeren Schicht, welches im vorangegangenen Schritt zugeführt wurde, formgepresst werden;
- einen Zufuhr-Schritt einer äußersten Schicht, bei dem Formmaterial für die äußerste Schicht in eine Aussparung oberhalb und ggf. seitlich des Formmaterials, das in den vorangegangenen Schritten zugeführt und verpresst wurde, zugeführt wird;
- einen Absenkungs-Schritt, bei dem der untere innere Stempel und der untere äußere Stempel abgesenkt werden;
- einen Gesamtpress-Schritt, bei dem die äußerste Schicht, die äußere Schicht und das Kernmaterial formgepresst werden; und
- einen Schritt des Entfernens des formgepressten Formteils, der nach dem Gesamtpress-Schritt durchgeführt wird; und
wobei zumindest bei einem Press-Schritt ein Zwischenstempel zwischen dem oberen Stempel und der Pressform positioniert wird.

9. Verfahren nach einem der vorhergehenden Ansprüche, **dadurch gekennzeichnet, dass** der Zwischenstempel bei den Pressschritten des ersten Teils der äußeren Schicht und des Kerns durch den oberen Stempel nach unten bewegt wird.

10. Formpressvorrichtung, die einen oberen Stempel (2) und einen unteren Stempel (1) umfasst, die in Vertikalrichtung einer Pressform (3) angeordnet sind, sowie einen Zwischenstempel (38) und eine Vorrichtung, mithilfe derer der Zwischenstempel zwischen dem oberen Stempel und der Pressform positioniert und aus dieser Position entfernt werden kann, **gekennzeichnet dadurch,**
**dass** der Zwischenstempel an seiner Unterseite eine Pressfläche (27) aufweist, mit welcher Hüll- und/oder Kernmaterial, welches aus mindestens zwei miteinander nicht fest verbundenen Partikeln besteht, beispielsweise aus Pulver, aus Granulat, mehreren Kristallen oder Mikropellets, verpresst werden kann
und/oder
**dass** der Zwischenstempel an seiner Unterseite keine Haltevorrichtung aufweist.

11. Formpressvorrichtung nach Anspruch 10, **gekennzeichnet dadurch, dass**
der Zwischenstempel an seiner Unterseite eine Pressfläche (27) aufweist, mit welcher Hüll- und/oder Kernmaterial, welches aus mindestens zwei miteinander nicht fest verbundenen Partikeln besteht, beispielsweise aus Pulver, aus Granulat, mehreren Kristallen oder Mikropellets, verpresst werden kann.

12. Formpressvorrichtung nach Anspruch 10 oder11, **gekennzeichnet dadurch, dass**
der Zwischenstempel an seiner Unterseite keine Haltevorrichtung aufweist
und/oder
dass die Formpressvorrichtung keine Haltevorrichtung aufweist, aus welcher der Zwischenstempel das Kernmaterial in die Pressform befördert.

13. Formpressvorrichtung nach Anspruch 10, 11 oder 12, **gekennzeichnet dadurch, dass**
der obere Stempel keinen zweiteiligen Aufbau, bestehend aus einem inneren Stempel und einem, den äußeren Rand des inneren Stempels umgebenden, äußeren Stempel, hat.

14. Formpressvorrichtung nach einem der Ansprüche 10-13, **gekennzeichnet dadurch, dass**
der untere Stempel einen mindestens zweiteiligen Aufbau hat, der einen inneren Stempel und einem äußeren Stempel umfasst, wobei der äußere Stempel den äußeren Rand des inneren Stempels umgibt, wobei sowohl der innere Stempel als auch der äußere Stempel sowohl Schiebebewegungen als auch Komprimierungsvorgänge ausführen können, und dass der untere Stempel vorzugsweise einen dreiteiligen Aufbau hat, welcher im Vergleich zum zweiteiligen Aufbau zusätzlich einen äußersten Stempel umfasst, wobei der äußerste Stempel den äußeren Rand des äußeren Stempels umgibt, und auch der äußerste Stempel sowohl Schiebebewegungen als auch Komprimierungsvorgänge ausführen kann.

15. Verfahren nach einem der Ansprüche 1-9 oder 17 oder Formpressvorrichtung nach einem der Ansprüche 10-14, **gekennzeichnet dadurch, dass** die Formpressvorrichtung eine Matrize oder einen Matrizeneinsatz (40) mit einem rohrförmigen Einsatz (43) aufweist, wobei der rohrförmige Einsatz gegenüber der Matrize oder dem Matrizeneinsatz vertikal verschiebbar ist,
und vorzugsweise, dass der rohrförmige Einsatz (43) bei auf Anspruch 6 oder 17 basierenden Verfahren oder bei auf Anspruch 14 basierenden Formpressvorrichtungen mit zweiteiligem Unterstempel die Funktionen des äußeren Unterstempels wahrnimmt und der Unterstempel statt zweiteilig koaxial nur noch einteilig ist,
und vorzugsweise, dass der rohrförmige Einsatz (43) bei auf Anspruch 8 basierenden Verfahren oder bei auf Anspruch 14 basierenden Formpressvorrichtungen mit dreiteiligem Unterstempel die Funktionen des äußersten Unterstempels wahrnimmt und der Unterstempel statt dreiteilig koaxial nur noch zweiteilig koaxial ist.

16. Verfahren oder Formpressvorrichtung nach Anspruch 15, **gekennzeichnet dadurch, dass** die vertikale Verschiebbarkeit des rohrförmigen Einsatzes (43) gegenüber der Matrize oder dem Matrizeneinsatz (40) limitiert ist durch einen oder mehrere Anschläge und/oder dadurch dass eine bestimmte Kraft für die Verschiebung erforderlich ist.

17. Verfahren nach einem der Ansprüche 1 bis 5, **gekennzeichnet dadurch, dass** der Ablauf des Verfahrens umfasst:
- einen Kern-Zufuhr-Schritt, bei dem das Formmaterial für den Kern in eine Aussparung oberhalb des unteren inneren Stempels, umschlossen vom unteren äußeren Stempel, zugeführt wird;
- einen Press-Schritt des Kerns, bei dem das im vorangegangenen Schritt zugeführte Formmaterial für den Kern formgepresst wird;
- einen Verschiebungs-Schritt, bei dem der untere äußere Stempel abgesenkt wird und/oder der untere innere Stempel angehoben wird;
- einen Zufuhr-Schritt einer äußeren Schicht, bei dem Formmaterial für die äußere Schicht (47) in eine Aussparung oberhalb und seitlich des Formmaterials, das in den vorangegangenen Schritten zugeführt und verpresst wurde, sowie seitlich des unteren inneren Stempels zugeführt wird;
- einen Absenkungs-Schritt, bei dem der untere innere Stempel abgesenkt wird;
- einen Gesamtpress-Schritt, bei dem die die äußere Schicht und das Kernmaterial formgepresst werden; und
- einen Schritt des Entfernens des formgepressten Formteils, der nach dem Gesamtpress-Schritt durchgeführt wird;
wobei der Verschiebungs-Schritt vor, nach oder während der Ausführung des Zufuhr-Schrittes der äußeren Schicht ausgeführt werden kann,
und
wobei zumindest bei einem Press-Schritt ein Zwischenstempel zwischen dem oberen Stempel und der Pressform positioniert wird.

## Claims

1. A method for the production of a molding with a core using a compression molding apparatus comprising an upper punch (2) and a lower punch (1) which are arranged in the vertical direction of a die (3), and also an intermediate punch (38), and a device, with which the intermediate punch can be positioned between the upper punch and the die and be removed from this position, **characterized in that** the intermediate punch has a pressing surface (27) on its underside, with which shell and/or core material can be compressed, and that
the material, which the core consists of, is inserted into the die before it comes into contact with the intermediate punch,
and / or
the core material, when inserted into the die, consists of at least two particles which are not firmly connected to each other, for example, of powders, of granules, several crystals or micro-pellets,
and / or
the intermediate punch neither has a holdfast for the core material, nor transfers the core material from a holdfast into the die.

2. A method according to claim 1, **characterized in that**
the intermediate punch neither has a holdfast for the core material, nor transfers the core material from a holdfast into the die.

3. A method according to claim 1, **characterized in that**
the material, which the core consists of, is inserted into the die before it comes into contact with the intermediate punch,
and
the core material, when inserted into the die, consists of at least two particles which are not firmly connected to each other, for example, of powders, of granules, several crystals or micro-pellets,
and
the intermediate punch neither has a holdfast for the core material, nor transfers the core material from a holdfast into the die.

4. A method according to any one of the preceding claims, **characterized in that** the lower punch has an at least two-part structure, comprising an inner punch and an outer punch, the outer punch surrounding the outer edge of the inner punch, wherein both the inner punch and the outer punch can perform both shifting movements and compression steps and that the lower punch preferably has a three-part structure, which, compared to the two-part structure, additionally comprises an outermost punch, wherein the outermost punch surrounds the outer edge of the outer punch, and also the outermost punch can perform both shifting movements and compression steps.

5. A method according to any one of the preceding claims, **characterized in that** the upper punch does not have a two-part structure comprising an inner punch and an outer punch surrounding the outer edge of the inner punch.

6. A method according to any one of the preceding claims, **characterized in that** the sequence of the method comprises:
- A supply step 1 of an outer layer, with which molding material for a first part of the outer layer is supplied into a recess above the lower inner punch, which is enclosed by the lower outer punch;
- a core supply step, in which the molding material for the core is supplied into a recess above the molding material for the first part of the outer layer, which has been supplied in the preceding step, and enclosed by the lower outer punch;
- a pressing step of the outer layer and the core, in which the molding material for the first outer layer and the molding material for the core, supplied in the preceding steps, are compression-molded;
- a supply step 2 of the outer layer, in which a molding material for a second part of the outer layer is supplied into a recess of a die above and / or around the first part of the outer layer and the core, which have been formed in the preceding step;
- an overall pressing step, in which the first part of the outer layer, the core and the molding material for the second part of the outer layer, which has been supplied in the preceding step are compression-molded; and
- a step of removal of the compression-molded molding, which is carried out after the overall pressing step;
and wherein at least during one pressing step, an intermediate punch is positioned between the upper punch and the die,
and preferably that the method comprises, between the supplying step 1 and the core supply step, at least one pressing step of the outer layer, with which the molding material for the first part of the outer layer is compression-molded.

7. A method according to any one of claims 1 to 5, **characterized in that** the sequence of the method comprises:
- a supply step 1 of an outer layer, with which molding material for a first part of the outer layer is supplied into a recess above the lower punch;
- a pressing step of the outer layer, with which a synclinal recess is compression-molded into the first part of the outer layer;
- a core supply step with which the molding material for the core is supplied into the synclinal recess of the first part of the outer layer;
- a supply step 2 of the outer layer, with which molding material for a second part of the outer layer is supplied into the die above the first part of the outer layer and the core;
- an overall pressing step, with which the first part of the outer layer, the core and the molding material for the second part of the outer layer, which has been supplied in the preceding step, are compression-molded; and
- a step of removal of the compression-molded molding, which is carried out after the overall pressing step;
and
wherein at least with one pressing step an intermediate punch is positioned between the upper punch and the die, and preferably that
- a pressing step of the outer layer and the core, with which at least the molding material for the core supplied with the preceding step is pressed, is inserted into the method between the core supply step and the supply step 2 of the outer layer.

8. A method according to claim 4 or 5, **characterized in that**
the lower punch has a three-part structure, which, compared to the two-part structure, additionally comprises an outermost punch, wherein the outermost punch surrounds the outer edge of the outer punch, and also the outermost punch can perform both shifting movements and compression steps and that the sequence of the method comprises:
- a supply step 1 of an outer layer, with which the molding material for a first part of the outer layer is supplied in a recess above the lower inner punch, which is enclosed by the lower outer punch;
- a core supply step, in which the molding material for the core is supplied into a recess above the molding material for the first part of the outer layer, which has been supplied in the preceding step, and enclosed by the lower outer punch;
- a pressing step of the outer layer and the core, in which the molding material for the first part of the outer layer and the molding material for the core supplied in the preceding steps, are compression-molded;
- a supply step 2 of the outer layer, in which a molding material for a second part of the outer layer is supplied into a recess above and if applicable laterally of the molding material, which has been supplied in the preceding steps, enclosed by the lower outermost punch;
- a pressing step in which the first part of the outer layer, the core and the molding material for the second part of the outer layer, which have been supplied in the preceding step are compression-molded;
- a supply step of an outermost layer, in which a molding material for the outermost layer is supplied into a recess above and if applicable laterally of the molding material that has been supplied and pressed in the preceding steps;
- a lowering step, with which the lower inner punch and the lower outer punch are lowered;
- an overall pressing step, with which the outermost layer, the outer layer and the core material are compression-molded; and
- a step of removal of the compression-molded molding, which is carried out after the overall pressing step;
and
wherein at least with one pressing step, an intermediate punch is positioned between the upper punch and the die.

9. A method according to any one of the preceding claims, **characterized in that** with the pressing steps of the first part of the outer layer and of the core the intermediate punch is moved downwards by the upper punch.

10. A compression molding apparatus comprising an upper punch (2) and a lower punch (1) which are arranged in the vertical direction of a die (3), and also an intermediate punch (38), and a device with which the intermediate punch can be positioned between the upper punch and the die and be removed from this position, **characterized in that**
the intermediate punch has a pressing surface (27) on its underside, with which shell and/or core material, consisting of at least two particles which are not firmly connected to each other, for example, of powders, of granules, several crystals or micro-pellets, can be compressed
and/or
that the intermediate punch has no holdfast at its underside.

11. A compression molding apparatus according to claim 10, **characterized in that**
the intermediate punch has a pressing surface (27) on its underside, with which shell and/or core material, consisting of at least two particles which are not firmly connected to each other, for example, of powders, of granules, several crystals or micro-pellets, can be compressed.

12. A compression molding apparatus according to claim 10 or 11, **characterized in that**
the intermediate punch has no holdfast at its underside
and/or
that the compression molding apparatus does not have a holdfast, from which the intermediate punch transfers the core material into the die.

13. A compression molding apparatus according to claim 10, 11 or 12, **characterized in that**
the upper punch does not have a two-part structure comprising an inner punch and an outer punch surrounding the outer edge of the inner punch.

14. A compression molding apparatus according to any one of claims 10-13, **characterized in that**
the lower punch has an at least two-part structure, comprising an inner punch and an outer punch, the outer punch surrounding the outer edge of the inner punch, wherein both the inner punch and the outer punch can perform both shifting movements and compression steps and that the lower punch preferably has a three-part structure, which, compared to the two-part structure, additionally comprises an outermost punch, wherein the outermost punch surrounds the outer edge of the outer punch, and also the outermost punch can perform both shifting movements and compression steps.

15. A method according to any one of claims 1-9 or 17 or a compression molding apparatus according to any one of claims 10-14, **characterized in that** the compression molding apparatus has a die or a die insert (40) with a tubular insert (43), the tubular insert being vertically shiftable with respect to the die or the die insert,
and preferably that the tubular insert (43) with methods according to claim 6 or 17 or with compression molding apparatuses according to claim 14 with a two-part lower punch performs as the outer lower punch and the lower punch instead of being two-part coaxial is only one piece,
and preferably that the tubular insert (43) with methods according to claim 8 or with compression molding apparatuses according to claim 14 with a three-part lower punch performs as the outermost lower punch and the lower punch is only two-part coaxial instead of three-part coaxial.

16. A method or compression molding apparatus according to claim 15, **characterized in that** the extent to which the tubular insert (43) can be shifted vertically with respect to the die or the die insert (40) is limited by one or more limit-stops and/or by a certain force being necessary for the shift.

17. A method according to any one of claims 1-5, **characterized in that** the sequence of the method comprises:
- a core supply step with which the molding material for the core is supplied into a recess above the lower inner punch, surrounded by the lower outer punch;
- a pressing step of the core, in which the molding material for the core supplied in the preceding step is compression-molded;
- a shifting step, with which the lower outer punch is lowered and/or the lower inner punch is raised;
- a supply step of an outer layer, with which a molding material for the outer layer (47) is supplied into a recess above and laterally of the molding material that has been supplied and pressed in the preceding steps, and is also supplied laterally of the lower inner punch;
- a lowering step with which the lower inner punch is lowered;
- an overall pressing step, with which the outer layer and the core material are compression-molded; and
- a step of removal of the compression-molded molding, which is carried out after the overall pressing step;
wherein the shifting step can be carried out before, after or during the execution of the supply-step of the outer layer,
and
wherein at least with one pressing step an intermediate punch is positioned between the upper punch and the die.

## Revendications

1. Procédé de fabrication d'un article moulé comportant un noyau, faisant appel à un dispositif de mise en forme par compression qui comprend un poinçon supérieur (2) et un poinçon inférieur (1) disposés à la verticale d'un moule de compression (3), et qui comprend aussi un poinçon intermédiaire (38) et un dispositif permettant de positionner le poinçon intermédiaire entre le poinçon supérieur et le moule de compression et de le retirer de cette position, **caractérisé en ce que** le poinçon intermédiaire présente, sur sa face inférieure, une surface de compression (27) permettant de comprimer du matériau enveloppant et/ou du matériau pour le noyau, et
**en ce que** le matériau composant le noyau est introduit dans le moule de compression avant d'entrer en contact avec le poinçon intermédiaire,
et/ou
**en ce que**, lorsqu'il est introduit dans le moule de compression, le matériau pour le noyau se compose d'au moins deux particules qui ne sont pas fermement solidaires l'une de l'autre, par exemple de poudre, de granulés, de plusieurs cristaux ou micropastilles,
et/ou
**en ce que** le poinçon intermédiaire ne présente aucun dispositif de retenue pour le matériau pour le noyau ni qu'il ne transporte le matériau pour le noyau dans le moule de compression à partir d'un dispositif de retenue.

2. Procédé suivant la revendication 1, **caractérisé en ce que**
le poinçon intermédiaire ne présente aucun dispositif de retenue pour le matériau pour le noyau ni qu'il ne transporte le matériau pour le noyau dans le moule de compression à partir d'un dispositif de retenue.

3. Procédé suivant la revendication 1, **caractérisé en ce que**
le matériau composant le noyau est introduit dans le moule de compression avant d'entrer en contact avec le poinçon intermédiaire,
et
**en ce que**, lorsqu'il est introduit dans le moule de compression, le matériau pour le noyau se compose d'au moins deux particules qui ne sont pas fermement solidaires l'une de l'autre, par exemple de poudre, de granulés, de plusieurs cristaux ou micropastilles,
et
**en ce que** le poinçon intermédiaire ne présente aucun dispositif de retenue pour le matériau pour le noyau ni qu'il ne transporte le matériau pour le noyau dans le moule de compression à partir d'un dispositif de retenue.

4. Procédé suivant l'une des revendications précédentes, **caractérisé en ce que** le poinçon inférieur présente une structure au moins en deux parties, composée d'un poinçon intérieur et d'un poinçon extérieur, le poinçon extérieur entourant la bordure extérieure du poinçon intérieur, le poinçon intérieur et le poinçon extérieur pouvant effectuer à la fois des mouvements coulissants et des opérations de compression, et **en ce que** le poinçon inférieur présente de préférence une structure en trois parties qui, comparée à la structure en deux parties, se compose, en plus, d'un poinçon le plus extérieur, le poinçon le plus extérieur entourant la bordure extérieure du poinçon extérieur et le poinçon le plus extérieur pouvant aussi effectuer à la fois des mouvements coulissants et des opérations de compression.

5. Procédé suivant l'une des revendications précédentes, **caractérisé en ce que** le poinçon supérieur ne présente pas de structure en deux parties composée d'un poinçon intérieur et d'un poinçon extérieur entourant le bord extérieur du poinçon intérieur.

6. Procédé suivant l'une des revendications précédentes, **caractérisé en ce que** le déroulement du procédé comprend :
- une étape d'alimentation 1 d'une couche extérieure, au cours de laquelle du matériau à mettre en forme pour une première partie de la couche extérieure est amené dans un évidement au-dessus du poinçon inférieur intérieur entouré par le poinçon inférieur extérieur ;
- une étape d'alimentation du noyau, au cours de laquelle le matériau à mettre en forme pour le noyau est amené dans un évidement au-dessus du matériau à mettre en forme pour la première partie de la couche extérieure, amené lors de l'étape précédente, en étant entouré par le poinçon inférieur extérieur ;
- une étape de compression de la couche extérieure et du noyau, au cours de laquelle le matériau à mettre en forme pour la première partie de la couche extérieure, amené lors des étapes précédentes, et le matériau à mettre en forme pour le noyau sont mis en forme par compression ;
- une étape d'alimentation 2 de la couche extérieure, au cours de laquelle du matériau à mettre en forme pour une deuxième partie de la couche extérieure est amené dans un évidement dans un moule de compression au-dessus et/ou autour de la première partie de la couche extérieure et du noyau qui ont été mis en forme lors de l'étape précédente ;
- une étape de compression générale au cours de laquelle la première partie de la couche extérieure, le noyau et le matériau à mettre en forme pour la deuxième partie de la couche extérieure, amené lors de l'étape précédente, sont mis en forme par compression ; et
- une étape de retrait de l'article moulé mis en forme par compression, qui a lieu à l'issue de l'étape de compression générale ;
et en sachant qu'au cours d'au moins une étape de compression un poinçon intermédiaire est positionné entre le poinçon supérieur et le moule de compression,
et, de préférence, **en ce que** le procédé comprend, entre l'étape d'alimentation 1 et l'étape d'alimentation du noyau, au moins une étape de compression de la couche extérieure au cours de laquelle le matériau à mettre en forme pour la première partie de la couche extérieure est mis en forme par compression.

7. Procédé suivant l'une des revendications 1 à 5, **caractérisé en ce que** le déroulement du procédé comprend :
- une étape d'alimentation 1 d'une couche extérieure, au cours de laquelle du matériau à mettre en forme pour une première partie de la couche extérieure est amené dans un évidement au-dessus du poinçon inférieur ;
- une étape de compression de la couche extérieure, au cours de laquelle un renfoncement en forme de cuvette est mis en forme par compression dans la première partie de la couche extérieure ;
- une étape d'alimentation du noyau, au cours de laquelle le matériau à mettre en forme pour le noyau est amené dans le renfoncement en forme de cuvette dans la première partie de la couche extérieure ;
- une étape d'alimentation 2 de la couche extérieure, au cours de laquelle du matériau à mettre en forme pour une deuxième partie de la couche extérieure est amené dans le moule de compression au-dessus de la première partie de la couche extérieure et du noyau ;
- une étape de compression générale au cours de laquelle la première partie de la couche extérieure, le noyau et le matériau à mettre en forme pour la deuxième partie de la couche extérieure, amené lors de l'étape précédente, sont mis en forme par compression ; et
- une étape de retrait de l'article moulé mis en forme par compression, qui a lieu à l'issue de l'étape de compression générale ;
et
en sachant que, lors d'au moins une étape de compression, un poinçon intermédiaire est positionné entre le poinçon supérieur et le moule de compression, et de préférence, **en ce qu'**est intercalée, dans le procédé, entre l'étape d'alimentation du noyau et l'étape d'alimentation 2 de la couche extérieure, une étape de compression de la couche extérieure et du noyau, au cours de laquelle est comprimé au moins le matériau à mettre en forme amené à l'étape précédente pour le noyau.

8. Procédé suivant la revendication 4 ou 5, **caractérisé en ce que** le poinçon inférieur présente une structure en trois parties qui, comparée à la structure en deux parties, comprend en plus un poinçon le plus extérieur, le poinçon le plus extérieur entourant le bord extérieur du poinçon extérieur et le poinçon le plus extérieur pouvant également effectuer aussi bien des mouvements coulissants que des opérations de compression, et **en ce que** le déroulement du procédé comprend :
- une étape d'alimentation 1 d'une couche extérieure, au cours de laquelle du matériau à mettre en forme pour une première partie de la couche extérieure est amené dans un évidement au-dessus du poinçon inférieur intérieur entouré par le poinçon inférieur extérieur ;
- une étape d'alimentation du noyau, au cours de laquelle le matériau à mettre en forme pour le noyau est amené dans un évidement au-dessus du matériau à mettre en forme pour la première partie de la couche extérieure, amené lors de l'étape précédente, en étant entouré par le poinçon inférieur extérieur ;
- une étape de compression de la couche extérieure et du noyau, au cours de laquelle le matériau à mettre en forme pour la première partie de la couche extérieure, amené lors des étapes précédentes, et le matériau à mettre en forme pour le noyau sont mis en forme par compression ;
- une étape d'alimentation 2 de la couche extérieure, au cours de laquelle du matériau à mettre en forme pour une deuxième partie de la couche extérieure est amené dans un évidement au-dessus de et, le cas échéant, sur les côtés du matériau à mettre en forme amené lors des étapes précédentes, en étant entouré par le poinçon inférieur plus extérieur ;
- une étape de compression au cours de laquelle la première partie de la couche extérieure, le noyau et le matériau à mettre en forme pour la deuxième partie de la couche extérieure, amené lors de l'étape précédente, sont mis en forme par compression ;
- une étape d'alimentation d'une couche la plus extérieure, au cours de laquelle du matériau à mettre en forme pour la couche la plus extérieure est amené dans un évidement au-dessus de et, le cas échéant, sur les côtés du matériau à mettre en forme amené et comprimé lors des étapes précédentes ;
- une étape de descente au cours de laquelle le poinçon inférieur intérieur et le poinçon inférieur extérieur sont descendus ;
- une étape de compression générale au cours de laquelle la couche la plus extérieure, la couche extérieure et le matériau pour le noyau sont mis en forme par compression ; et
- une étape de retrait de l'article moulé mis en forme par compression, qui a lieu à l'issue de l'étape de compression générale ;
et
en sachant qu'au cours d'au moins une étape de compression un poinçon intermédiaire est positionné entre le poinçon supérieur et le moule de compression.

9. Procédé suivant l'une des revendications précédentes, **caractérisé en ce que** le poinçon intermédiaire est déplacé vers le bas au moyen du poinçon supérieur lors des étapes de compression de la première partie de la couche extérieure et du noyau.

10. Dispositif de mise en forme par compression qui comprend un poinçon supérieur (2) et un poinçon inférieur (1) disposés à la verticale d'un moule de compression (3), et qui comprend aussi un poinçon intermédiaire (38) et un dispositif permettant de positionner le poinçon intermédiaire entre le poinçon supérieur et le moule de compression et de le retirer de cette position, **caractérisé en ce que**
le poinçon intermédiaire présente, sur sa face inférieure, une surface de compression (27) permettant de comprimer du matériau enveloppant et/ou du matériau pour le noyau se composant d'au moins deux particules qui ne sont pas fermement solidaires l'une de l'autre, par exemple de poudre, de granulés, de plusieurs cristaux ou micropastilles,
et/ou
**en ce que** le poinçon intermédiaire ne présente aucun dispositif de retenue sur sa face inférieure.

11. Dispositif de mise en forme par compression suivant la revendication 10, **caractérisé en ce que**
le poinçon intermédiaire présente, sur sa face inférieure, une surface de compression (27) permettant de comprimer du matériau enveloppant et/ou du matériau pour le noyau se composant d'au moins deux particules qui ne sont pas fermement solidaires l'une de l'autre, par exemple de poudre, de granulés, de plusieurs cristaux ou micropastilles.

12. Dispositif de mise en forme par compression suivant la revendication 10 ou 11, **caractérisé en ce que**
le poinçon intermédiaire ne présente aucun dispositif de retenue sur sa face inférieure,
et/ou
**en ce que** le dispositif de mise en forme par compression ne présente aucun dispositif de retenue à partir duquel le poinçon intermédiaire transporte le matériau pour le noyau dans le moule de compression.

13. Dispositif de mise en forme par compression suivant la revendication 10, 11 ou 12, **caractérisé en ce que**
le poinçon supérieur ne présente pas de structure en deux parties composée d'un poinçon intérieur et d'un poinçon extérieur entourant le bord extérieur du poinçon intérieur.

14. Dispositif de mise en forme par compression suivant l'une des revendications 10-13, **caractérisé en ce que**
le poinçon inférieur présente une structure au moins en deux parties, composée d'un poinçon intérieur et d'un poinçon extérieur, le poinçon extérieur entourant le bord extérieur du poinçon intérieur, le poinçon intérieur et le poinçon extérieur pouvant, tous deux, effectuer aussi bien des mouvements coulissants que des opérations de compression, et **en ce que** le poinçon inférieur présente, de préférence, une structure en trois parties qui, comparée à la structure en deux parties, comprend en plus un poinçon le plus extérieur, le poinçon le plus extérieur entourant le bord extérieur du poinçon extérieur et le poinçon le plus extérieur pouvant également effectuer aussi bien des mouvements coulissants que des opérations de compression.

15. Procédé suivant l'une des revendications 1-9 ou 17, ou dispositif de mise en forme par compression suivant l'une des revendications 10-14, **caractérisé en ce que** le dispositif de mise en forme par compression présente une matrice ou un insert de matrice (40) pourvu d'un insert tubulaire (43), l'insert tubulaire pouvant être coulissé à la verticale par rapport à la matrice ou à l'insert de matrice,
et, de préférence, **en ce que** l'insert tubulaire (43), dans le procédé suivant la revendication 6 ou17 ou dans le dispositif de mise en forme par compression suivant la revendication 14 avec poinçon inférieur en deux parties, assure les fonctions du poinçon inférieur extérieur et que le poinçon inférieur ne se compose plus que d'une seule pièce au lieu de deux pièces coaxiales,
et, de préférence, **en ce que** l'insert tubulaire (43), dans le procédé suivant la revendication 8 ou dans le dispositif de mise en forme par compression suivant la revendication 14 avec poinçon inférieur en trois parties, assure les fonctions du poinçon inférieur le plus extérieur et que le poinçon inférieur ne se compose plus que de deux pièces coaxiales au lieu de trois pièces coaxiales.

16. Procédé ou dispositif de mise en forme par compression suivant la revendication 15, **caractérisé en ce que** le coulissement vertical de l'insert tubulaire (43) par rapport à la matrice ou l'insert de matrice (40) est limité par une ou plusieurs butées et/ou par le fait qu'une force spécifique est requise pour le coulissement.

17. Procédé suivant l'une des revendications 1 à 5, **caractérisé en ce que** le déroulement du procédé comprend :
- une étape d'alimentation du noyau, au cours de laquelle du matériau à mettre en forme pour le noyau est amené dans un évidement au-dessus du poinçon inférieur intérieur entouré par le poinçon inférieur extérieur ;
- une étape de compression du noyau, au cours de laquelle le matériau à mettre en forme amené à l'étape précédente est mis en forme par compression pour le noyau ;
- une étape de coulissement au cours de laquelle le poinçon inférieur extérieur est descendu et/ou le poinçon inférieur intérieur est levé ;
- une étape d'alimentation d'une couche extérieure, au cours de laquelle du matériau à mettre en forme pour la couche extérieure (47) est amené dans un évidement au-dessus et sur les côtés du matériau à mettre en forme amené et comprimé lors des étapes précédentes, ainsi que sur les côtés du poinçon inférieur intérieur ;
- une étape de descente au cours de laquelle le poinçon inférieur intérieur est descendu ;
- une étape de compression générale au cours de laquelle la couche extérieure et le matériau pour le noyau sont mis en forme par compression ; et
- une étape de retrait de l'article moulé mis en forme par compression, qui a lieu à l'issue de l'étape de compression générale ;
en sachant que l'étape de coulissement peut être exécutée avant, après ou pendant l'exécution de l'étape d'alimentation de la couche extérieure,
et
en sachant qu'au cours d'au moins une étape de compression un poinçon intermédiaire est positionné entre le poinçon supérieur et le moule de compression.
